# EUROPÄISCHE PATENTANMELDUNG

(11) **EP 2 687 511 A1**
(43) Veröffentlichungstag der Anmeldung: **22.01.2014**
(21) Anmeldenummer: 12176776.8
(22) Anmeldetag: 17.07.2012
(51) Int. Cl.: C07D 213/75, C07D 401/12, C07D 405/12, C07D 409/12, C07D 413/12, C07D 417/12, C07D 493/10, A61K 31/4412, A61K 31/443, A61K 31/4545, A61K 31/4436, A61K 31/4439, A61K 31/4433, A61P 1/00

(54) **Pyridinonderivate als Gewebetransglutaminaseinhibitoren**

(71) Anmelder: Zedira GmbH, 64293 Darmstadt (DE)
(72) Erfinder: Büchold, Christian, 61118 Bad Vilbel (DE); Gerlach, Uwe, 65795 Hattersheim (DE); Hils, Martin, 64295 Darmstadt (DE); Pasternack, Ralf, 64347 Griesheim (DE); Weber, Johannes, 64295 Darmstadt (DE)
(74) Vertreter: Arth, Hans-Lothar

(57) **Zusammenfassung**

Die Erfindung betrifft Pyridinonderivate als Inhibitoren für die Gewebetransglutaminase, Verfahren zur Herstellung der Pyridinonderivate, pharmazeutische Zusammensetzungen enthaltend diese Pyridinonderivate sowie deren Verwendung zur Prophylaxe und Behandlung von gewebetransglutaminase-assoziierten Erkrankungen.

## Beschreibung

Die Erfindung betrifft Pyridinonderivate als Inhibitoren für die Gewebetransglutaminase, Verfahren zur Herstellung der Pyridinonderivate, pharmazeutische Zusammensetzungen enthaltend diese Pyridinonderivate sowie deren Verwendung zur Prophylaxe und Behandlung von Gewebetransglutaminase-assoziierten Erkrankungen.

Die Transglutaminasen gehören zur Klasse der Transferasen, nach EC-Nomenklatur korrekterweise "Protein-Glutamin: Amin-γ-Glutamyltransferasen" und besitzen die EC-Nummer EC 2.3.2.13. Sie verknüpfen die ε-Aminogruppe der Aminosäure Lysin mit der γ-Glutamylgruppe der Aminosäure Glutamin unter Freisetzung von Ammoniak zu einer Isopeptidbindung.

Daneben spielen die Transglutaminasen eine große Rolle auf vielen Indikationsgebieten, wie zum Beispiel Herz-Kreislauf (Thrombose), Autoimmunkrankheiten (Zöliakie, Morbus Duhring), neurodegenerative Krankheiten (Alzheimer, Parkinson, Chorea Huntington), dermatologische Krankheiten (Ichthyosis, Psoriasis, Akne), sowie auch bei Wundheilung und in Entzündungskrankheiten (Gewebsfibrose) (J.M. Wodzinska, Mini-Reviews in medical cheimistry, 2005, 5, 279-292).

Eine der wichtigsten Indikationen ist jedoch die Glutenunverträglichkeit Zöliakie. Zöliakie ist eine chronische Entzündung der Dünndarmschleimhaut. Die Darmepithelien werden bei den betroffenen Patienten, nach Aufnahme von glutenhaltigen Lebensmitteln, sukzessive zerstört, was eine gestörte Resorption von Nährstoffen nach sich zieht und massive Auswirkungen auf die betroffenen Patienten hat und zum Beispiel mit Gewichtsverlust, Anämie, Diarrhoe, Erbrechen, Appetitlosigkeit und Müdigkeit einhergeht. Auf Grund dieser Erkenntnisse entsteht somit ein großer Bedarf, ein Medikament für die Zöliakiebehandlung sowie anderer Gewebetransglutaminaseassoziierter Erkrankungen zu entwickeln. Die Gewebetransglutaminase ist ein zentrales Element in der Pathogenese. Das körpereigene Enzym katalysiert die Deamidierung von Gluten/Gliadin in der Dünndarmschleimhaut und verstärkt dadurch massiv die Entzündungsreaktion. Deshalb sind Inhibitoren der Gewebetransglutaminase geeignet, um als Wirkstoffe zur medikamentösen Behandlung eingesetzt zu werden.

Der vorliegenden Erfindung liegt die Aufgabe zugrunde neue Inhibitoren für die Gewebetransglutaminase zur Verfügung zu stellen sowie pharmazeutische Formulierungen enthaltend diese Inhibitoren und Synthesewege für diese Inhibitoren. Ferner werden neue Verwendungen dieser Inhibitoren aufgezeigt.

Diese Aufgabe wird durch die technische Lehre der unabhängigen Ansprüche gelöst. Weitere vorteilhafte Ausgestaltungen, Aspekte und Details der Erfindung ergeben sich aus den abhängigen Ansprüchen, der Beschreibung und den Beispielen.

Überraschend wurde gefunden, dass Pyridinonderivate, welche mindestens eine akzeptorsubstituierte Doppelbindung aufweisen und ferner ein Pyridinonfragment in molekular räumlicher Nähe zu der akzeptorsubstituierten Doppelbindung tragen, besonderes gute Inhibitoren für die Gewebetransglutaminase sind, welche auch als Transglutaminase 2 oder TG2 bezeichnet wird. Diese Bezeichnungen werden hierin synonym verwendet. Solche akzeptorsubstituierte Doppelbindungen sind vorzugsweise Michael-Systeme (MS) aus einer Carbonylfunktion (C=O) oder einer Sulfonylfunktion (SO₂) sowie einer damit konjugierten Kohlenstoff-Kohlenstoff-Doppelbindung (C=C), also ein Michael-Akzeptor-System der Art C=C―C=O oder C=C― SO₂. Wahrscheinlich handelt es sich bei den erfindungsgemäßen Pyridinonderivaten um Suizid-Inhibitoren, welche irreversibel an die Transglutaminase binden.

Somit betrifft die vorliegende Erfindung Verbindungen der allgemeinen Formel (I): 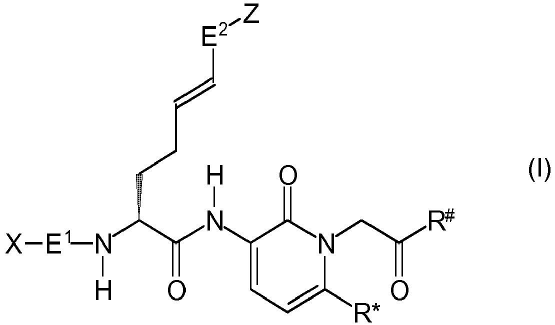 worin
**E¹, E²** unabhängig voneinander für ―CO― oder ―SO₂― stehen,
**R#** einen der folgenden Reste bedeutet ―NYY', ―NH―CH₂―COOH, ―NH―CH(CH₃)― COOH, ―NH―CH(CH₂CH₂SCH₃)―COOH, ―NH―CH(CH₂OH)―COOH, ―NH― CH(CH₂SH)―COOH, ―NH―CH(CH₂CONH₂)―COOH, ―NH―CH(CH₂CH₂CONH₂)―COOH, 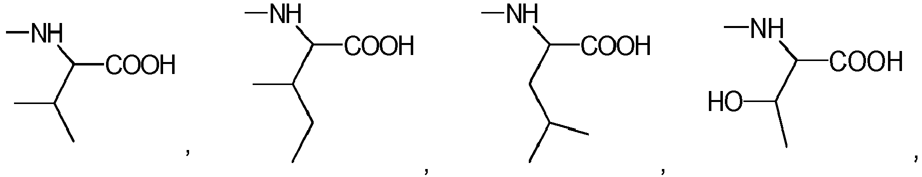 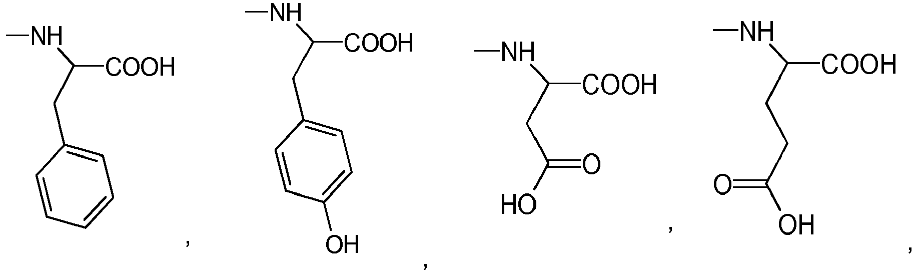 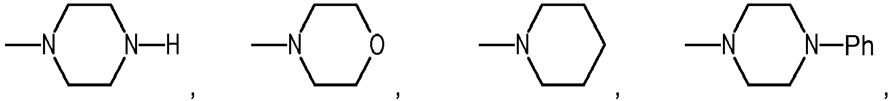 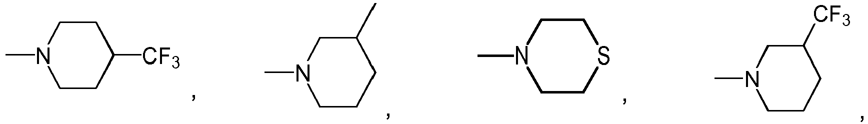 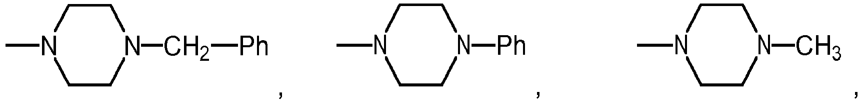 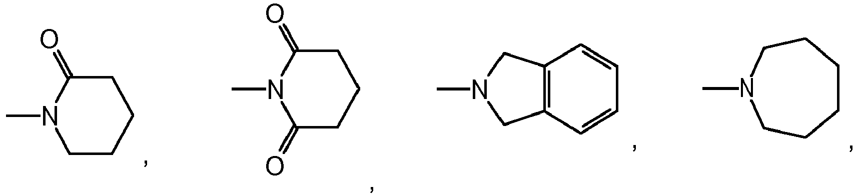 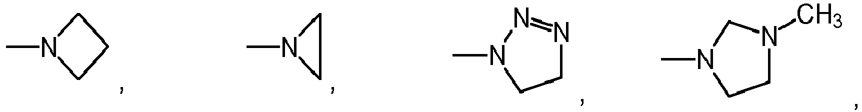 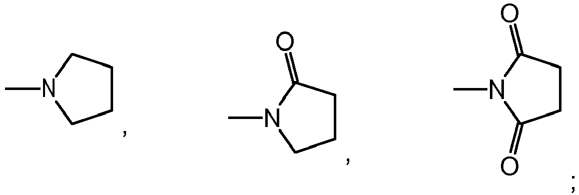 **Y** für einen der folgenden Reste steht: ―CH₂R¹, ―CHR¹―CH₂R², ―CHR¹―CHR²―CH₂R³, -CHR¹-CHR²-CHR³-CH₂R⁴, ―CHR¹―CHR²―CHR³―CHR⁴―CH₂R⁵, ―CHR¹CHR²―CHR³―CHR⁴―CHR⁵―CH₂R⁶
**Y', R¹⁷** bis **R²⁰** unabhängig voneinander ausgewählt werden aus: ―H, ―CH₃, ―C₂H₅, ―C₃H₇, ―CH(CH₃)₂, ―C₄H₉ ―CH₂―CH(CH₃)₂, ―CH(CH₃)―C₂H₅, ―C(CH₃)₃, ―C₅H₁₁, ―CH(CH₃)―C₃H₇, ―CH₂―CH(CH₃)―C₂H₅, ―CH(CH₃)―CH(CH₃)₂, ―C(CH₃)₂―C₂H₅, ―CH₂―C(CH₃)₃, ―CH(C₂H₅)₂ und ―C₂H₄―CH(CH₃)₂;
**X** bedeutet: ―CH₂R⁷, ―CHR⁷―CH₂R⁸, ―O―CH₂R⁷, ―O―CHR⁷―CH₂R⁸, 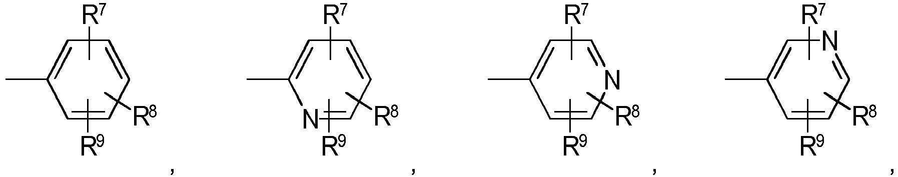 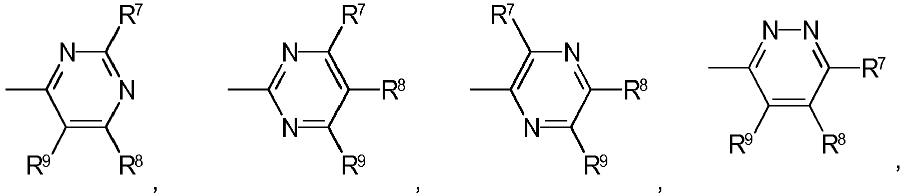 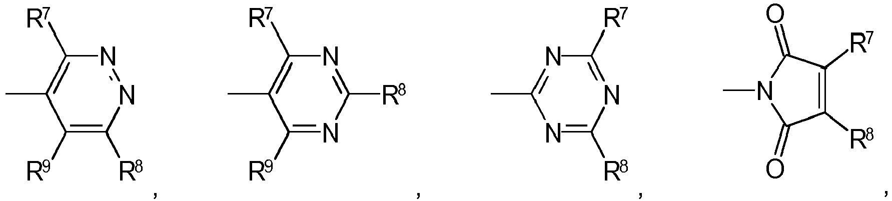 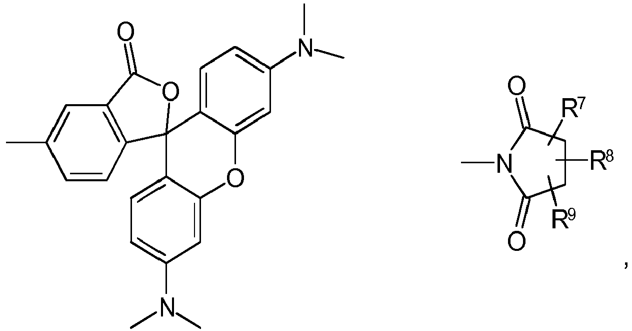 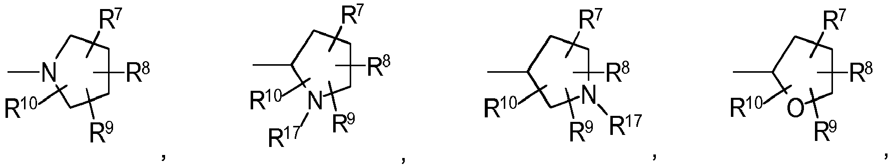 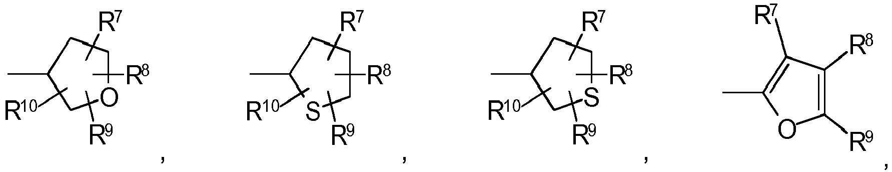 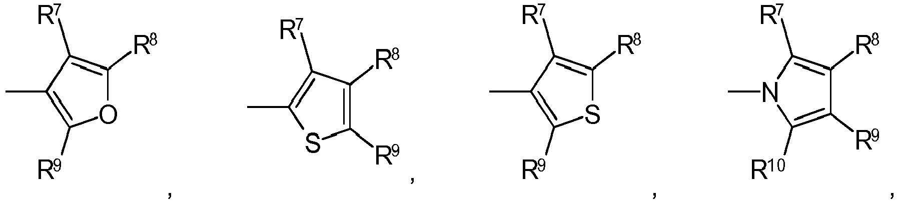 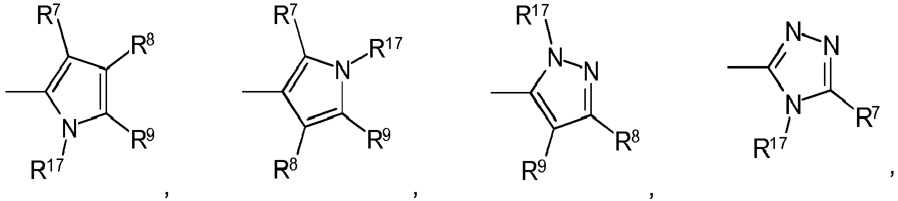 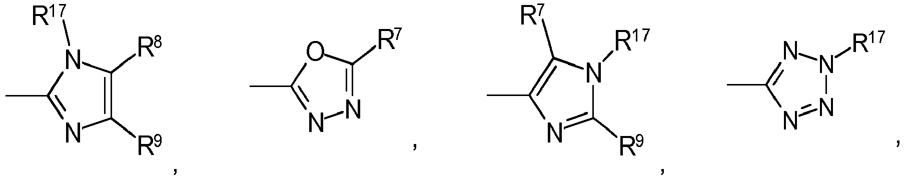 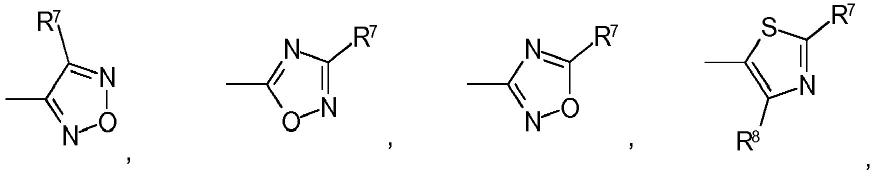 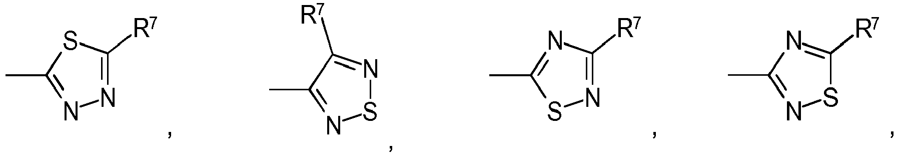 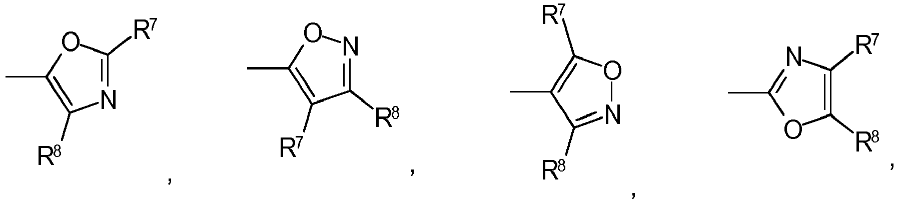 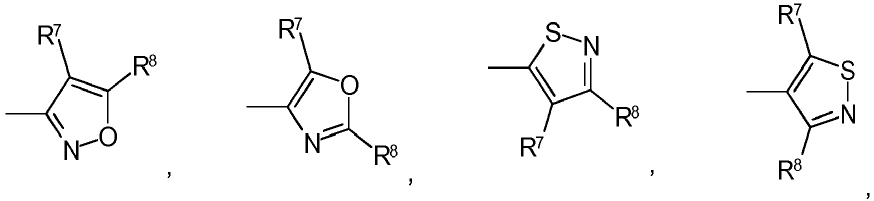 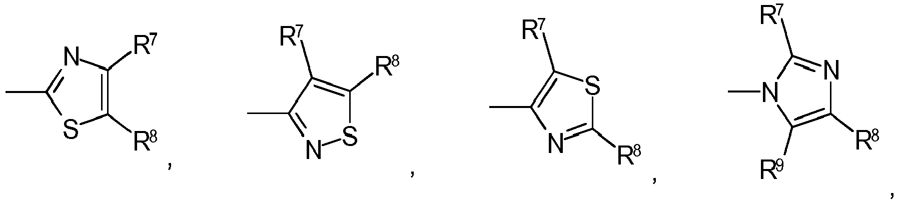 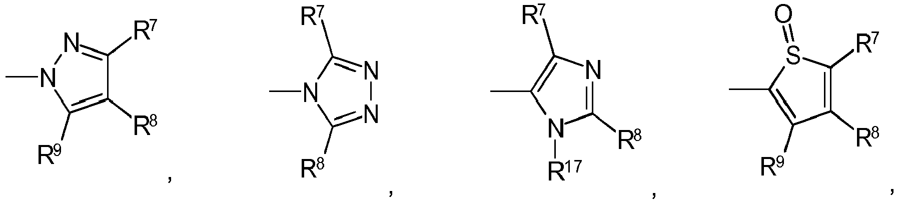 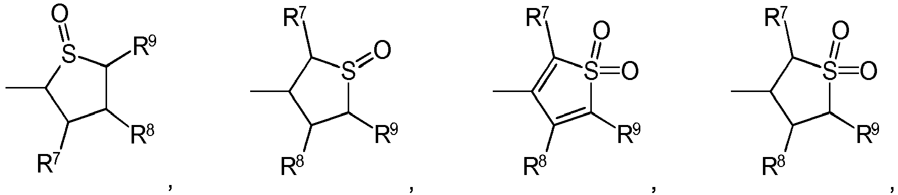 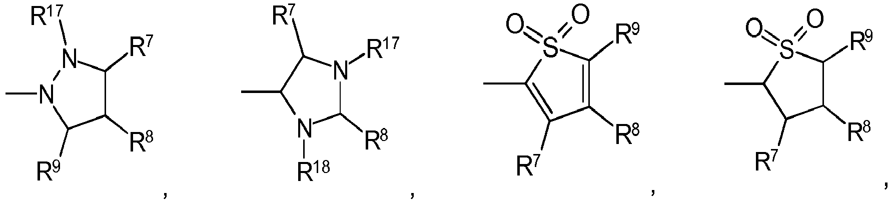 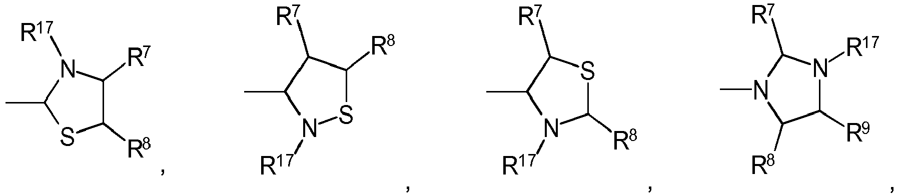 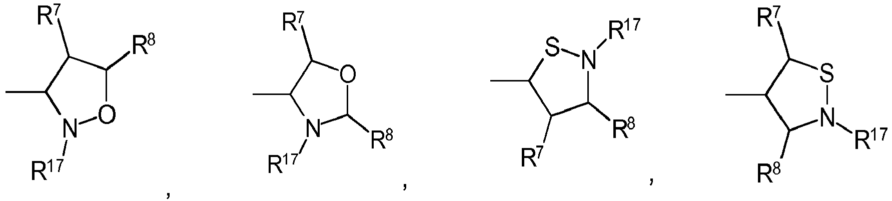 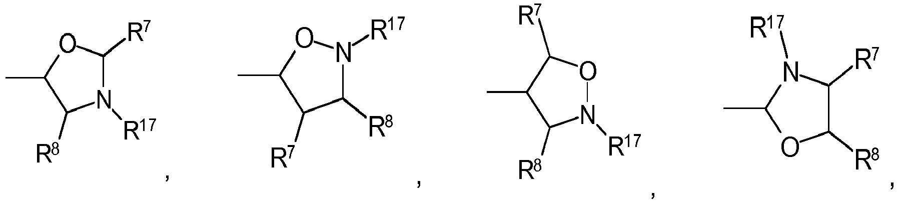 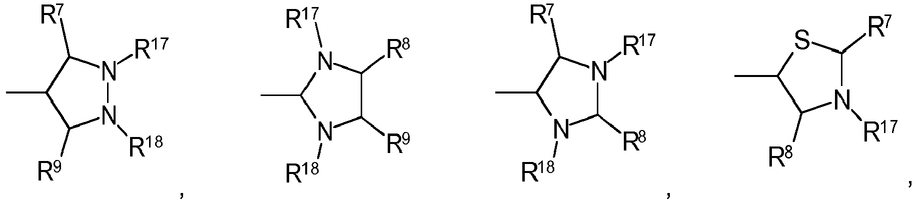 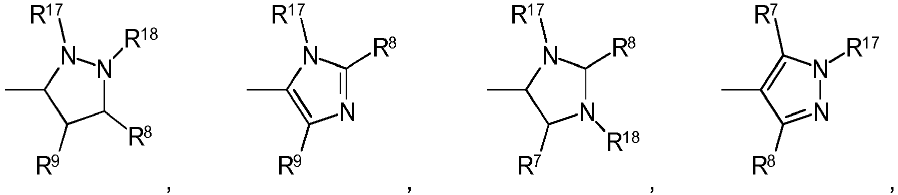 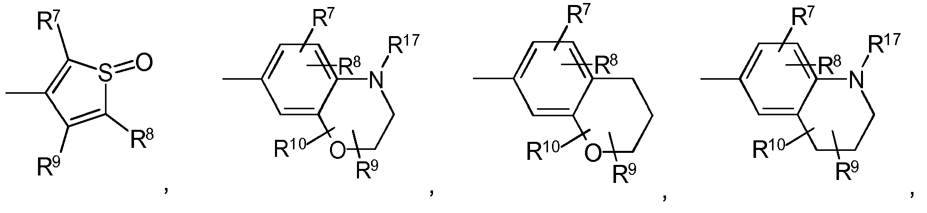 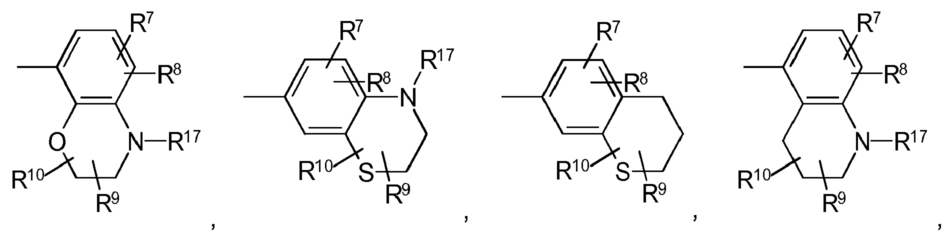 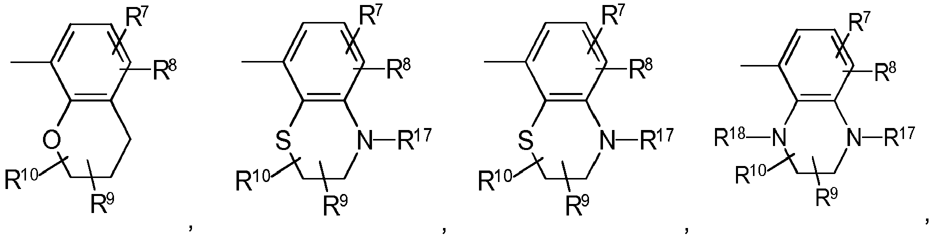 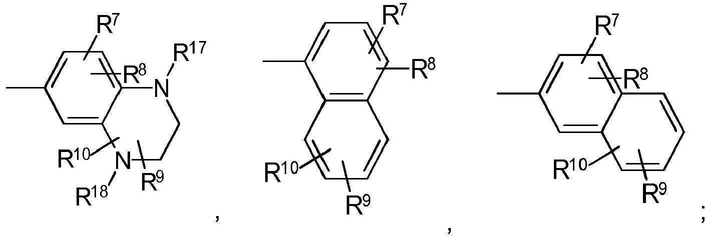 **Z** bedeutet: ―CH₃, ―C₂H₅, ―C₃H₇, ―CH(CH₃)₂, ―C₄H₉, ―Ph, ―CH₂―Ph, ―OCH₃, ―OC₂H₅, ―OC₃H₇, ―OCH(CH₃)₂, ―OC₄H₉, ―OPh, ―OCH₂―Ph, ―OCH=CH₂ oder ―OCH₂―CH=CH₂;
**R¹** bis **R¹⁰** unabhängig voneinander für folgende Gruppen stehen: ―H, ―OH, ―OCH₃, ―OC₂H₅, ―OC₃H₇, ―O―cyclo-C₃H₅, ―OCH(CH₃)₂, ―OC(CH₃)₃, ―OC₄H₉, ―OPh, ―OCH₂―Ph, ―OCPh₃, ―SH, ―NO₂, ―F, ―Cl, ―Br, ―l, ―N₃, ―CN, ―OCN, ―NCO, ―SCN, ―NCS, ―CHO, ―COCH₃, ―COC₂H₅, ―COC₃H₇, ―CO―cyclo-C₃H₅, ―COCH(CH₃)₂, ―COC(CH₃)₃, ―COOH, ―COCN, ―COOCH₃, ―COOC₂H₅, ―COOC₃H₇, ―COO―cyclo-C₃H₅, ―COOCH(CH₃)₂, ―COOC(CH₃)₃, ―CONH₂, ―CONHCH₃, ―CONHC₂H₅, ―CONHC₃H₇, ―CONH―cyclo-C₃H₅, ―CONH[CH(CH₃)₂], ―CONH[C(CH₃)₃], ―CON(CH₃)₂, ―CON(C₂H₅)₂, ―CON(C₃H₇)₂, ―CON(cyclo―C₃H₅)₂, ―CON[CH(CH₃)₂]₂, ―CON[C(CH₃)₃]₂, ―NHCOCH₃, ―NHCOC₂H₅, ―NHCOC₃H₇, ―NHCO―cyclo-C₃H₅, ―NHCO―CH(CH₃)₂, ―NHCO―C(CH₃)₃, ―NHCO―OCH₃, ―NHCO―OC₂H₅, ―NHCO―OC₃H₇, ―NHCO―O―cyclo-C₃H₅, ―NHCO―OCH(CH₃)₂, ―NHCO―OC(CH₃)₃, ―NH₂, ―NHCH₃, ―NHC₂H₅, ―NHC₃H₇, ―NH-cyclo-C₃H₅, ―NHCH(CH₃)₂, ―NHC(CH₃)₃, ―N(CH₃)₂, ―N(C₂H₅)₂, ―N(C₃H₇)₂, ―N(cyclo-C₃H₅)₂, ―N[CH(CH₃)₂]₂, ―N[C(CH₃)₃]₂, ―SOCH₃, ―SOC₂H₅, ―SOC₃H₇, ―SO―cyclo-C₃H₅, ―SOCH(CH₃)₂, ―SOC(CH₃)₃, ―SO₂CH₃, ―SO₂C₂H₅, ―SO₂C₃H₇, ―SO₂―cyclo-C₃H₅, ―SO₂CH(CH₃)₂, ―SO₂C(CH₃)₃, ―SO₃H, ―SO₃CH₃, ―SO₃C₂H₅, ―SO₃C₃H₇, ―SO₃―cyclo-C₃H₅, ―SO₃CH(CH₃)₂, ―SO₃C(CH₃)₃, ―SO₂NH₂, ―OCF₃, ―OC₂F₅, ―O―COOCH₃, ―O―COOC₂H₅, ―O―COOC₃H₇, ―O―COO―cyclo-C₃H₅, ―O―COOCH(CH₃)₂, ―O―COOC(CH₃)₃, ―NH―CO―NH₂, ―NH―CO―NHCH₃, ―NH―CO―NHC₂H₅, ―NH―CO―NHC₃H₇, ―NH―CO―NH―cyclo-C₃H₅, ―NH―CO―NH[CH(CH₃)₂], ―NH―CO―NH[C(CH₃)₃], ―NH―CO―N(CH₃)₂, ―NH―CO―N(C₂H₅)₂, ―NH―CO―N(C₃H₇)₂, ―NH―CO―N(cyclo-C₃H₅)₂, ―NH―CO―N[CH(CH₃)₂]₂, ―NH―CO―N[C(CH₃)₃]₂, ―NH―CS―NH₂, ―NH―CS―NHCH₃, ―NH―CS―NHC₂H₅, ―NH―CS―NHC₃H₇, ―NH―CS―NH―cyclo-C₃H₅, ―NH―CS―NH[CH(CH₃)₂], ―NH―CS―NH[C(CH₃)₃], ―NH―CS―N(CH₃)₂, ―NH―CS―N(C₂H₅)₂, ―NH―CS―N(C₃H₇)₂, ―NH―CS―N(cyclo-C₃H₅)₂, ―NH―CS―N[CH(CH₃)₂]₂, ―NH―CS―N[C(CH₃)₃]₂, ―NH_{2*}HOOCCF₃, ―CH₂F, ―CF₂Cl, ―CHF₂, ―CF₃, ―CH₂Cl, ―CH₂Br, ―CH₂l, ―CH₂―CH₂F, ―CH₂―CHF₂, ―CH₂―CF₃, ―CH₂―CH₂Cl, ―CH₂―CH₂Br, ―CH₂―CH₂l, cyclo-C₃H₅, cyclo-C₄H₇, cyclo-C₅H₉, cyclo-C₆H₁₁, cyclo-C₇H₁₃, cyclo-C₈H₁₅, ―Ph, ―CH₂―Ph, ―CPh₃, ―CH₃, ―C₂H₅, ―C₃H₇, ―CH(CH₃)₂, ―C₄H₉, ―CH₂―CH(CH₃)₂, ―CH(CH₃)―C_{2H5}, ―C(CH₃)₃, ―C₅H₁₁, ―CH(CH₃)―C₃H₇, ―CH₂―CH(CH₃)―C₂H₅, ―CH(CH₃)―CH(CH₃)₂, ―C(CH₃)₂―C₂H₅, ―CH₂―C(CH₃)₃, ―CH(C₂H₅)₂, ―C₂H₄―CH(CH₃)₂, ―C₆H₁₃, ―C₃H₆―CH(CH₃)₂, ―C₂H₄―CH(CH₃)―C₂H₅, ―CH(CH₃)―C₄H₉, ―CH₂―CH(CH₃)―C₃H₇, ―CH(CH₃)―CH₂―CH(CH₃)₂, ―CH(CH₃)―CH(CH₃)―C₂H₅, ―CH₂―CH(CH₃)―CH(CH₃)₂, ―CH₂―C(CH₃)₂―C₂H₅, ―C(CH₃)₂―C₃H₇, ―C(CH₃)₂―CH(CH₃)₂, ―C₂H₄―C(CH₃)₃, ―CH(CH₃)―C(CH₃)₃, ―CH=CH₂, ―CH₂―CH=CH₂, ―C(CH₃)=CH₂, ―CH=CH―CH₃, ―C₂H₄―CH=CH₂, ―C₇H₁₅, ―C₈H₁₇, ―CH₂―CH=CH―CH₃, ―CH=CH―C₂H₅, ―CH₂―C(CH₃)=CH₂, ―CH(CH₃)―CH=CH, ―CH=C(CH₃)₂, ―C(CH₃)=CH―CH₃, ―CH=CH―CH=CH₂, ―C₃H₆―CH=CH₂, ―C₂H₄―CH=CH―CH₃, ―CH₂―CH=CH―C₂H₅, ―CH=CH―C₃H₇, ―CH₂―CH=CH―CH=CH₂, ―CH=CH―CH=CH―CH₃, ―CH₂NH₂, ―CH₂OH, ―CH₂SH, ―CH₂―CH₂NH₂, ―CH₂―CH₂SH, ―C₆H₄―OCH₃, ―C₆H₄―OH, ―CH₂―CH₂―OCH₃, ―CH₂―CH₂OH, ―CH₂―OCH₃, ―CH₂―C₆H₄―OCH₃, ―CH₂―C₆H₄―OH, ―C≡CH, ―C≡C―CH₃, ―CH₂-C≡CH, ―C₂H₄―C≡CH, -CH₂-C≡C-CH₃, ―C≡C―C₂H₅, ―C₃H₆―C≡CH, ―C₂H₄―C≡C―CH₃, ―CH₂―C≡C―C₂H₅, ―C≡C―C₃H₇, 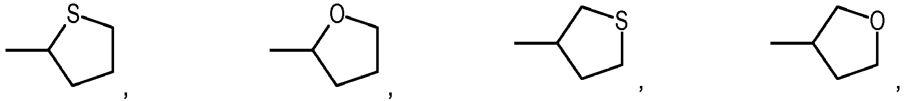 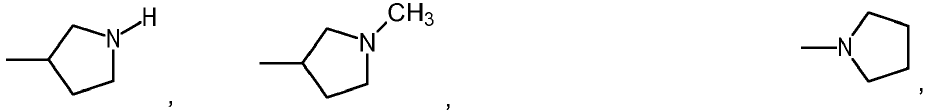 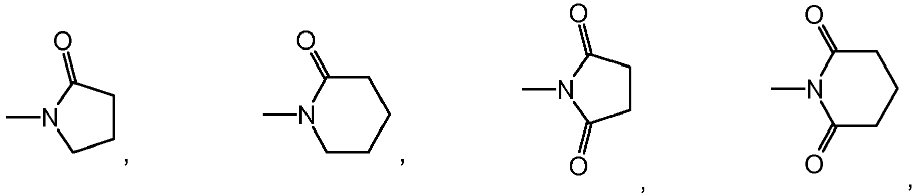 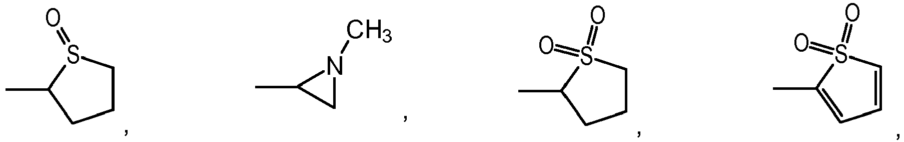 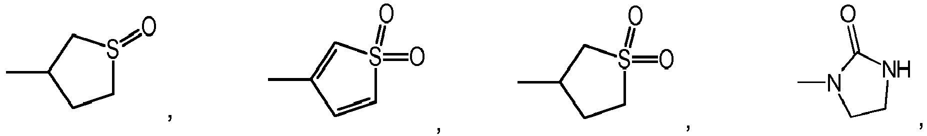 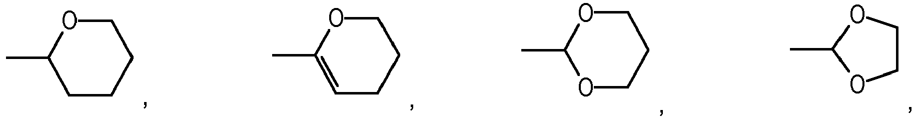 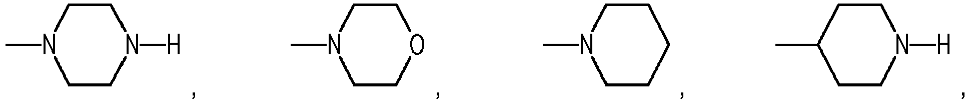 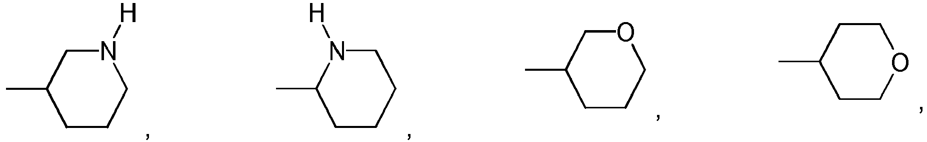 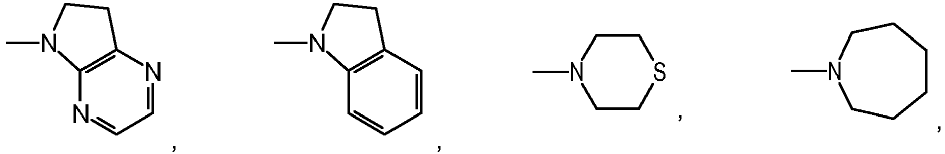 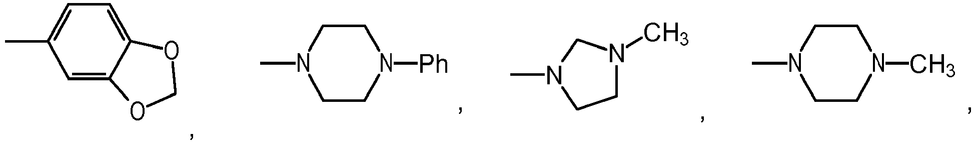 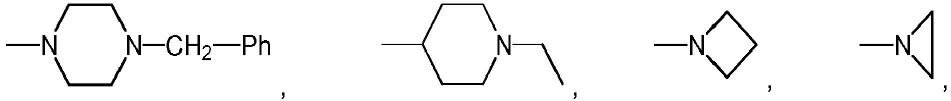 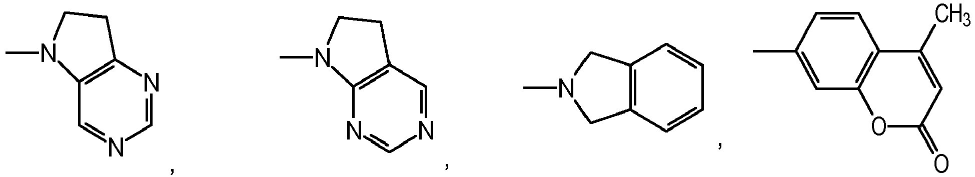 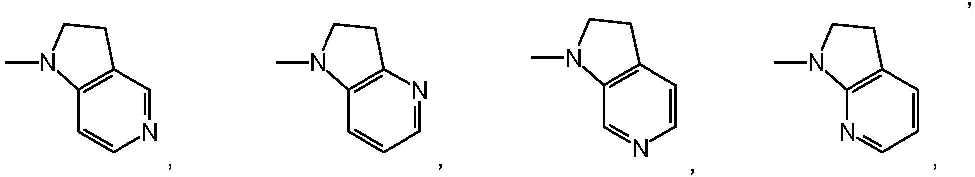 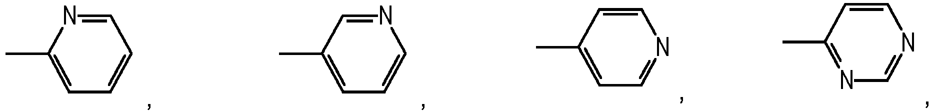 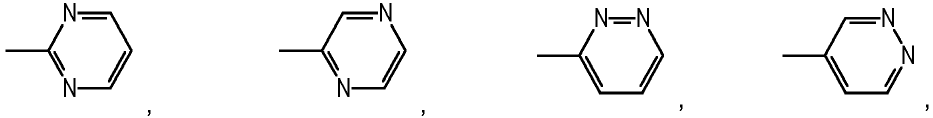 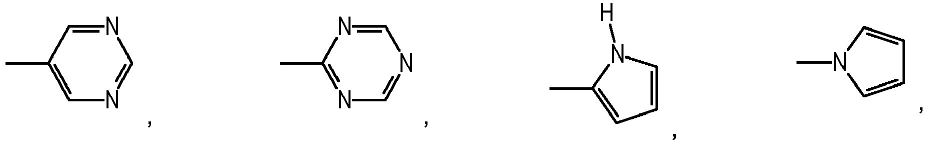 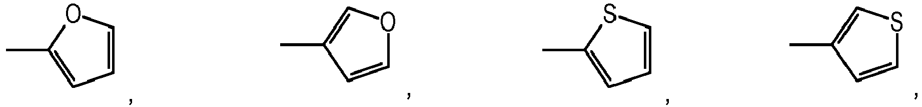 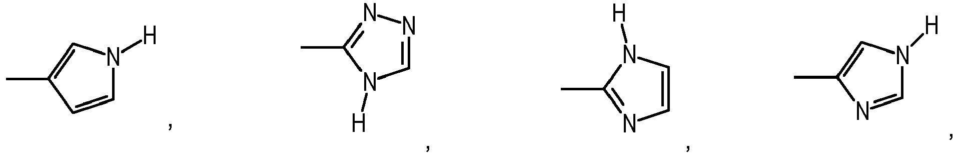 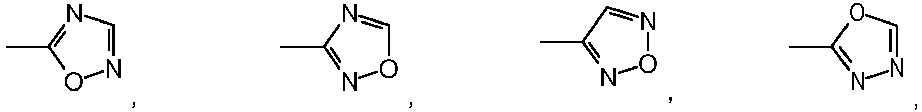 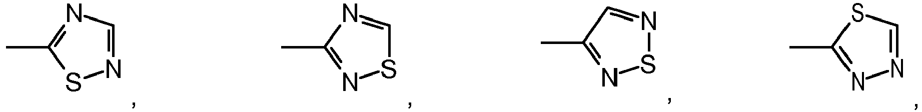 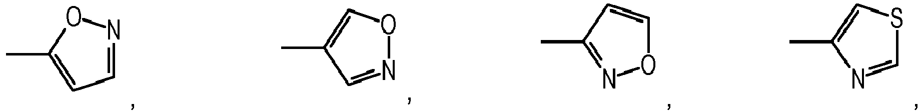 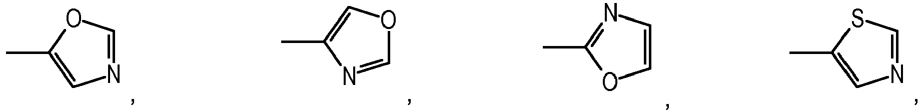 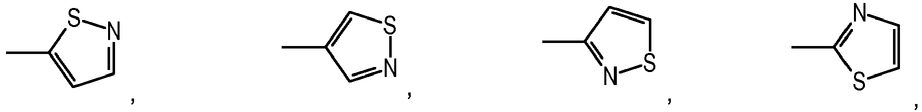 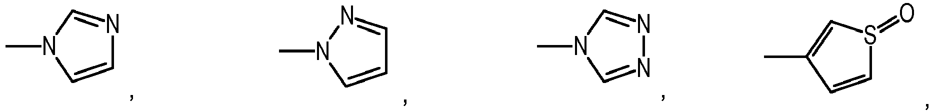 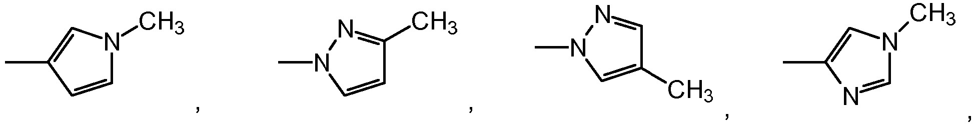 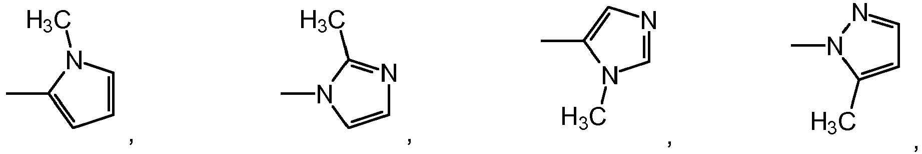 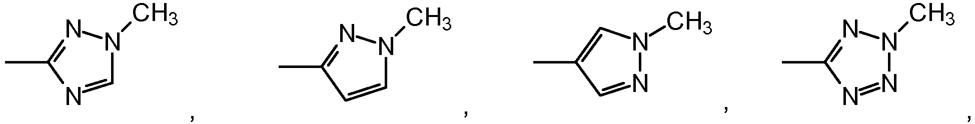 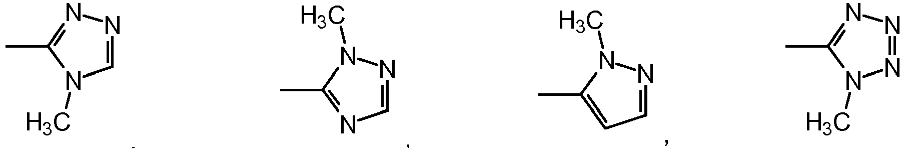 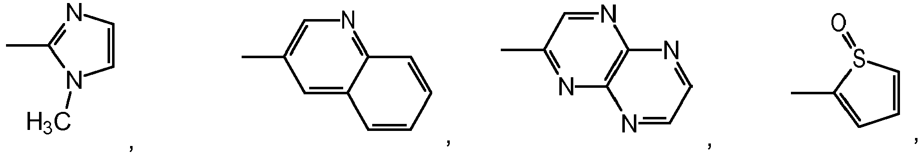 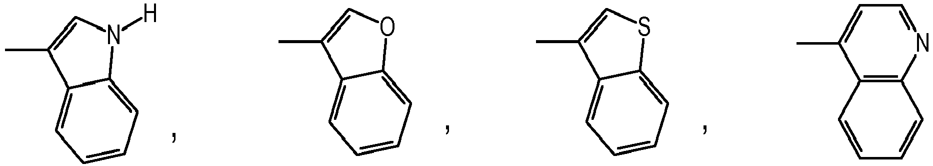 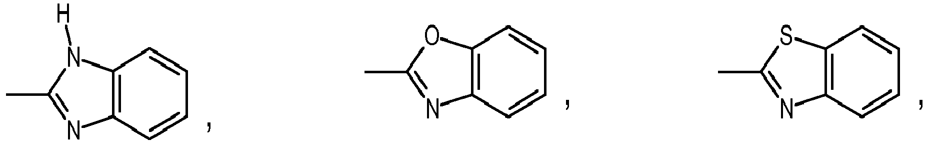 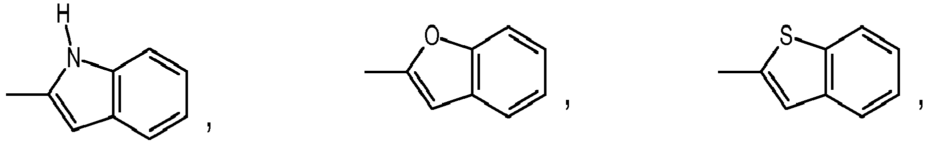 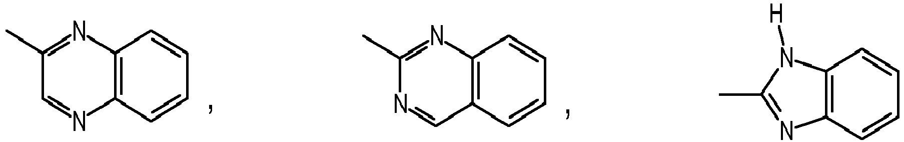 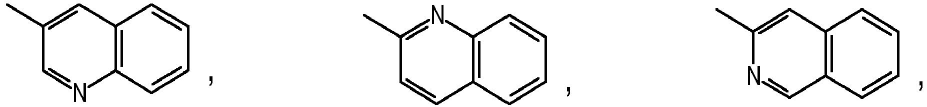 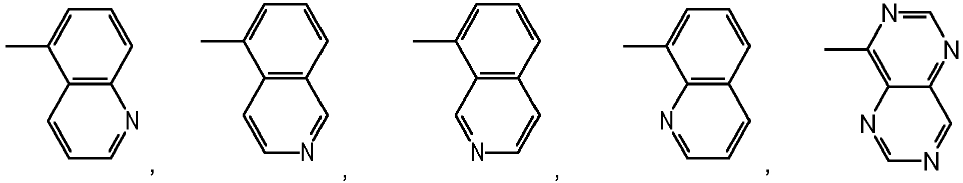 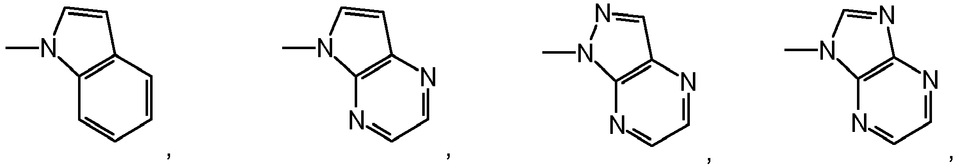 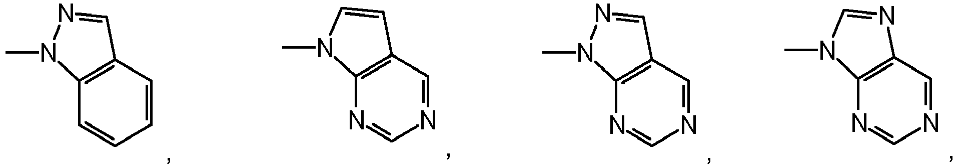 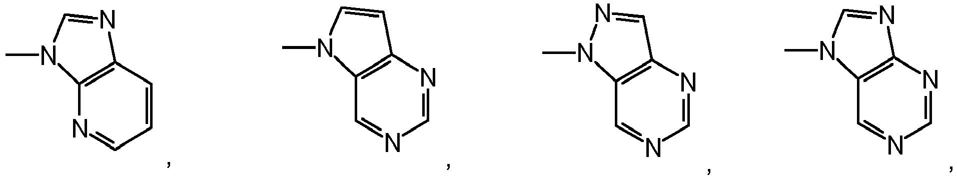 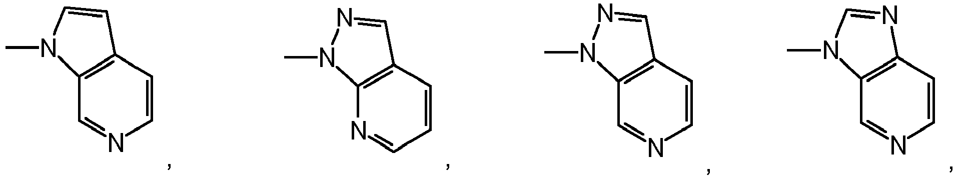 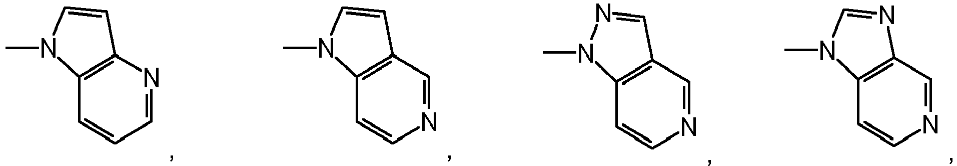 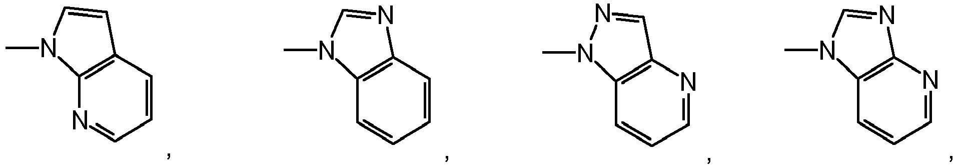 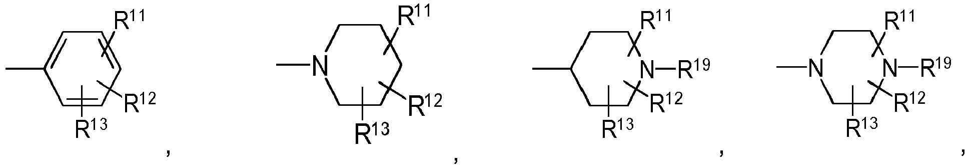 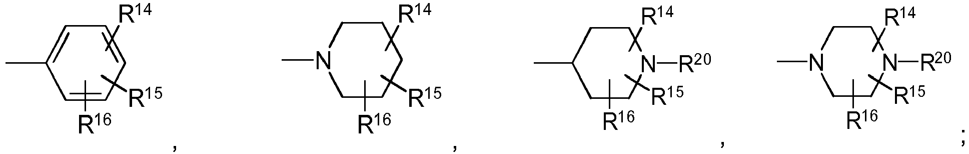 die Reste **R¹¹** bis **R¹⁶** unabhängig voneinander für folgende Gruppen stehen: ―H, ―NH₂, ―OH, ―OCH₃, ―OC₂H₅, ―OC₃H₇, ―OCF₃, ―CF₃, ―F, ―Cl, ―Br, ―I, ―CH₃, ―C₂H₅, ―C₃H₇, ―CH(CH₃)₂, ―Ph und ―CN;
sowie stereoisomere Formen, E/Z-Isomere, Enantiomere, Enantiomerengemische, Diastereomere, Diastereomerengemische, Racemate, Tautomere, Anomere, Keto-Enol-Formen, Betainformen, Prodrugs, Solvate, Hydrate als auch pharmakologisch verträgliche Salze der vorgenannten Verbindungen.

Unter dem Begriff "Prodrug" wird eine Wirkstoffvorstufe verstanden, welche eine Verbindung gemäß allgemeiner Formal (I) bis (VI) beinhaltet, welche zudem unter physiologischen Bedingungen abspaltbare Gruppen umfasst oder welche eine Verbindung gemäß allgemeiner Formal (I) bis (VI) unter physiologischen Bedingungen freisetzt.

Überraschenderweise wurde gefunden, dass die erfindungsgemäßen Verbindungen der allgemeinen Formel (I) außergewöhnliches Potential aufweisen zur Inhibition von Gewebetransglutamase. Zudem zeigen die erfindungsgemäßen Pyridinonderivate Selektivität für die TG2. Dabei hat sich ferner gezeigt, dass die erfindungsgemäßen Verbindungen zur Entfaltung ihres Potentials eine große Toleranz gegenüber den Substituenten am N-terminalen Ende ("X"-Ligand) aufweisen. Aufgrund dieser Toleranz ist es möglich, den Verbindungen der vorliegenden Erfindung durch gezielte Verwendung von geeigneten funktionellen Gruppen spezielle Eigenschaften zu verleihen, wie beispielsweise ein gewünschtes pH-abhängiges Löslichkeitsprofil, Löslichkeit, Polarität und der gleichen. Somit ist es möglich über den N-terminalen Substituenten die erfindungsgemäßen Verbindungen basisch, sauer oder neutral einzustellen. Ferner kann durch die Toleranz gegenüber den N-terminalen Substituenten die Löslichkeit, Membrangängigkeit (Membranpermeabilität), Stabilität gegenüber Mikrosomen und generelle Verträglichkeit positiv beeinflusst werden.

Durch die speziell ausgewählten Substituenten auf der C-terminalen Seite der erfindungsgemäßen Verbindungen kann das sterische Ausmaß so sehr genau eingestellt werden, dass eine Bindungstasche eines gewünschten Zielmoleküls im hohen und übereinstimmenden Maße adressiert werden kann. Es wurde überraschender Weise gefunden, dass durch die Verbindungen der vorliegenden Erfindung selektiv die Gewebetransglutamase inhibiert werden kann.

Zudem scheint der 2-Oxo-1,2-dihydropyridinrest bzw. der 6-Alkyl-2-oxo-1,2-dihydropyridinrest eine wichtige Aufgabe dahingehend zu erfüllen, die akzeptorsubstituierte Doppelbindung der Seitenkette derart im aktiven Zentrum der TG2 zu platzieren, dass eine Thiolfunktion einer Cysteinaminosäure der TG2 an das Michaelsystem addieren kann. Aus diesem Grund ist es auch wichtig, dass die Aminosäure in den erfindungsgemäßen Pyridinonderivaten, welche die Seitenkette mit dem Michaelsystem trägt, über eine Aminogruppe an Position 3 des Pyridinonrestes gebunden ist. Des weiteren scheint für eine sehr gute inhibitorische Wirkung die Anbindung einer Methylcarbamoylgruppe an den Ringstickstoff des Pyridinonrestes bevorzugt zu sein. Ferner wurde gefunden, dass ein bestimmter Abstand zwischen der akzeptorsubstituierten Doppelbindung und dem alpha-Kohlenstoffatom der Aminosäure bestehen muss, woran die Seitenkette mit dem Michaelsystem gebunden ist. Dieser optimale Abstand, der nicht überschritten aber auch nicht unterschritten werden sollte, wird durch den Ethylenyl-Linker (―CH₂―CH₂―) erreicht. Methylenyl-Linker (―CH₂―) als auch Propylenyl-Linker (―CH₂―CH₂―CH₂―) liefern schlechtere Inhibitionswerte.

Bevorzugt sind Verbindungen der allgemeinen Formel (I), (II), (III), (IV), (V) oder (VI), worin **Z** für einen der folgenden Reste steht: ―CH₃, ―C₂H₅, ―C₃H₇, ―CH(CH₃)₂, ―OCH₂―Ph, ―OCH₃, ―OC₂H₅, ―OC₃H₇, ―OCH(CH₃)₂, ―OCH=CH₂ oder ―OCH₂―CH=CH₂, weiter bevorzugt für ―CH₃, ―C₂H₅, ―C₃H₇, ―OCH₃, -OC₂H₅, ―OC₃H₇, ―OCH(CH₃)₂ oder ―OCH₂―Ph, noch weiter bevorzugt für ―CH₃, ―C₂H₅, ―OCH₃, ―OC₂H₅ oder ―OCH₂―Ph, noch weiter bevorzugt für ―OCH₃, ―OC₂H₅ oder ―OCH₂―Ph, noch weiter bevorzugt für ―OCH₃ oder ―OC₂H₅ und insbesondere bevorzugt für ―OCH₃.

Ferner sind Verbindungen der allgemeinen Formel (I), (II), (III), (IV), (V) oder (VI) bevorzugt, worin **R#** für ―NHY steht.

Als Reste **Y** und vor allem als Reste **Y** in dem Rest ―NHY sind bevorzugt ―CHR¹― CH₂R², ―CHR¹―CHR²―CH₂R³, ―CHR¹―CHR²―CHR³―CH₂R³, ―CHR¹―CHR²―CHR³― CHR⁴―CH₂R⁵ und ―CHR¹―CHR²―CHR³―CHR⁴―CHR⁵―CH₂R⁶, weiter bevorzugt ―CHR¹―CHR²―CH₂R³, ―CHR¹―CHR²―CHR³―CH₂R⁴ und ―CHR¹―CHR²―CHR³― CHR⁴―CH₂R⁵, noch weiter bevorzugt ―CHR¹―CHR²―CH₃, ―CHR¹―CHR²―CHR³―CH₃ und ―CHR¹―CHR²―CHR³―CH₂―CH₃ und am meisten bevorzugt ―CHR¹―CH₂―CH₃, ―CH₂―CHR²―CH3, ―CH₂―CHR²―CH₂―CH₃, ―CHR¹―CH₂―CH₂―CH₃, ―CH₂―CH₂― CHR³―CH₃, ―CH₂―CH₂―CHR³―CH₂―CH₃ und ―CH₂―CHR²―CH₂―CH₂―CH₃.

Allgemein und insbesondere in dem vorgenannten Rest Y bedeuten **R¹** ― **R⁶** unabhängig voneinander vorzugsweise ―H, ―OCH₃, ―OC₂H₅, ―OC₃H₇, ―CH₂F, ―CHF₂, ―CF₃, ―CH₂Cl, ―CH₂―CH₂F, ―CH₂―CHF₂, ―CH₂―CF₃, ―CH₃, ―C₂H₅, ―C₃H₇, ―CH(CH₃)₂ und ―C₄H₉, weiter bevorzugt ―H, ―CH₂F, ―CF₃, ―CH₂―CH₂F, ―CH₂―CF₃, ―CH₃, ―C₂H₅, ―C₃H₇, ―CH(CH₃)₂ und ―C₄H₉ noch weiter bevorzugt ―H, ―CH₂F, ―CH₂―CH₂F, ―CH₃, ―C₂H₅, ―C₃H₇ und ―CH(CH₃)₂, noch weiter bevorzugt ―H, ―CH₃, ―C₂H₅, ―C₃H₇ und am meisten bevorzugt ―H und ―C₂H₅.

Als Rest ―NHY sind bevorzugt: ―NH(CH(CH₃)―CH₂―CH₃), ―NH(CH₂―CH(CH₃)₂), ―NH(CH(C₂H₅)₂), ―NH(CH(CH₃)―CH₂―CH₂―CH₃), ―NH(CH₂―CH(CH₃)―CH₂―CH₃), ―NH(CH₂CH₂―CH(CH₃)₂), ―NH(CH(C₂H₅)―CH₂―CH₂―CH₃), ―NH(CH₂―CH(C₂H₅)₂), ―NH(CH₂―CH₂―CH(CH₃)―C₂H₅), ―NH(CH(C₃H₇)₂) und NH(CH₂―CH₂―CH(C₂H₅)₂).

Ferner sind Verbindungen der allgemeinen Formel (I) bevorzugt, worin
**Z** für ―OCH₃, ―OCH₂CH₃, ―OCH(CH₃)₂ oder ―OCH₂―Ph steht und **R^{#}** für ―**NHY** steht.

Ferner sind Verbindungen der allgemeinen Formel (I) bevorzugt, worin
**Z** für ―OCH₃, ―OCH₂CH₃, ―OCH(CH₃)₂ oder ―OCH₂―Ph steht und **R^{#}** für ―**NHY** steht,
**Y** für ―CH₂R¹, ―CHR¹―H₂R², ―CHR¹―CHR²―CH₂R³, ―CHR¹―CHR²―CHR³―CH₂R⁴, oder ―CHR¹―CHR²―CHR³―CHR⁴―CH₂R⁵ steht, und
R¹ bis R⁵ unabhängig voneinander aus den folgenden Substituenten ausgewählt sind: ―COOH, ―COOCH₃, ―N(CH₃)₂, ―N(C₂H₅)₂, ―Ph, ―CH₃, ―C₂H₅, CH₂―CH(CH₃)₂, ―C(CH₃)₃,

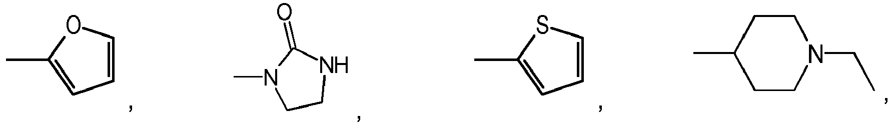

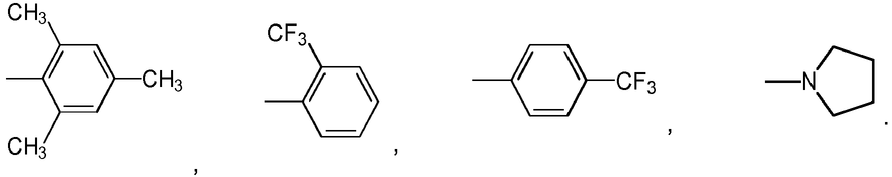

Desweiteren sind Verbindungen der allgemeinen Formel (I) bevorzugt, worin
**Z** für ―OCH₃, ―OCH₂CH₃, ―OCH(CH₃)₂ oder ―OCH₂―Ph steht, **R^{#}** für ―**NHY** steht, und
**Y** ausgewählt wird aus ―CH₃, ―C₂H₅, ―C₃H₇, ―C₄H₉ ―C₅H₁₁, ―C₂H₄―CH(CH₃)₂, ―CH(CH₃)―CH₂―CH(CH₃)₂, ―CH₂―CH(C₂H₅)₂, ―CH₂―CH(CH₃)―C₂H₅, ―C₂H₄―C(CH₃)₃, C₂H₄―CH(C₂H₅)₂, 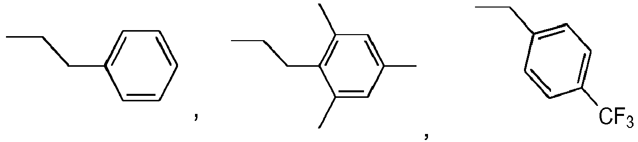 und

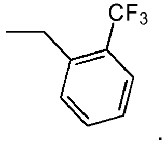

Desweiteren sind Verbindungen der allgemeinen Formel (I) bevorzugt, worin
**X** für ―CH₂R⁷, CHR⁷―CH₂R⁸, 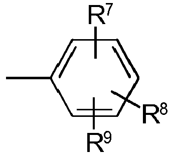 oder 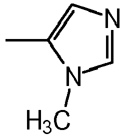 steht.

Ebenfalls bevorzugt sind Verbindungen der allgemeinen Formel (I), worin
**Z** für -OCH₃, ―OCH₂CH₃ oder ―OCH₂―Ph steht,
**X** für 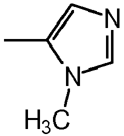 steht
**R^{#}** für ―NHY steht,
**Y** ausgewählt wird aus ―CH₃, ―C₂H₅, ―C₃H₇, ―C₄H₉, ―C₅H₁₁, ―C₂H₄―CH(CH₃)₂, ―CH(CH₃)―CH₂―CH(CH₃)₂, ―CH₂―CH(C₂H₅)₂, ―CH₂―CH(CH₃)―C₂H₅, ―C₂H₄―C(CH₃)₃, C₂H₄―CH(C₂H₅)₂, 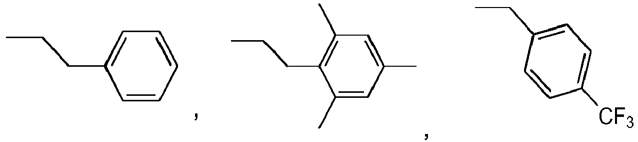 und

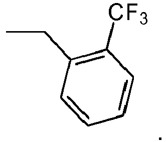

Gleichfalls bevorzugt sind Verbindungen der allgemeinen Formel (I), worin **Z** für ―OCH₃, ―OCH₂CH₃ oder ―OCH₂―Ph steht,
**X** für ―CH₂R⁷ oder ―CHR⁷―CH₂R⁸ steht
**R^{#}** für ―NHY steht,
**Y** ausgewählt wird aus ―CH₃, ―C₂H₅, ―C₃H₇, ―C₄H₉, ―C₅H₁₁, ―C₂H₄―CH(CH₃)₂, ―CH(CH₃)―CH₂―CH(CH₃)₂, ―CH₂―CH(C₂H₅)₂, ―CH₂―CH(CH₃)―C₂H₅, ―C₂H₄―C(CH₃)₃, ―C₂H₄―CH(C₂H₅)₂, 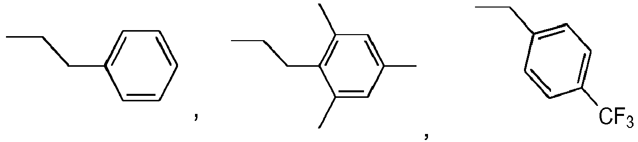 und

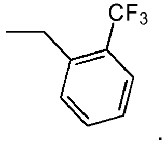

Darüber hinaus sind Verbindungen der allgemeinen Formel (I) bevorzugt, worin
**Z** für ―OCH₃, ―OCH₂CH₃ oder ―OCH₂―Ph steht,
**X** für 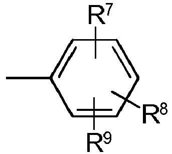 steht,
**R^{#}** für ―NHY steht,
**Y** ausgewählt wird aus ―CH₃, ―C₂H₅, ―C₃H₇, ―C₄H₉, ―C₅H₁₁, ―C₂H₄―CH(CH₃)₂, ―CH(CH₃)―CH₂―CH(CH₃)₂, ―CH₂―CH(C₂H₅)₂, ―CH₂―CH(CH₃)―C₂H₅, ―C₂H₄―C(CH₃)₃, ―C₂H₄―CH(C₂H₅)₂, 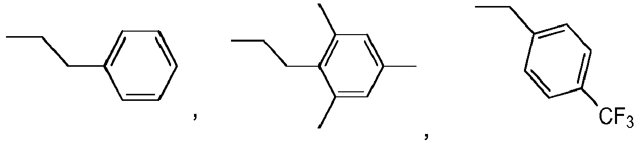 und

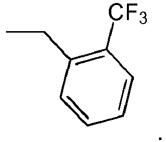

Ferner sind im besonderen Maße Verbindungen der allgemeinen Formel (I) bevorzugt, worin
**Z** für -OCH₃, ―OCH₂CH₃ oder ―OCH₂―Ph steht,
**E¹** für ―CO― oder ―SO₂― steht,
**E²** für ―CO― steht
**R^{#}** für einen der folgenden Reste steht: ―NYY', 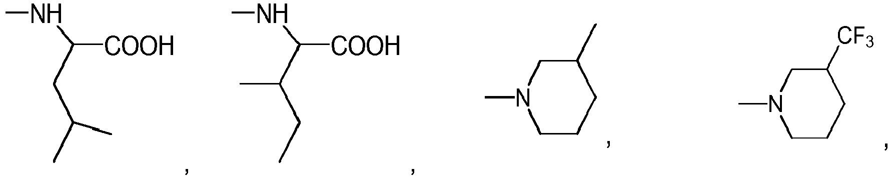 oder 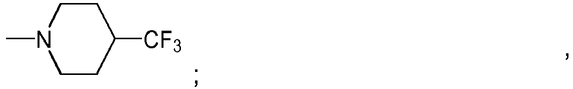 **Y'** für ―H steht;
**Y** für ―CH₂R¹, ―CHR¹―CH₂R², ―CHR¹―CHR²―CH₂R³, ―CHR¹―CHR²―CHR³―CH₂R⁴ oder ―CHR¹―CHR²―CHR³―CHR⁴―CH₂R⁵ steht;
**R¹** bis **R⁵** unabhängig voneinander ausgewählt werden aus: ―H, ―CH₃, ―C₂H₅, ―Ph, ―CH₂―CH(CH₃)₂, ―N(CH₃)₂, ―N(C₂H₅)₂, ―COOH, ―COOCH₃, ―C(CH₃)₃, 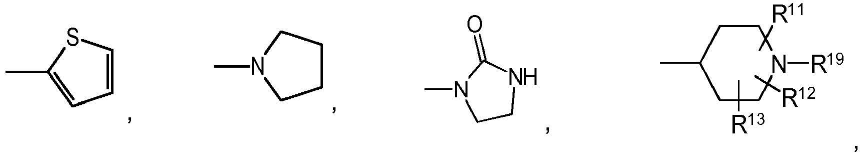 und 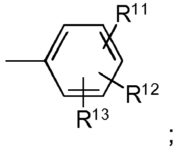 **R¹¹** bis **R¹³** unabhängig voneinander ―H, ―CH₃, ―C₂H₅ oder ―CF₃ bedeuten, **R¹⁹** ―C₂H₅ bedeutet,
**X** ausgewählt wird aus ―CH₂R⁷, ―CHR⁷―CH₂R⁸, ―O―CH₂R⁷, 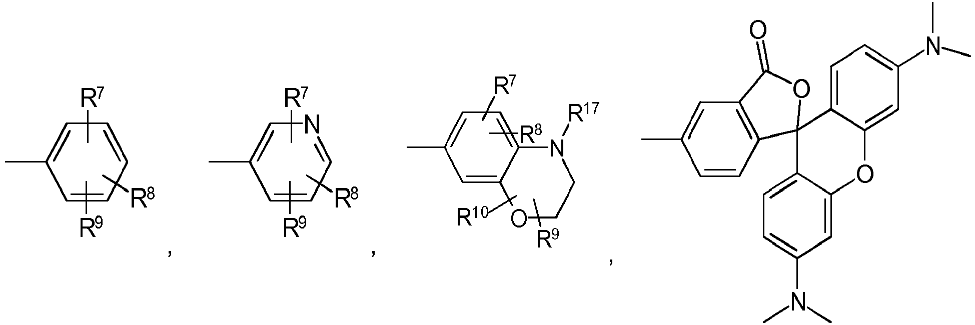 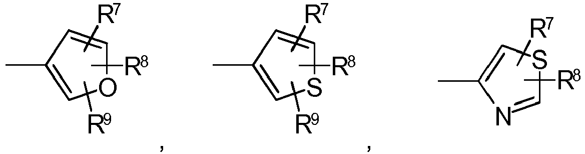 und 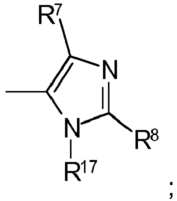 **R⁷** bis **R¹⁰** unabhängig voneinander bedeuten ―H, ―OH, ―COOH, ―SO₂NH₂, ―CH₃, ―CF₃, ―NH―CO―NH₂, ―NH_{2*}HOOCCF₃, ―NH₂, 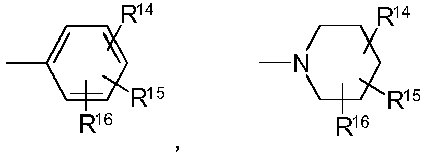 oder 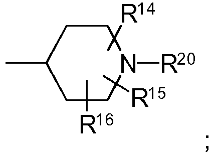 **R¹⁴** bis **R¹⁶** unabhängig voneinander ―H, ―Cl oder ―CH₃, bedeuten, und
**R¹⁷** und **R²⁰** ―CH₃ bedeuten.

Ferner sind besonders bevorzugt Verbindungen der allgemeinen Formel (I), worin
**Z** für ―OCH₃ steht,
**E²** für ―CO― steht,
**R^{#}** für für ―NHY steht,
**Y** für ―C₂H₄―CH(CH₃)₂, ―CH₂―CH(CH₃)―C₂H₅, ―C₂H₄―C(CH₃)₃ oder ―C₂H₄―CH(C₂H₅)₂ steht,
**X** einen der folgenden Reste bedeutet ―CH₂R⁷, ―CHR⁷―CH₂R⁸, ―O―CH₂R⁷, 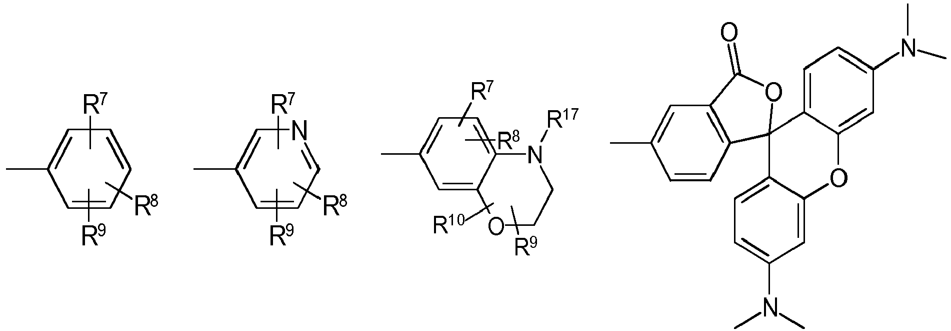 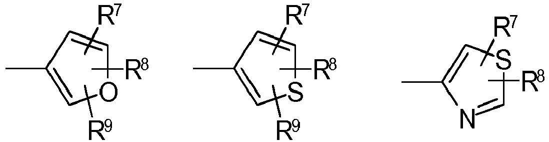 oder 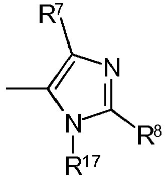 **R⁷** bis **R¹⁰** unabhängig voneinander bedeuten ―H, ―OH, ―COOH, ―SO₂NH₂, ―CH₃, ―CF₃, ―NH―CO―NH₂, ―NH₂*HOOCCF₃, ―NH₂, 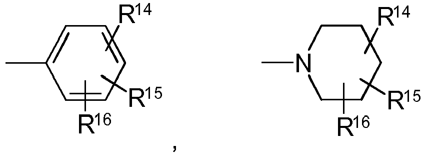 oder 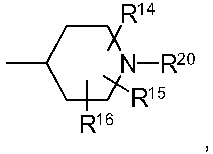 **R¹⁷** ―CH₃ bedeutet
**R¹⁴** bis **R¹⁶** unabhängig voneinander ―H, ―Cl oder ―CH₃ bedeuten, und
**R²⁰** ―C₂H₅ bedeutet.

Des weiteren wird R^{#} vorzugsweise ausgewählt aus folgenden Resten: 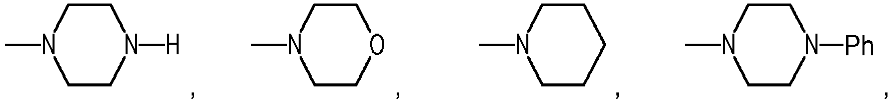 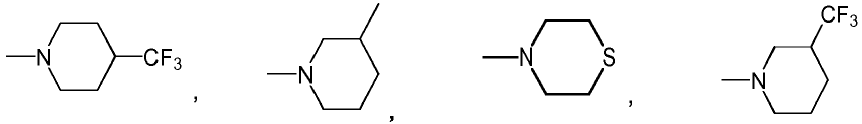 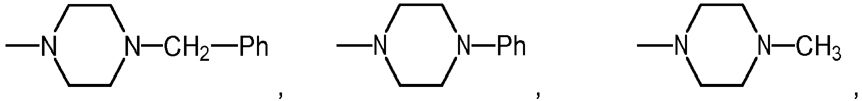 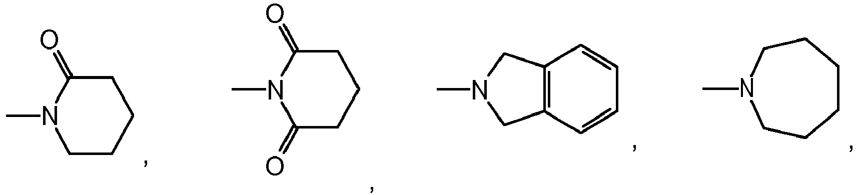 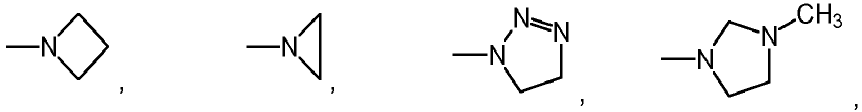 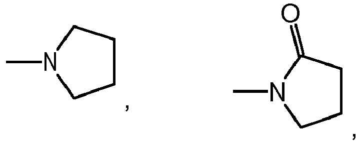 und

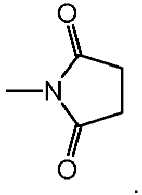

Besonders bevorzugt sind aus den vorgenannten Resten für R^{#} die folgenden Reste: 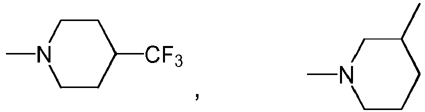 und

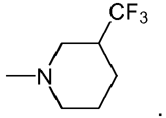

Eine bevorzugte Ausführungsform der vorliegenden Erfindung betrifft Verbindungen der allgemeinen Formel (II): 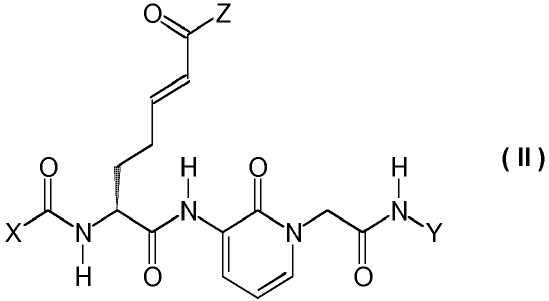 worin
**X, Y** und **Z** die Beduetung haben wie oben definiert und vorzugsweise
**Z** für ―OCH₃, ―OCH₂CH₃, ―OCH(CH₃)₂ oder ―OCH₂―Ph steht.

Ebenfalls sind Verbindungen der allgemeinen Formel (III) bevorzugt 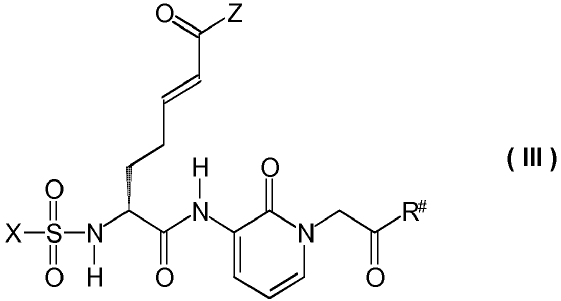 mit **X, Z** und **R^{#}** wie hierin definiert.

Ebenfalls sind Verbindungen der allgemeinen Formeln (IV), (V) und (VI) bevorzugt: 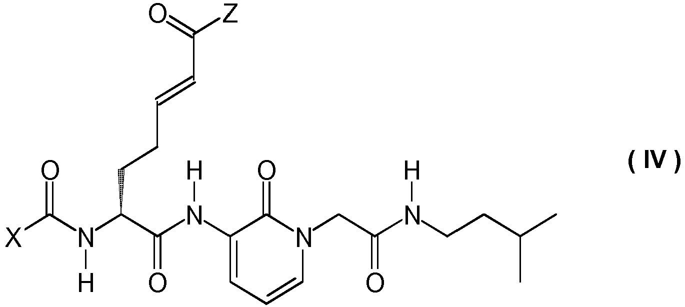 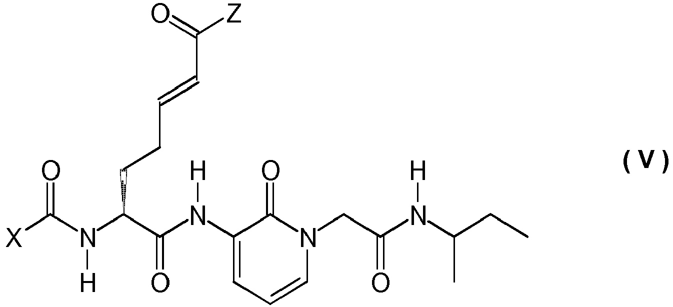 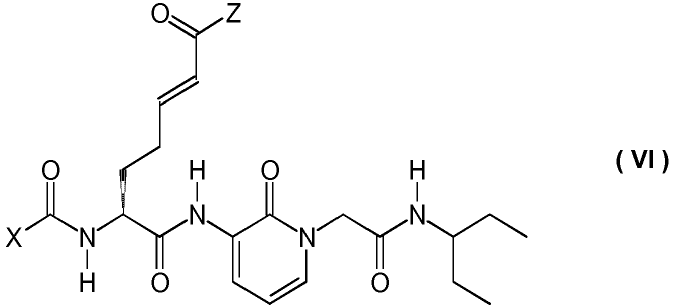 mit **X** und **Z** wie hierin definiert, bevorzugt mit Z als ―OCH₃, ―OCH₂CH₃ oder ―OCH₂―Ph und noch mehr bevorzugt mit Z als ―OCH₃.

Gemäß der vorliegenden Verbindung sind Verbindungen insbesondere bevorzugt, die ausgewählt sind aus der Gruppe bestehend aus:

| |
|---|
| (S,E)-Ethyl 6-(benzyloxycarbonylamino)-7-oxo-7-(2-oxo-1-(2-oxo-2-(2,4,6-trimethylphenethylamino)ethyl)-1,2-dihydropyridin-3-ylamino)hept-2-enoate (A1) |
| (S,E)-Ethyl 6-(benzyloxycarbonylamino)-7-(1-(2-(isopentylamino)-2-oxoethyl)-6-methyl-2-oxo-1,2-dihydropyridin-3-ylamino)-7-oxohept-2-enoate (A2) |
| (S,E)-Ethyl 6-(benzyloxycarbonylamino)-7-(1-(2-(isopentylamino)-2-oxoethyl)-2-oxo-1,2-dihydropyridin-3-ylamino)-7-oxohept-2-enoate (A3) |
| (S,E)-Ethyl 6-acetamido-7-(1-(2-(isopentylamino)-2-oxoethyl)-6-methyl-2-oxo-1,2-dihydropyridin-3-ylamino)-7-oxohept-2-enoate (A4) |
| 3-(2-(3-((S,E)-2-(Benzyloxycarbonylamino)-7-ethoxy-7-oxohept-5-enamido)-6-methyl-2-oxopyridin-1(2H)-yl)acetamido)-5-methylhexanoic acid (A5) |
| (S, E)-isopropyl 6-acetamido-7-(1-(2-(isopentylamino)-2-oxoethyl)-2-oxo-1,2-dihydro-pyridin-3-ylamino)-7-oxohept-2-enoate (A6) |
| (S,E)-Ethyl 6-acetam ido-7-(1-(2-(isopentylam ino)-2-oxoethyl)-2-oxo-1,2-d ihydro-pyridin-3-ylamino)-7-oxohept-2-enoate (A7) |
| (S,E)-Ethyl 7-(6-methyl-2-oxo-1-(2-oxo-2-(phenethylamino)ethyl)-1,2-dihydropyridin-3-ylamino)-6-(nicotinamido)-7-oxohept-2-enoate (A8) |
| (S,E)-ethyl 6-((4-chlorophenyl)methylsulfonamido)-7-(1-(2-(isopentylamino)-2-oxo-ethyl)-2-oxo-1,2-dihydropyridin-3-ylamino)-7-oxohept-2-enoate (A9) |
| (S,E)-Ethyl 6-benzamido-7-(1-(2-(isopentylamino)-2-oxoethyl)-2-oxo-1,2-dihydro-pyridin-3-ylamino)-7-oxohept-2-enoate (A10) |
| (S,E)-Ethyl 6-(furan-3-carboxamido)-7-(1-(2-(isopentylamino)-2-oxoethyl)-2-oxo-1,2-dihydropyridin-3-ylamino)-7-oxohept-2-enoate (A11) |
| (S,E)-Ethyl 7-(1-(2-(isopentylamino)-2-oxoethyl)-2-oxo-1,2-dihydropyridin-3-ylamino)-7-oxo-6-(thiophene-3-carboxamido)hept-2-enoate (A12) |
| (S,E)-Ethyl 6-(furan-3-sulfonamido)-7-(1-(2-(isopentylamino)-2-oxoethyl)-2-oxo-1,2-dihydropyridin-3-ylamino)-7-oxohept-2-enoate (A13) |
| (S,E)-Ethyl 7-(1-(2-(isopentylamino)-2-oxoethyl)-2-oxo-1,2-dihydropyridin-3-ylamino)-7-oxo-6-(4-sulfamoylbenzamido)hept-2-enoate (A14) |
| (S,E)-Ethyl 7-(1-(2-(isopentylamino)-2-oxoethyl)-2-oxo-1,2-dihydropyridin-3-ylamino)-6-(5-methylthiazole-4-carboxamido)-7-oxohept-2-enoate (A15) |
| (S,E)-Ethyl 7-(1-(2-(isopentylamino)-2-oxoethyl)-2-oxo-1,2-dihydropyridin-3-ylamino)-6-(nicotinamido)-7-oxohept-2-enoate (A16) |
| (S,E)-Ethyl 6-(3,5-bis(trifluoromethyl)benzamido)-7-(1-(2-(isopentylamino)-2-oxoethyl)-2-oxo-1,2-dihydropyridin-3-ylamino)-7-oxohept-2-enoate (A17) |
| (S,E)-Ethyl 7-(1-(2-(isopentylamino)-2-oxoethyl)-2-oxo-1,2-dihydropyridin-3-ylamino)-7-oxo-6-(4-(piperidin-1-yl)benzamido)hept-2-enoate (A18) |
| (S,E)-Ethyl 7-(1-(2-(isopentylamino)-2-oxoethyl)-2-oxo-1,2-dihydropyridin-3-ylamino)-6-(1-methyl-1H-imidazole-5-carboxamido)-7-oxohept-2-enoate (A19) |
| (S,E)-Ethyl 7-(1-(2-(isopentylamino)-2-oxoethyl)-2-oxo-1,2-dihydropyridin-3-ylamino)-6-(4-(4-methylpiperazin-1-yl)benzamido)-7-oxohept-2-enoate (A20) |
| (S,E)-Ethyl 7-(1-(2-(isopentylamino)-2-oxoethyl)-2-oxo-1,2-dihydropyridin-3-ylamino)-6-(4-methyl-3,4-dihydro-2H-benzo[b][1,4]oxazine-7-carboxamido)-7-oxohept-2-enoate (A21) |
| (S,E)-Ethyl 7-(1-(2-(isopentylamino)-2-oxoethyl)-2-oxo-1,2-dihydropyridin-3-ylamino)-7-oxo-6-(phenylsulfonamido)hept-2-enoate (A22) |
| (S,E)-5-(N-(7-Ethoxy-1-(1-(2-(isopentylamino)-2-oxoethyl)-2-oxo-1,2-dihydropyridin-3-ylamino)-1,7-dioxohept-5-en-2-yl)sulfamoyl)-2-hydroxybenzoic acid (A23) |
| (S,E)-4-(7-Ethoxy-1-(1-(2-(isopentylamino)-2-oxoethyl)-2-oxo-1,2-dihydropyridin-3-ylamino)-1,7-dioxohept-5-en-2-ylamino)-4-oxobutanoic acid (A24) |
| (S,E)-Ethyl 6-acetamido-7-(1-(2-(2-(diethylamino)ethylamino)-2-oxoethyl)-2-oxo-1,2-dihydropyridin-3-ylamino)-7-oxohept-2-enoate (A25) |
| (S,E)-4-(1-(1-(2-(2-(Diethylamino)ethylamino)-2-oxoethyl)-2-oxo-1,2-dihydropyridin-3-ylamino)-7-ethoxy-1,7-dioxohept-5-en-2-ylamino)-4-oxobutanoic acid (A26) |
| (S,E)-Ethyl 6-acetamido-7-(1-(2-(2-(dimethylamino)ethylamino)-2-oxoethyl)-2-oxo-1,2-dihydropyridin-3-ylamino)-7-oxohept-2-enoate (A27) |
| (S,E)-Methyl 6-acetam ido-7-(1-(2-(isopentylam ino)-2-oxoethyl)-2-oxo-1,2-dihydro-pyridin-3-ylamino)-7-oxohept-2-enoate (A28) |
| (S,E)-methyl 6-acetam ido-7-(1-(2-(isopentylam ino)-2-oxoethyl)-2-oxo-1,2-d ihydro-pyridin-3-ylamino)-7-oxohept-2-enoate (A29) |
| (S, E)-4-(7-Ethoxy-1-(1-(2-(2-ethylbutylam ino)-2-oxoethyl)-2-oxo-1,2-d ihydropyrid in-3-ylamino)-1,7-dioxohept-5-en-2-ylamino)-4-oxobutanoic acid (A30) |
| (S,E)-Ethyl 6-acetamido-7-(1-(2-((S)-1-methoxy-4-methyl-1-oxopentan-2-ylamino)-2-oxoethyl)-2-oxo-1,2-dihydropyridin-3-ylamino)-7-oxohept-2-enoate (A31) |
| 4-((S,E)-7-Ethoxy-1-(1-(2-((S)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A32) |
| (S,E)-Ethyl 6-acetamido-7-(1-(2-((R)-1-methoxy-4-methyl-1-oxopentan-2-ylamino)-2-oxoethyl)-2-oxo-1,2-dihydropyridin-3-ylamino)-7-oxohept-2-enoate (A33) |
| 4-((S,E)-7-Ethoxy-1-(1-(2-((R)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A34) |
| (S,E)-ethyl 6-acetamido-7-(1-(2-((2S,3R)-1-methoxy-3-methyl-1-oxopentan-2-ylamino)-2-oxoethyl)-2-oxo-1,2-dihydropyridin-3-ylamino)-7-oxohept-2-enoate (A35) |
| 4-((S,E)-7-Ethoxy-1-(1-(2-((2S,3R)-1-methoxy-3-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A36) |
| (S,E)-4-(7-Ethoxy-1,7-dioxo-1-(2-oxo-1-(2-oxo-2-(4-(trifluoromethyl)benzylamino)ethyl)-1,2-dihydropyridin-3-ylamino)hept-5-en-2-ylamino)-4-oxobutanoic acid (A37) |
| (S,E)-4-(1-(1-(2-(3,3-Dimethylbutylamino)-2-oxoethyl)-2-oxo-1,2-dihydropyridin-3-ylamino)-7-ethoxy-1,7-dioxohept-5-en-2-ylamino)-4-oxobutanoic acid (A38) |
| (S,E)-Ethyl 6-acetamido-7-(1-(2-(3,3-dimethylbutylamino)-2-oxoethyl)-2-oxo-1,2-dihydropyridin-3-ylamino)-7-oxohept-2-enoate (A39) |
| (S,E)-Ethyl 6-acetamido-7-(1-(2-((S)-1-methoxy-4,4-dimethyl-1-oxopentan-2-ylamino)-2-oxoethyl)-2-oxo-1,2-dihydropyridin-3-ylamino)-7-oxohept-2-enoate (A40) |
| 4-((S,E)-7-Ethoxy-1-(1-(2-((S)-1-methoxy-4,4-dimethyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A41) |
| (S,E)-ethyl 6-acetamido-7-(1-(2-(3-ethylpentylamino)-2-oxoethyl)-2-oxo-1,2-dihydropyridin-3-ylamino)-7-oxohept-2-enoate (A42) |
| (S,E)-4-(7-Ethoxy-1-(1-(2-(3-ethylpentylamino)-2-oxoethyl)-2-oxo-1,2-dihydropyridin-3-ylamino)-1,7-dioxohept-5-en-2-ylamino)-4-oxobutanoic acid (A43) |
| (S,E)-4-(7-Ethoxy-1,7-dioxo-1-(2-oxo-1-(2-oxo-2-(2-(trifluoromethyl)benzylamino)ethyl)-1,2-dihydropyridin-3-ylamino)hept-5-en-2-ylamino)-4-oxobutanoic acid (A44) |
| (S,E)-4-(7-Ethoxy-1,7-dioxo-1-(2-oxo-1-(2-oxo-2-(4-(trifluoromethyl)piperidin-1-yl)ethyl)-1,2-dihydropyridin-3-ylamino)hept-5-en-2-ylamino)-4-oxobutanoic acid (A45) |
| (S,E)-Ethyl 6-acetamido-7-oxo-7-(2-oxo-1-(2-oxo-2-(2-(pyrrolidin-1-yl)ethylamino)ethyl)-1,2-dihydropyridin-3-ylamino)hept-2-enoate (A46) |
| (S,E)-4-(7-Ethoxy-1,7-dioxo-1-(2-oxo-1-(2-oxo-2-(2-(thiophen-2-yl)ethylamino)ethyl)-1,2-dihydropyridin-3-ylamino)hept-5-en-2-ylamino)-4-oxobutanoic acid (A47) |
| (S,E)-Ethyl 6-acetamido-7-(1-(2-((1-ethylpiperidin-4-yl)methylamino)-2-oxoethyl)-2-oxo-1,2-dihydropyridin-3-ylamino)-7-oxohept-2-enoate (A48) |
| (S,E)-4-(7-Ethoxy-1,7-dioxo-1-(2-oxo-1-(2-oxo-2-(2-(2-oxoimidazolidin-1-yl)ethylamino)ethyl)-1,2-dihydropyridin-3-ylamino)hept-5-en-2-ylamino)-4-oxobutanoic acid (A49) |
| (6S,E)-Ethyl 6-acetamido-7-(1-(2-(2-methylbutylamino)-2-oxoethyl)-2-oxo-1,2-dihydropyridin-3-ylamino)-7-oxohept-2-enoate (A50) |
| 4-((2S,E)-7-Ethoxy-1-(1-(2-(2-methylbutylamino)-2-oxoethyl)-2-oxo-1,2-dihydropyridin-3-ylamino)-1,7-dioxohept-5-en-2-ylamino)-4-oxobutanoic acid (A51) |
| (6S,E)-Ethyl 6-acetamido-7-(1-(2-(3-methylpiperidin-1-yl)-2-oxoethyl)-2-oxo-1,2-dihydropyridin-3-ylamino)-7-oxohept-2-enoate (A52) |
| (2S,3R)-2-(2-(3-((S,E)-7-Ethoxy-2-(1-methyl-1H-imidazole-5-carboxamido)-7-oxohept-5-enamido)-2-oxopyridin-1(2H)-yl)acetamido)-3-methylpentanoic acid (A53) |
| (S,E)-Ethyl 6-acetamido-7-(1-(2-(isobutylamino)-2-oxoethyl)-2-oxo-1,2-dihydropyridin-3-ylamino)-7-oxohept-2-enoate (A54) |
| (6S,E)-Ethyl 6-acetamido-7-(1-(2-(3-methylbutan-2-ylamino)-2-oxoethyl)-2-oxo-1,2-dihydropyridin-3-ylamino)-7-oxohept-2-enoate (A55) |
| (S,E)-Ethyl 7-(1-(2-(isopentylamino)-2-oxoethyl)-2-oxo-1,2-dihydropyridin-3-ylamino)-7-oxo-6-(3-ureidopropanamido)hept-2-enoate (A56) |
| (S,E)-Ethyl 6-acetamido-7-(1-(2-((S)-1-methoxy-3-methyl-1-oxobutan-2-ylamino)-2-oxoethyl)-2-oxo-1,2-dihydropyridin-3-ylamino)-7-oxohept-2-enoate (A57) |
| (S,E)-Ethyl 7-(1-(2-(2-ethylbutylamino)-2-oxoethyl)-2-oxo-1,2-d ihyd ropyrid in-3-ylamino)-6-(1-methyl-1H-imidazole-5-carboxamido)-7-oxohept-2-enoate (A58) |
| (2S,3R)-2-(2-(3-((S,E)-2-Benzamido-7-ethoxy-7-oxohept-5-enamido)-2-oxopyridin-1(2H)-yl)acetamido)-3-methylpentanoic acid (A59) |
| (2S,3R)-2-(2-(3-((S,E)-7-Methoxy-2-(1-methyl-1H-imidazole-5-carboxamido)-7-oxohept-5-enamido)-2-oxopyridin-1(2H)-yl)acetamido)-3-methylpentanoic acid (A60) |
| (2S,3R)-2-(2-(3-((S,E)-2-benzamido-7-methoxy-7-oxohept-5-enamido)-2-oxopyridin-1 (2H)-yl)acetamido)-3-methylpentanoic acid (A61) |
| (S,E)-Ethyl 6-acetamido-7-(1-(2-(2-ethylbutylamino)-2-oxoethyl)-2-oxo-1,2-dihydropyridin-3-ylamino)-7-oxohept-2-enoate (A62) |
| (S,E)-Methyl 7-(1-(2-(2-ethylbutylamino)-2-oxoethyl)-2-oxo-1,2-dihydropyrid in-3-ylamino)-6-(1-methyl-1H-imidazole-5-carboxamido)-7-oxohept-2-enoate A63 |
| (6S,E)-Methyl 6-acetamido-7-(1-(2-(3-methylpiperidin-1-yl)-2-oxoethyl)-2-oxo-1,2-dihydropyridin-3-ylamino)-7-oxohept-2-enoate (A64) |
| (S,E)-Methyl 6-acetamido-7-oxo-7-(2-oxo-1-(2-oxo-2-(4-(trifluoromethyl)piperidin-1-yl)ethyl)-1,2-dihydropyridin-3-ylamino)hept-2-enoate (A65) |
| (6S,E)-Methyl 6-acetamido-7-oxo-7-(2-oxo-1-(2-oxo-2-(3-(trifluoromethyl)piperidin-1-yl)ethyl)-1,2-dihydropyridin-3-ylamino)hept-2-enoate (A66) |
| (S,E)-Methyl 7-(1-(2-(2-ethylbutylamino)-2-oxoethyl)-2-oxo-1,2-dihydropyridin-3-ylamino)-6-(nicotinamido)-7-oxohept-2-enoate (A67) |
| (6S,E)-Methyl 6-(2-aminopropanamido)-7-(1-(2-(2-ethylbutylamino)-2-oxoethyl)-2-oxo-1,2-dihydropyridin-3-ylamino)-7-oxohept-2-enoate 2,2,2-trifluoroacetate (A68) |
| (S, E)-Methyl 6-(2-am inoacetam ido)-7-(3-((2-(2-ethyl butylam ino)-2-oxoethyl)(methyl)amino)-3-oxoprop-1-en-2-ylamino)-7-oxohept-2-enoate 2,2,2-trifluoroacetate (A69) |
| (S,E)-Methyl 6-(2-aminobenzamido)-7-(1-(2-(2-ethylbutylamino)-2-oxoethyl)-2-oxo-1,2-dihydropyridin-3-ylamino)-7-oxohept-2-enoate 2,2,2-trifluoroacetate (A70) |
| (S,E)-Methyl 6-(3',6'-bis(dimethylamino)-3-oxo-3H-spiro[isobenzofuran-1,9'-xanthene]-5-ylcarboxamido)-7-(1-(2-(2-ethylbutylamino)-2-oxoethyl)-2-oxo-1,2-dihydropyridin-3-ylamino)-7-oxohept-2-enoate (A71) |
| (S,E)-Ethyl 6-acetamido-7-(1-(2-(isopentylamino)-2-oxoethyl)-2-oxo-1,2-dihydropyridin-3-ylamino)-7-oxohept-2-enoate (A72) |
| (S,E)-Methyl 6-acetamido-7-(1-(2-(isopentylamino)-2-oxoethyl)-2-oxo-1,2-dihydropyridin-3-ylamino)-7-oxohept-2-enoate (A73) |
| (S,E)-Ethyl 7-(1-(2-(2-ethylbutylamino)-2-oxoethyl)-2-oxo-1,2-d ihyd ropyrid in-3-ylamino)-6-(1-methyl-1H-imidazole-5-carboxamido)-7-oxohept-2-enoate (A74) |
| (2S,3R)-2-(2-(3-((S,E)-2-Benzamido-7-methoxy-7-oxohept-5-enamido)-2-oxopyridin-1(2H)-yl)acetamido)-3-methylpentanoic acid (A75) |
| (R,E)-Methyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (A76) |
| (S,E)-2-acetamido-N-(1-(2-(isopentylamino)-2-oxoethyl)-2-oxo-1,2-dihydropyridin-3-yl)-6-(methylsulfonyl)hex-5-enamide (A77) |
| (S,E)-Benzyl 6-acetam ido-7-(1-(2-(isopentylam ino)-2-oxoethyl)-2-oxo-1,2-d ihydro-pyridin-3-ylamino)-7-oxohept-2-enoate A78 |
| (S,E)-Methyl 6-acetamido-7-(1-(2-(2-ethylbutylam ino)-2-oxoethyl)-2-oxo-1,2-dihydropyridin-3-ylamino)-7-oxohept-2-enoate (A79) |

Ein weiterer Aspekt der vorliegenden Erfindung betrifft daher Verbindungen gemäß allgemeiner Formel (I) als Arzneimittel sowie deren Verwendung in der Medizin.

Im Speziellen bevorzugt ist die Verwendung als Inhibitor für die Gewebetransglutaminase.

Ein weiterer Aspekt der vorliegenden Erfindung umfasst die Verwendung der erfindungsgemäßen Verbindungen der allgemeinen Formel (I) bei der Behandlung oder Prophylaxe von Zöliakie, Fibrosen, neurodegenerative Erkrankungen, Chorea Huntington, Parkinson, Alzheimer, Katarakt, Akne, Psoriasis, Hautalterung, Candiose und anderen Transglutaminase-abhängigen Erkrankungen.

Der Begriff "Transglutaminase-abhängige Erkrankungen" umfasst alle Erkrankungen, Störungen oder sonstige Beeinträchtigungen des Gesundheitszustandes, die ihre Ursache in oder im Zusammenhang mit einer Fehlfunktion, Störung oder einer Überaktivität von Transglutaminase 2 im Körper haben.

Die besondere Eignung der erfindungsgemäßen Verbindungen der allgemeinen Formel (I) ist direkt mit den sterischen und elektronischen Eigenschaften, die aus der Molekülstruktur hervorgehen verbunden. Die mit mindestens einer Elektronen wegziehenden Gruppe substituierte Vinylgruppe (Michael-Akzeptor-Gruppe) scheint eine wesentliche Einheit der Transglutaminase-Inhibitoren zu sein und führt in Kombination mit dem Pyridinon-enthaltenden Grundgerüst zu potenten Gewebetransglutaminase-Inhibitoren. Es hat sich überraschend herausgestellt, dass diese Kombination von einem Pyridinon-enthaltenden Gründgerüst, an dem auf der N-terminalen Seite eine Vielzahl von funktionellen Gruppen zur Einstellung gewünschter (physiko)chemischer Eigenschaften angebracht sein kann, zusammen mit der die akzeptorsubstituierte Doppelbindung tragenden Seitengruppe eine höhere Aktivität als auch Selektivität im Vergleich zu bekannten Inhibitoren mit Michael-Systemen aufweist. Somit scheint nicht nur die Anwesenheit eines Michael-Akzeptorsystems bzw. einer akzeptorsubstituierten Doppelbindung wichtig zu sein, sondern auch dessen weiterer konkreter Aufbau. Es hat sich als sehr vorteilhaft erwiesen, wenn die Seitengruppe mit akzeptorsubstituierter Doppelbindung bzw. mit Michael-System bioisoster zum Glutamin ist.

In einer bevorzugten Ausführungsform steht der Rest R^{#} für:
Die vorgenannten Reste können D- oder L-Konfiguration besitzen, wobei die L-Konfiguration bevorzugt ist.

Ein weiterer Aspekt der vorliegenden Erfindung betrifft die Bereitstellung der Verbindungen der allgemeinen Formel (I). Die erfindungsgemäßen Verbindungen lassen sich herstellen, indem die Aminosäure Glu (Glutaminsäure) am C-Terminus (PG2) und N-Terminus (PG1 und eventuell PG1'), sowie an der Carboxylfunktion (PG3) der Seitenkette mit Schutzgruppen (PG1, PG2 und PG3) versehen wird (1), danach die Carboxylfunktion der Seitenkette zum Aldehyd reduziert wird (2) und der erhaltene Aldehyd in eine akzeptorsubstituierte elektrophile Doppelbindung überführt wird (3). In einer bevorzugten Ausführungsform werden Schutzgruppen sowohl am N-Terminus als auch am C-Terminus entfernt (4/5) und der C-Terminus mit einem Pyridinonfragment verlängert (6). Nach Verlängerung der C-terminalen Carboxylfunktion wird der N-Terminus entschützt und anschließend verlängert (7/8). In einer weiteren bevorzugten Ausführungsform wird nach Einführung der akzeptorsubstituierten Doppelbindung zunächst der N-Terminus entschützt (4) und danach mit einem gewünschten Substituenten verlängert (5). Anschließend wird am C-Terminus die Schutzgruppe entfernt (6) und mit einem Pyridinonfragment verlängert (7). Der systematische Syntheseablauf mit den zwei unterschiedlichen bevorzugten Ausführungsformen ist in den folgenden Schemata dargestellt. 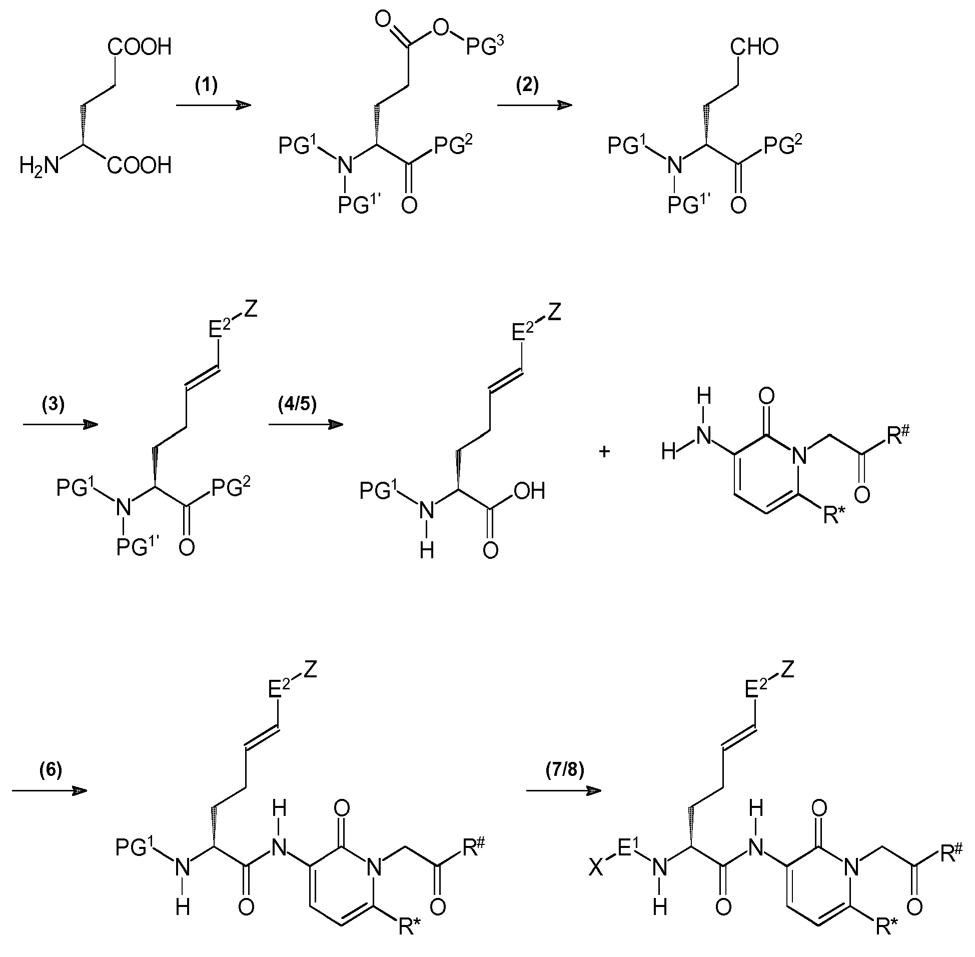
(0) Bereistellung von Glutaminsäure,
(1) Versehen der Glutaminsäure am C-Terminus und N-Terminus, sowie an der Carboxylfunktion der Seitenkette mit Schutzgruppen (PG1, PG2 und PG3),
(2) Reduktion der Carboxylfunktion der Seitenkette der Glutaminsäure zum Aldehyd,
(3) Überführung des erhaltenen Aldehyds in eine akzeptorsubstituierte elektrophile Doppelbindung,
(4) Entfernung einer Schutzgruppe am N-Terminus,
(5) Entfernung der Schutzgruppe am C-Terminus,
(6) Verlängerung des C-Terminus mit einem Pyridinonfragment,
(7) Entfernung der zweiten Schutzgruppe am N-Terminus
(8) Verlängerung des N-Terminus.
   oder 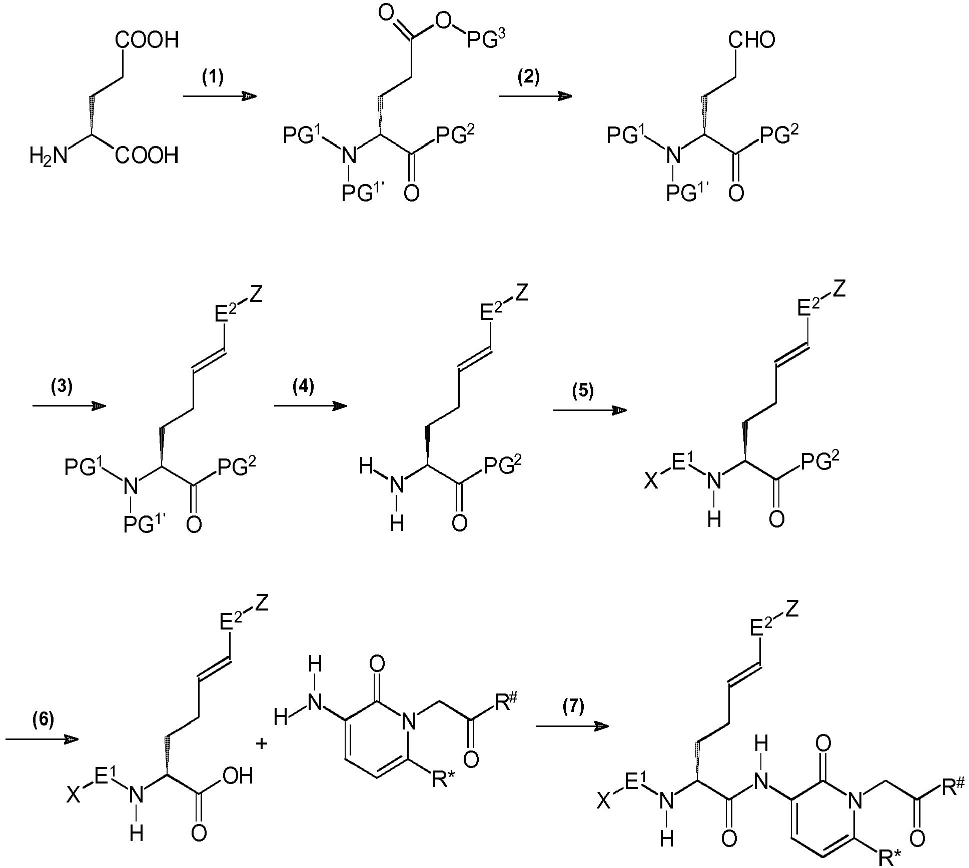 Schritte (0), (1), (2) und (3) wie oben
(4) Entfernung der Schutzgruppen am N-Terminus,
(5) Verlängerung des N-Terminus,
(6) Entfernung der Schutzgruppe am C-Terminus,
(7) Verlängerung des C-Terminus mit einem Pyridinonfragment.

Vorstufen zu den Pyridinonfragmenten der erfindungsgemäßen Verbindungen können gemäß dem folgenden Vorgehen hergestellt werden. Ausgehend von einem geeigneten 2-Hydroxynicotinsäurederivat werden zunächst eine geschützte Aminogruppe und am Pyridinstickstoff eine geschützte Carbonylmethylengruppe eingeführt und ferner wird das Hydroxypyridinderivat zu einem Pyridinonderivat umgesetzt (1). Anschließend wird an der geschützten Carbonylfunktion der Rest R^{#} eingeführt (2). Schließlich die geschützte Aminogruppe entschützt (3). Der systematische Syntheseablauf ist in folgendem Schema dargestellt: 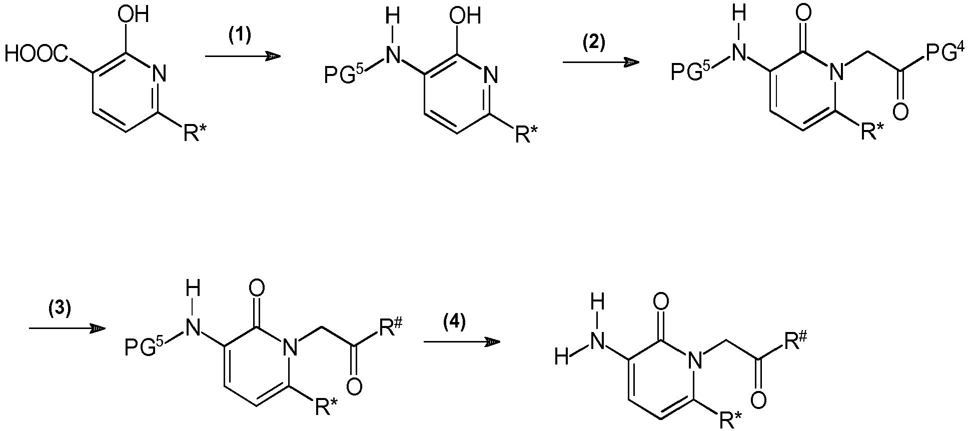
(1) Einführung einer geschützten Aminofunktion in ein 2-Hydroxynicotinsäurederivat an der Carboxylfunktion,
(2) Einführung einer Methylgruppe mit einer geschützten Carbonylfunktion (PG4),
(3) Einführung des Restes R^{#} an der geschützten Carbonylfunktion,
(4) Entschützung der Pyridinonylaminogruppe.

Alternativ können die Vorstufen zu den Pyridinonfragmenten der erfindungsgemäßen Verbindungen auch gemäß dem folgenden Vorgehen hergestellt werden. Ausgehend von einem geeigneten 2-Hydroxy-3-nitropyridinderivat wird am Pyridinstickstoff zunächst eine geschützte Carbonylmethylengruppe eingeführt und ferner wird das Hydroxypyridinderivat zu einem Pyridinonderivat umgesetzt (1). Anschließend wird an der geschützten Carbonylfunktion der Rest R^{#} eingeführt (2). Schließlich wird die Nitrogruppe zu einer Aminogruppe reduziert (3). Der systematische Syntheseablauf ist in folgendem Schema dargestellt: 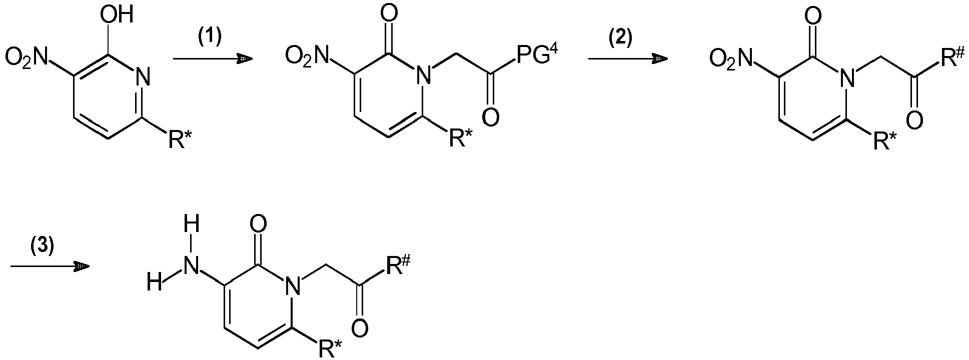
(1) Einführung einer Methylgruppe mit einer geschützten Carbonylfunktion (PG4) in ein 2-Hydroxy-3-nitropyridinderivat,
(2) Einführung des Restes R^{#} an der geschützten Carbonylfunktion,
(3) Reduktion der Nitrogruppe zu einer Aminogruppe.

Die hierin beschriebenen Verbindungen gemäß allgemeiner Formel (I) eignen sich insbesondere zur Behandlung und Prophylaxe von Zöliakie sowie anderer mit Transglutaminase 2 assoziierten Erkrankungen.

Zöliakie wird auch als Sprue, nichttropische oder einheimische Sprue, gluteninduzierte Enteropathie, Glutenunverträglichkeit oder intestinaler Infantilismus bezeichnet. Zöliakie ist eine Unverträglichkeit gegen "Gluten", das bei genetischer Prädisposition des Immunsystems zu einer chronischen Erkrankung der Dünndarmschleimhaut führt.

Gluten ist ein Klebereiweiß aus Prolamin und Glutenin, das in vielen Getreidesorten vorkommt wie beispielsweise Weizen, Bulgur (Weizensorte), Dinkel (Weizensorte), Einkorn (Weizensorte), Emmer (Weizensorte), Kamut (Weizensorte), Gerste, Grünkern, Roggen, Triticale (Weizen-Roggen-Kreuzung). Diese Getreidesorten besitzen einen Proteingehalt von etwa 7-15%, der zu ca. 90% aus Gluten besteht. Das Prolamin wird bei Weizen als Gliadin bezeichnet und bei Roggen als Secalin und bei Gerste als Hordein.

Zu den anderen mit Transglutaminase 2 assoziierten Erkrankungen zählen unter anderem Fibrosen, neurodegenerative Erkrankungen, Katarakt, Akne, Psoriasis, Hautalterung, Entzündungen ― insbesondere das gastrointestinal Trakts - und Candiose.

Diese Erkrankungen sind Beispiele für Indikationen, welche durch die hierin beschriebenen Verbindungen behandelt werden können.

Ein weiterer Aspekt der vorliegenden Erfindung umfasst eine pharmazeutische Zusammensetzung enthaltend mindestens eine Verbindung der allgemeinen Formeln (I), (II), (III), (IV), (V) oder (VI) und mindestens einen pharmakologisch verträglichen Träger, Hilfsstoff oder Lösungsmittel. Ferner betrifft die vorliegende Erfindung auch pharmazeutische Zusammensetzungen, welche des weiteren einen Wirkstoff ausgewählt aus der Gruppe umfassend Vitamine, Peptidasen, monoklonale Antikörper, Immunmodulatoren, Entzündunghemmer, Peptidasen und/oder Proteasen umfasst.

Somit können die hierin beschriebenen Verbindungen der Formel (I) oder entsprechend der vorliegenden Erfindung selbst oder in Form eines pharmakologisch wirksamen Salzes verabreicht werden.

Ferner betrifft die vorliegende Erfindung pharmazeutische Zusammensetzungen umfassend mindestens eine Verbindung gemäß allgemeiner Formel (I), (II), (III), (IV), (V) oder (VI) und/oder pharmakologisch verträgliche Salze davon und mindestens einen pharmakologisch verträglichen Träger, Hilfsstoff oder zumindest ein pharmakologisch verträgliches Lösungsmittel.

Zudem sind Kombinationspräparate mit anderen Wirkstoffen möglich, wobei die oder der weitere Wirkstoff mit zumindest einer hierin offenbarten Verbindung der allgemeinen Formel (I), (II), (III), (IV), (V) oder (VI) gemischt oder in Kombination verabreicht wird. Bevorzugt werden die Transglutaminase-Inhibitoren zur Unterstützung der Glutenfreien Diät gegeben. Natürlich kann eine Ergänzung mit Vitaminen, Mineralien und Spurenelementen indiziert sein. Auch eine Unterstützung von Enzympräparaten, bei denen beispielsweise Prolylendopeptidasen oder andere Peptidasen verwendet werden ist sinnvoll. Des weiteren kommen Kombinationen mit Entzündungshemmern (steroide und nicht-steroide), mit T-Zell-Silencern oder Cytokinen oder mit monoklonalen Antikörpern oder "tight junctions" Modulatoren in Frage.

Die pharmazeutischen Zusammensetzungen werden insbesondere für die Behandlung und Prophylaxe von Zöliakie sowie anderer mit der Transglutaminase 2 assoziierten oder durch Transglutaminase 2 verursachten Erkrankungen eingesetzt.

Die Verbindungen der allgemeinen Formel (I), (II), (III), (IV), (V) oder (VI) können ferner in Form ihrer pharmazeutisch aktiven Salze optional unter Verwendung von im Wesentlichen nicht toxischen pharmazeutisch verträglichen Trägern, Hilfsstoffen oder Verdünnern verabreicht werden. Die Medikationen werden in einem herkömmlichen festen oder flüssigen Träger oder Verdünnern und einem herkömmlichen pharmazeutisch verträglichen Hilfsstoff mit einer geeigneten Dosierung in einer bekannten Weise hergestellt. Die bevorzugten Präparationen sind in einer verabreichbaren Form, die für orale Anwendung geeignet ist wie beispielsweise Pillen, Tabletten, Filmtabletten, beschichtete Tabletten, Kapseln und Pulver.

Die bevorzugten pharmazeutischen Formulierungen sind Tabletten, Filmtabletten, beschichtete Tabletten, Gelatinkapseln und opake Kapseln. Jede pharmazeutische Zusammensetzung enthält mindestens eine Verbindung der allgemeinen Formel (I), (II), (III), (IV), (V) oder (VI) und/oder pharmazeutisch verträgliche Salze davon in einer Menge von 5 mg bis 500 mg, bevorzugt 10 mg bis 250 mg und am meisten bevorzugt in einer Menge von 10 bis 100 mg pro Formulierung.

Außerdem schließt der Gegenstand der vorliegenden Erfindung auch pharmazeutische Präparationen für orale, parenterale, dermale, intradermale, intragastralen, intrakutane, intravasale, intravenöse, intramuskuläre, intraperitoneale, intranasale, intravaginale, intrabukkale, perkutane, rektale, subkutane, sublinguale, topische, transdermale oder inhalative Anwendung ein, die zusätzlich zu typischen Vehikeln und Verdünnern eine Verbindung der allgemeinen Formel (I), (II), (III), (IV), (V) oder (VI) und/oder ein pharmazeutisch verträgliches Salz davon als einen aktiven Bestandteil enthalten.

Die pharmazeutischen Zusammensetzungen der vorliegenden Erfindung enthalten ein hierin offenbartes Pyridinonderivat als aktiven Bestandteil, typischerweise in einer Mischung mit geeigneten Trägermaterialien, ausgewählt im Hinblick auf die beabsichtigte Form der Verabreichung, d.h. oral verabreichbare Tabletten, Kapseln (entweder fest gefüllt, halbfest gefüllt oder flüssig gefüllt), Pulver, oral verabreichbare Gele, Elixiere, dispergierbare Granulate, Sirups, Suspensionen und dergleichen in Übereinstimmung mit herkömmlichen pharmazeutischen Praktiken. Zum Beispiel kann für die orale Verabreichung in der Form von Tabletten oder Kapseln das Pyridinonderivat als aktive Wirkstoffkomponente mit einem beliebigen oralen nicht toxischen pharmazeutisch verträglichen inerten Träger, wie Laktose, Stärke, Sucrose, Zellulose, Magnesiumstearat, Dikalziumphosphat, Kalziumsulfat, Talkum, Mannitol, Ethylalkohol (flüssige Formen) und dergleichen kombiniert werden. Außerdem können nach Bedarf geeignete Bindemittel, Gleitmittel, Sprengmittel und Färbemittel ebenfalls der Mischung beigefügt werden. Pulver und Tabletten können aus etwa 5Gew.-% bis zu etwa 95Gew.-% der erfinderischen Zusammensetzung aus diesen inerten Trägern bestehen.

Geeignete Bindemittel schließen Stärke, Gelatine, natürliche Zucker, Maissüßstoffe, natürliche und synthetische Gummis, wie Akaziengummi, Natriumalginat, Carboxymethyl-Cellulose, Polyethylenglycol und Wachse ein. Unter den Gleitmitteln können für die Verwendung in diesen Dosierungsformen Borsäure, Natriumbenzoat, Natriumacetat, Natriumchlorid und dergleichen erwähnt werden. Sprengmittel schließen Stärke, Methylcellulose, Guargummi und dergleichen ein. Süßstoffe und Geschmacksstoffe und Konservierungsstoffe können, falls dienlich, ebenfalls eingeschlossen sein. Einige der oben angeführten Ausdrücke, nämlich Sprengmittel, Verdünner, Gleitmittel, Bindemittel und dergleichen werden unten genauer diskutiert.

Zusätzlich können die Zusammensetzungen der vorliegenden Erfindung in einer Form mit verzögerter Freisetzung formuliert werden, um die Geschwindigkeits-gesteuerte Freisetzung einer beliebigen oder mehrerer Komponenten oder aktiven Bestandteile bereitzustellen, um die therapeutischen Wirkungen zu optimieren, d.h. antihistaminische Aktivität und dergleichen. Geeignete Dosierungsformen für eine verzögerte Freisetzung schließen Schichttabletten ein, die Schichten mit variierenden Abbaugeschwindigkeiten oder polymere Matrizen mit gesteuerter Freisetzung enthalten, die mit den aktiven Komponenten imprägniert sind, und in Tablettenform oder Kapseln gestaltet sind, die derartige imprägnierte oder verkapselte poröse polymere Matrizen beinhalten.

Präparationen in flüssiger Form schließen Lösungen, Suspensionen und Emulsionen ein. Als ein Beispiel können Wasser oder Wasser-Propylenglycol-Lösungen für parenterale Injektionen oder der Zusatz von Süßstoffen und Trübungsmitteln für orale Lösungen, Suspensionen und Emulsionen erwähnt werden.

Zur Inhalation geeignete Aerosol-Präparationen können Lösungen und Feststoffe in Pulverform einschließen, die mit einem pharmazeutisch verträglichen Träger, wie ein komprimiertes Inertgas, z.B. Stickstoff, in Kombination sein können.

Für die Zubereitung von Suppositorien wird zuerst ein niedrig schmelzendes Wachs, wie z.B. eine Mischung von Fettsäureglyceriden, wie z.B. Kakaobutter, geschmolzen und der aktive Bestandteil wird darin durch Rühren oder ähnliches Vermischen homogen dispergiert. Die geschmolzene homogene Mischung wird dann in passend bemessene Formen gegossen, man lässt abkühlen und dadurch verfestigen.

Ferner eingeschlossen sind Präparationen in fester Form, die kurz vor der Verwendung zu Präparationen in flüssiger Form für entweder orale oder parenterale Verabreichung umgewandelt werden sollen. Solche flüssigen Formen schließen Lösungen, Suspensionen und Emulsionen ein.

Die Verbindungen der vorliegenden Erfindung können ferner transdermal verabreichbar sein. Die transdermalen Zusammensetzungen können die Form von Cremes, Lotionen, Aerosolen und/oder Emulsionen annehmen.

Der Begriff Kapsel bezieht sich auf einen speziellen Behälter oder Gehäuse, das aus Methylzellulose, Polyvinylalkoholen oder denaturierten Gelatinen oder Stärke hergestellt ist, worin die Wirkstoffe eingeschlossen werden können. Hartmantelkapseln sind typischerweise aus Mischungen von Knochen und Schweinehautgelatinen relativ hoher Gelstärke hergestellt. Die Kapsel selbst kann kleine Mengen von Farbstoffen, Trübungsmitteln, Weichmachern und Konservierungsstoffen enthalten.

Tablette bedeutet komprimierte oder gegossene feste Dosierungsform, die die aktiven Bestandteile mit geeigneten Verdünnern enthält. Die Tablette kann durch Komprimieren von Mischungen oder Granulaten hergestellt werden, die durch Nassgranulierung, Trockengranulierung oder durch Kompaktierung erhalten wurden, die einem Fachmann bekannt sind.

Orale Gele beziehen sich auf die aktiven Bestandteile, die in einer hydrophilen halbfesten Matrix dispergiert oder solubilisiert sind.

Pulver für Zusammensetzungen beziehen sich auf Pulvermischungen, die die aktiven Bestandteile und geeignete Verdünner beinhalten, die in Wasser oder Säften suspendiert werden können.

Geeignete Verdünner sind Substanzen, die für gewöhnlich den Großteil der Zusammensetzung oder Dosierungsform ausmachen. Geeignete Verdünner schließen Zucker, wie Lactose, Sucrose, Mannitol und Sorbitol, von Weizen, Mais, Reis und Kartoffeln abgeleitete Stärken, und Zellulosen, wie mikrokristalline Zellulose ein. Die Menge an Verdünnern in der Zusammensetzung kann sich von etwa 5 bis etwa 95 Gew.-% der gesamten Zusammensetzung, bevorzugt von etwa 25 bis etwa 75 Gew.-% und weiter bevorzugt von etwa 30 bis etwa 60 Gew.-% erstrecken.

Der Ausdruck Sprengmittel bezieht sich auf Materialien, die der Zusammensetzung hinzugefügt wurden, um sie beim Aufbrechen (Zersprengen) und Freigeben der Medikamente zu unterstützen. Geeignete Sprengmittel schließen Stärken, "Kaltwasser-lösliche" modifizierte Stärken, wie Natrium-Carboxymethylstärke, natürliche und synthetische Gummis, wie Johannisbrotkernmehl, Karaya, Guar, Tragacanth und Agar, Cellulosederivate, wie Methylcellulose und Natrium-Carboxymethylcellulose, mikrokristalline Cellulosen und quervernetzte mikrokristalline Cellulosen, wie Natrium-Croscarmellose, Alginate, wie Alginsäure und Natriumalginat, Tonerden, wie Bentonite, und schäumende Mischungen. Die Menge an Sprengmittel in der Zusammensetzung kann sich von etwa 2 bis 20 Gew.-% der Zusammensetzung und weiter bevorzugt von etwa 5 bis etwa 10 Gew.-% erstrecken.

Bindemittel charakterisieren Substanzen, die Pulver miteinander binden oder "verkleben" und somit als "Kleber" in der Formulierung dienen. Bindemittel fügen eine Kohäsionsstärke hinzu, die in den Verdünnern oder dem Aufgehmittel bereits verfügbar ist. Geeignete Bindemittel schließen Zucker, wie Sucrose, von Weizen, Mais, Reis und Kartoffeln abgeleitete Stärken, natürliche Gummis, wie Akaziengummi, Gelatine und Tragacanth, Derivate von Seetang, wie Alginsäure, Natriumalginat und Ammonium-Calcium-Alginat, Zellulosematerialien, wie Methylcellulose und Natrium-Carboxymethylcellulose und Hydroxypropyl-methylcellulose, Polyvinylpyrrolidon und anorganische Verbindungen, wie Magnesium-Aluminium-Silicat ein. Die Menge der Bindemittel in der Zusammensetzung kann sich von etwa 2 bis etwa 20 Gew.-% der Zusammensetzung, weiter bevorzugt von etwa 3 bis etwa 10 Gew.-% und noch weiter bevorzugt von etwa 3 bis etwa 6 Gew.-% erstrecken.

Gleitmittel bezieht sich auf eine der Dosierungsform hinzugefügte Substanz, um zu ermöglichen, dass die Tablette, Granulat usw., nachdem sie komprimiert wurden, aus der Gießform oder Pressform durch Verringern der Friktion oder Reibung freigegeben werden. Geeignete Gleitmittel schließen metallische Stearate, wie Magnesiumstearat, Calciumstearat oder Kaliumstearat, Stearinsäure, Wachse mit hohem Schmelzpunkt, und wasserlösliche Gleitmittel, wie Natriumchlorid, Natriumbenzoat, Natriumacetat, Natriumoleat, Polyethylenglycole und D,L-Leucin ein. Gleitmittel werden gewöhnlich bei dem letzten Schritt vor dem Komprimieren hinzugefügt, da sie auf den Oberflächen der Granulate und zwischen ihnen und den Teilen der Tablettenpresse vorhanden sein müssen. Die Menge an Gleitmittel in der Zusammensetzung kann sich von etwa 0,2 bis etwa 5 Gew.-% der Zusammensetzung, bevorzugt von etwa 0,5 bis etwa 2 Gew.-% und weiter bevorzugt von etwa 0,3 Gew.-% bis etwa 1,5 Gew.-% erstrecken.

Gleitmittel sind Materialien, die eine Anbackung verhindern und die Fließcharakteristika von Granulaten verbessern, so dass der Fluss glatt und einheitlich ist. Geeignete Gleitmittel schließen Siliziumdioxid und Talkum ein. Die Menge von Gleitmittel in der Zusammensetzung kann sich von 0,1 bis etwa 5 Gew.-% der gesamten Zusammensetzung und bevorzugt von etwa 0,5 bis etwa 2 Gew.-% erstrecken.

Färbemittel sind Hilfsstoffe, die der Zusammensetzung oder der Dosierungsform eine Färbung bereitstellen. Derartige Hilfsstoffe können Farbstoffe mit Lebensmittelqualität und Farbstoffe mit Lebensmittelqualität einschließen, die auf einem geeigneten Adsorptionsmittel, wie Tonerde oder Aluminiumoxid adsorbiert sind. Die Menge des Färbemittels kann von etwa 0,1 bis etwa 5 Gew.-% der Zusammensetzung und bevorzugt von etwa 0,1 bis etwa 1 Gew.-% variieren.

Wie hierin verwendet ist eine "pharmazeutisch wirksame Menge" eines Transglutaminaseinhibitors die Menge oder die Aktivität, die wirksam ist, um das erwünschte physiologische Ergebnis entweder in in-vitro behandelten Zellen oder in einem in-vivo behandelten Patienten zu erreichen. Spezifisch ist eine pharmazeutisch wirksame Menge eine Menge, die ausreichend ist, um für eine gewisse Zeitspanne ein oder mehrere der klinisch definierten pathologischen Prozesse, die mit der Transglutaminase 2 assoziiert sind, zu inhibieren. Die wirksame Menge kann in Abhängigkeit des spezifischen Pyridinonderivates variieren und ist ferner von einer Vielfalt von Faktoren und Zuständen abhängig, die mit dem zu behandelnden Subjekt und der Schwere der Erkrankung in Beziehung stehen. Wenn zum Beispiel ein Inhibitor in-vivo verabreicht werden soll, dann wären Faktoren, wie das Alter, Gewicht und die Gesundheit des Patienten als auch Dosisreaktionskurven und Toxizitätsdaten, die aus vorklinischen Tierstudien erhalten wurden, unter den zu berücksichtigenden Faktoren. Falls der Inhibitor in Form der hierin beschriebenen Pyridinonderivate mit den Zellen in-vivo in Kontakt gebracht werden soll, würde man ferner eine Vielfalt von vorklinischen in-vitro Studien entwerfen, um solche Parameter, wie Aufnahme, Halbwertszeit, Dosis, Toxizität, usw. zu bestimmen. Die Bestimmung einer pharmazeutisch wirksamen Menge für ein gegebenes Pyridinonderivat gehört zu den normalen Fähigkeiten eines Fachmanns.

### Figurenbeschreibung

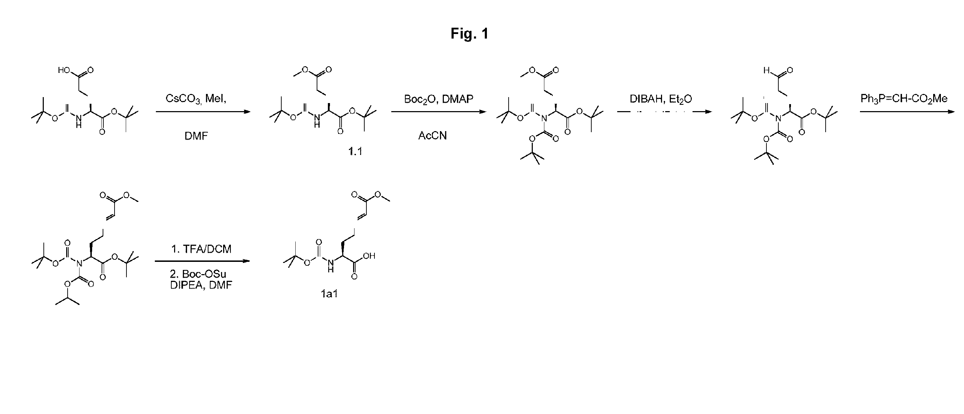

- Figur 1: zeigt das Syntheseschema für die Darstellung des L-2-Amino-hept-5-en-dicarbonsäurederivats 1a1.

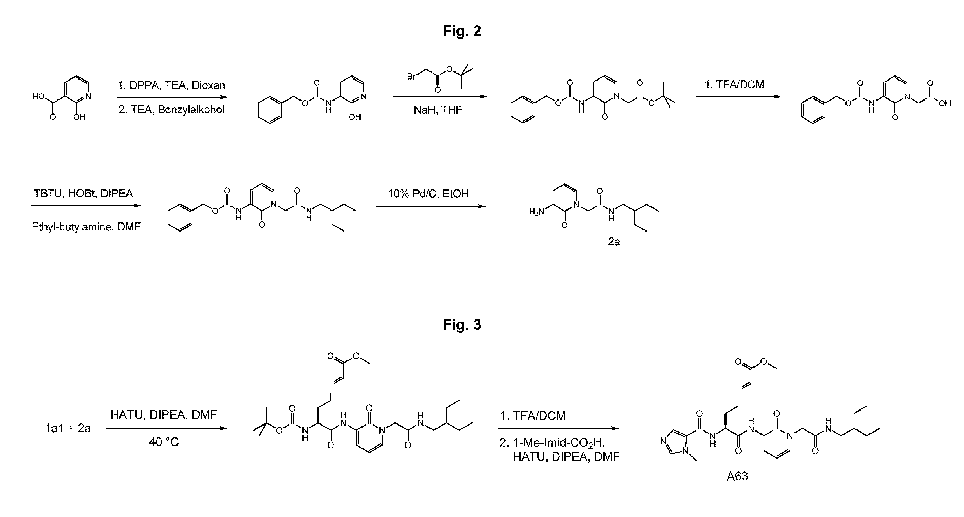

- Figur 2: zeigt das Syntheseschema für die Darstellung des Pyridinonderivats 2a.
- Figur 3: zeigt das Syntheseschema für die Darstellung der beispielverbindung A63. In den Figuren 1 bis 3 haben folgende Abkürzungen folgende Bedeutungen:
DMAP: 4-(Dimethylamino)-pyridin
DPPA: Diphenylphosphorylazid
TEA: Triethylamin
DMF: Dimethylformamid
ACN: Acetonitril
TBTU: O-(Benzotriazol-1-yl)-N,N,N',N'-tetramethyluroniumtetrafluoroborat
HOBt: 1-Hydroxybenzotriazol
DIPEA: N-Ethyldiisopropylamin
TFA: Trifluoressigsäure

### Allgemeine Synthesebeschreibung

### Aufbau des erfindungsgemäßen Michael-Akzeptorsystemes

Michael-Akzeptoren sind Olefine die mit mindestens einem elektronenziehenden Substituenten in Konjugation stehen. Für den Aufbau solcher Michael-Akzeptoren sind deshalb alle die Reaktionen geeignet, die ein solches Olefin generieren. Beispielhaft aber nicht beschränkend seien hierfür Alkenylierungsreaktionen an Metallorganylen, Corey-Winter-Reaktionen, Horner-Wadsworth-Emmonsreaktionen, Knoevenagel-Reaktionen, Wittig-Reaktionen, Wittig-Horner-Reaktionen, Julia-Lytgoe-Reaktionen und Peterson-Olefinierungen genannt. Diese und andere olefinbildenden Reaktionen sind dem Fachmann wohlbekannt. Besonders bevorzugt sind dabei die Reaktionen, in denen ein Aldehyd mit einem geeignet substituiertem Phosphory-Ylid oder einem entsprechenden Phosphonat reagiert (Wittig-Reaktion, Wittig-Horner-Reaktion, Horner-Wadsworth-Emmonsreaktion). Dragovich et al. zeigen die breite Anwendbarkeit dieses Reaktionstyps zum Aufbau von Michael-Akzeptorsystemen (Dragovich et al., J. Med. Chem., 1998, 41,15, 2806-2818). Die dafür benötigten Reagenzien sind in großem Umfang kommerziell erhältlich (z.B. Sigma-Aldrich) oder in der Literatur beschrieben. Im Folgenden ist eine allgemeine Synthesevorschrift für diese Olefinierungsreaktionen von Aldehyden gegeben. Konkrete Ausführungsbeispiele sind weiter unten aufgeführt.

Ausgegangen wird von einem geeignet substituierten Aldehyd, d.h. von einem Aldehyd der allgemeinen Struktur, worin X ein beliebiger Rest ist:

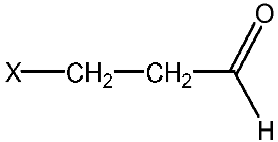

Dieser Aldehyd kann beispielhaft aus Derivaten der Glutaminsäure dargestellt werden.

Ein Äquivalent eines Phosphor-Ylides (z.b. Triphenylphosphonium-Ylide) wird in einem geeignetem Lösungsmittel (z.B. Benzol, Toluol oder DMF) gelöst und mit einer Base deprotoniert (z.B. NaH, n-BuLi, NaNH₂). Nach beendeter Reaktion wird ein Äquivalent des jeweiligen Aldehyds zugesetzt. Nach beendeter Reaktion wird das Lösungsmittel i. Vak. entfernt und das erhaltene Olefin durch chromatographische Methoden gereinigt.

### Allgemeine Vorschrift 1:

### Allgemeine Synthesevorschrift zu Verbindungen mit einem Alkyloxycarbonylethenyl-Michael-System:

Ausgegangen wird von der Aminosäure Glu (Glutaminsäure), welche am C-Terminus und N-Terminus sowie an der Carboxylfunktion der Seitenkette mit Schutzgruppen versehen wird. Als Schutzgruppen können säurelabile Schutzgruppen wie beispielsweise tert-Butyloxycarbonyl, tert.-Butylester, Methylester oder 2-Phenylisopropylester verwendet werden. Mittels Diisobutylaluminiumhydrid wird die Seitenkette selektiv zum Aldehyd reduziert und danach mit einem Phosphoran zwecks Erzeugung des Michael-Systems umgesetzt. Nach säureinduzierter Spaltung beispielsweise mittels Trifluoressigsäure der Schutzgruppen wird an das C-terminale Ende ein Pyridinonfragment über eine peptidische Bindung eingeführt. Anschließend wird an das N-terminale Ende eine Seitekette eingeführt. Dies erfolgt vorzugsweise mittels einer aktivierten Carbonsäure beispielsweise als Aktivester oder Carbonsäureanhydrid. Diese Reaktionen sind universell einsetzbar, dem Fachmann wohl bekannt und daher können an die Aminosäure mit dem Michael-System eine Vielzahl unterschiedlichster Reste sowohl am C- als auch am N-Terminus hinzugefügt werden.

### Beispiele

Die folgenden Beispiele sollen die Erfindung an ausgewählten Verbindungen veranschaulichen, ohne jedoch den Schutzumfang des vorliegenden Schutzrechtes auf diese konkreten Beispiele zu beschränken. Es ist für einen Fachmann ersichtlich, dass analoge Verbindungen und Verbindungen hergestellt nach analogen Synthesewegen unter den Schutzumfang des vorliegenden Schutzrechtes fallen.

### 1. Darstellung der Beispielverbindung A63 (ZED1227)

### 1.1 Darstellung von 6-Amino-hept-2-endicarbonsäure-derivaten

### (S)-1-tert-Butyl 5-methyl 2-(tert-butoxycarbonylamino)pentanedioate

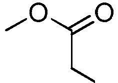

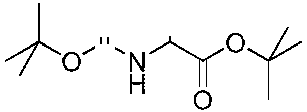

Summenformel: C15H27NO6
Molekulargewicht: 317,38

12,0 g Boc-Glu-OtBu (39,6 mmol) werden in 200 ml DMF gelöst. Unter Argonatmosphäre werden 7,09 g Cäsiumcarbonat (21,8 mmol, 0,55 eq) zugesetzt und die erhaltene Suspension eine Stunde bei Raumtemperatur gerührt. Nach dieser Zeit werden 2,47 ml Methyliodid (39,6 mmol) zugesetzt und bei Raumtemperatur über Nacht weiter gerührt. Das Lösungsmittel wird i. Vak. entfernt und der erhaltene Rückstand in 400 ml Ethylacetat aufgenommen. Vom ungelösten Feststoff wird abfiltriert und das Filtrat mit je 75 ml 10%iger Citronensäure-, 10% NaHCO₃⁻ und gesättigter NaCl-Lsg. je dreimal gewaschen. Nach Trocknen der organischen Phase über Na₂SO₄ wird das Lösungsmittel i. Vak. entfernt. Man erhält das Produkt als gelbes Öl. Das Produkt kann ohne weitere Reinigung in der Folgereaktion eingesetzt werden.
Ausbeute: 13,4 g, >100%
ESI-MS: 340,2 [M+Na]⁺

### Alternativ:

2,3 g N-tert.Butyloxycarbonyl-L-Glutaminsäure-1-tert.-Butylester (7,58 mmol) werden in 80 ml Methanol gelöst und bei Raumtemperatur eine frisch hergestellten Diazomethanlösung zugetropft (23 mmol Diazald^{®}). Nach einer Stunde wird das Lösungsmittel i. Vak. entfernt. Die Reinigung der Verbindung erfolgt durch Chromatographie an Kieselgel. (Säule: 18,5*4 cm, DCM/MeOH = 99/1, R_{f} = 0,99) Ausbeute: 1,3 g

### (S)-1-tert-Butyl 5-methyl 2-(bis(tert-butoxycarbonyl)amino)pentanedioate

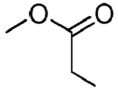

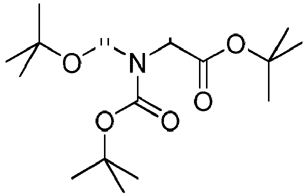

Summenformel: C20H35NO8
Molekulargewicht: 417,49

13,4 g Boc-Glu(OMe)-OtBu (~39,6 mmol) werden in 30 ml Acetonitril gelöst und mit 986 mg DMAP (7.91 mmol, 0,2 eq) versetzt. Unter Stickstoffatmosphäre wird eine Lösung aus 17,6 g Di-tert-butylbicarbonat (77,1 mmol, 2 eq) in 100 ml Acetonitril zugesetzt. Nach Rühren über Nacht wird das Lösungsmittel i. Vak. entfernt und das erhaltene Rohprodukt durch Chromatographie an Kieselgel gereinigt (Säule: 31*6,0 cm, Petrolether/Ethylacetat 9:1).
SC: in 250 ml Fraktionen gesammelt, Produkt: Fraktionen 6-13
DC-Kontrolle: Petrolether/Ethylacetat 8:2, R_{f} = 0,70
Ausbeute: 13,7 g, 32,8 mmol, 83 %
ESI-MS: 440,3 [M+Na]⁺

### (S)-tert-Butyl 2-(bis(tert-butoxycarbonyl)amino)-5-oxopentanoate

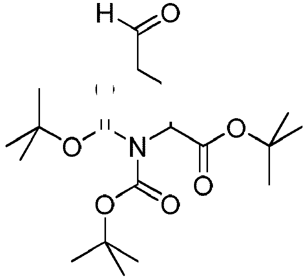

Summenformel: C19H33NO7
Molekulargewicht: 387,47

13,7 g Boc₂-Glu(OMe)-OtBu (32,8 mmol) werden in 200 ml absolutem Diethylether gelöst und unter Argonatmosphäre auf - 78 °C gekühlt. Bei dieser Temperatur werden langsam 36,1 ml (36,1 mmol, 1.1 eq) einer Lösung von Diisobutylaluminiumhydrid (1M in Hexan) zugetropft. Nach beendeter Zugabe wird für weitere 15 min bei - 78 °C gerührt, bevor bei dieser Temperatur der Reaktionsansatz durch Zugabe von 50 ml Wasser gequencht wird. Unter starkem Rühren wird auf Raumtemperatur aufgetaut und die trübe Lösung über Celite filtriert. Das Filtrat wird bis zur Trockne eingeengt und Restwasser durch Codestillation mit Toluol entfernt. Man erhält ein helles Öl, das ohne weitere Reinigung in der Folgereaktion eingesetzt wird.
DC-Kontrolle: Petrolether/Ethylacetat 8:2, R_{f} = 0,54
Ausbeute: 13.3 g, >100 % (Reinheit 86,1 %)
500-MHz-¹H-NMR-cosy (DMSOd6): δ[ppm] = 9,65 (s, 1H, H-4), 4,63 (dd, 1H, H-1, J_{1/2a} = 4,8 Hz, J_{1/2b} = 9,85 Hz), 2,51-2,50 (m, 1H, H-3ₐ), 2,48-4,40 (m, 1H, H-3_{b}), 2,27-2,20 (m, 1H, H-2a), 1,98-1,91 (m, 1H, H-2_{b}), 1,44 (s, 18H, 6*CH₃(Boc)), 1,92 (s, 9H, 3*CH₃(O-tBu)
ESI-MS: 410,4 [M+Na]⁺

### (S,E)-7-tert-Buty) 1-methyl 6-(bis(tert-butoxycarbonyl)amino)hept-2-enedioate

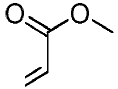

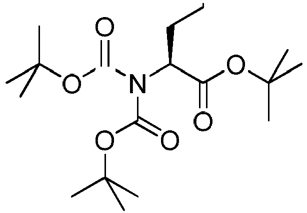

Summenformel: C22H37NO8
Molekulargewicht: 443,53

13,2 g Boc₂-Glu(H)-OtBu (~32,8 mmol) werden in 20 ml trockenem Benzol vorgelegt und unter Argonatmosphäre bei Raumtemperatur eine Lösung von 11,2 g (Methoxycarbonylmethylen)-triphenylphosphoran (32,8 mmol) zugesetzt. Nach Rühren über Nacht, wird das Lösungsmittel i. Vak. entfernt und der erhaltene ölige Rückstand durch Chromatographie an Kieselgel gereinigt (Säule: 39*6,0 cm, Petrolether/Ethylacetat 9:1).
SC: in 250 ml Fraktionen gesammelt, Produkt: Fraktionen 2-12
DC-Kontrolle: Petrolether/Ethylacetat 8:2, R_{f} = 0,54
Ausbeute: 12.0 g, 27.1 mmol, 83 %
500-MHz-¹H-NMR-cosy (DMSO_{d6}): δ[ppm] = 6,66 (dt, 1H, H-4, J_{4/3} = 6,8 Hz J_{4/5} = 15,9 Hz), 5,64 (d, 1H, H-5, J_{5/4} = 15,9 Hz), 4,45-4,2 (m, 1H, H-1), 3,44 (s, 3H, CH₃-6), 2,01-1,95 (m, 2-H, H-3ₐ, H-3_{b}), 1,95-1,86 (m, 1H, H-2ₐ), 1,78-1,67 (m, 1H, H-2_{b}), 1,24 (s, 18H, 6*CH₃(Boc)),
ESI-MS: 466,3 [M+Na]⁺

### (S,E)-2-(tert-Butoxycarbonylamino)-7-methoxy-7-oxohept-5-enoic acid (1a1)

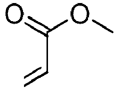

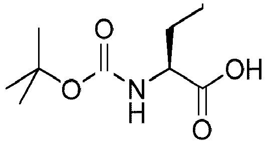

Summenformel: C13H21 N06
Molekulargewicht: 287,31

7,0 g (S,E)-7-tert-Butyl 1-methyl 6-(bis(tert-butoxycarbonyl)amino)hept-2-enedioate (15,8mmol) werden in 40 ml Dichlormethan gelöst und zur Lösung 70 ml Trifluoressigsäure zugesetzt. Bei Raumtemperatur wird für 4 h gerührt. Das Lösungsmittel wird i. Vak. entfernt und der grünliche Rückstand im Hochvakuum getrocknet. Das erhaltene Öl wird ohne Reinigung weiter umgesetzt.

Dazu wird das Öl in 50 ml DMF aufgenommen und mit 5,37 ml DIPEA versetzt. Durch sukzessiven Zusatz von DIPEA wird der pH-Wert auf ca. 7 eingestellt. Es werden 4,08 g Boc-OSu (18,9 mmol, 1,2 eq) zugegeben und bei Raumtemperatur über Nacht gerührt. Das Lösungsmittel wird i. Vak. entfernt und der Rückstand in 130 ml 5%iger KHSO₄-Lsg. suspendiert. Es wird mit Ethylacetat extrahiert (1 x 150 ml, 2 x 100 ml) und die vereinigten org. Phasen mit gesättigter NaCl-Lsg. (75 ml) gewaschen. Nach Trocknen der organischen Phase über Na₂SO₄ wird das Lösungsmittel i. Vak. entfernt.

Der Rückstand wird durch Chromatographie an Kieselgel gereinigt (Säule: 13*6,0 cm, Toluol/Ethylacetat 65:35, 0,5% Essigsäure). Man erhält ein farbloses Öl.
SC: in 200 ml Fraktionen gesammelt, Produkt: Fraktionen 2-5, Vorlauf 500 ml DC-Kontrolle: Toluol/Ethylacetat 1:1, 0,5% Essigsäure, R_{f} = 0,35
Ausbeute: 4,04 g, 14,1 mmol, 89 % (Reinheit 88,6 %)
ESI-MS: 310.1 [M+Na]⁺

### 1.2 Darstellung von Pyridinonderivaten

### Variante A

### Benzyl-3-hydroxypyridin-3-yl-carbamate

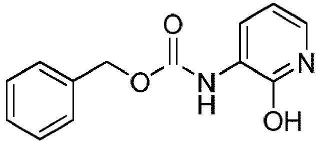

Summenformel: C13H12N2O3
Molekulargewicht: 244,25

15 g 2-Hydroxy-nicotinsäure (108 mmol) werden in 180 ml trockenem Dioxan suspendiert. Nach Zugabe von 14,9 ml Triethylamin (108 mmol) klart die Suspension weitgehend auf. 24 ml Diphenylphophorsäureazid (DPPA, 108 mmol) werden zugesetzt und die Reaktionslösung unter Argonatmosphäre zum Rückfluss (130 °C) erhitzt. Dabei ist eine Gasentwicklung zu beobachten. Nach 16 h werden nacheinander weitere 16,3 ml TEA sowie 12,8 ml Benzylakohol (117 mmol, 1,1 eq) zugesetzt und für weitere 24 h unter Rückfluss erhitzt.

Das Lösungsmittel wird i. Vak. entfernt und der erhaltene braune Rückstand in einer Mischung aus 300 ml DCM und 300 ml ges. wässriger NaCl-Lsg. aufgenommen. Mittels 1 M HCl-Lsg. wird der pH-Wert auf ca. 1 eingestellt (ca. 22 ml), die Phasen getrennt und anschließend die wässrige Phase zweimal mit je 200 ml DCM extrahiert. Die vereinigten organischen Phasen werden mit 10%iger NaHCO₃-Lsg. (3x150 ml) und ges. NaCl-Lsg. (1x150 ml) gewaschen, über Na₂SO₄ getrocknet, abfiltriert und i. Vak. zur Trockne eingeengt. Der erhaltene braune Feststoff wird aus 300 ml Methanol umkristallisiert.
DC-Kontrolle: DCM/MeOH 9:1, Rf = 0,70
Ausbeute: 16,2 g, 66,4 mmol, 62 % (hellbrauner, filzartiger Feststoff)
ESI-MS: 245,1 [M+H]⁺

### tert-Butyl 2-(3-(benzyloxycarbonylamino)-2-oxopyridin-1(2H)-yl)acetate

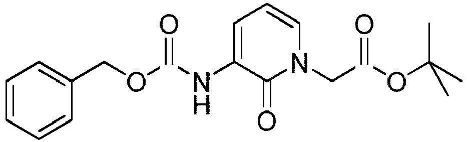

Summenformel: C19H22N2O5
Molekulargewicht: 358,39

16,2 g Benzyl-3-hydroxypyridin-3-yl-carbamate (66,4 mmol) werden in 900 ml absolutem THF suspendiert und unter Argonatmosphäre auf 0 °C abgekühlt und 2,92 g NaH (60%ig auf Mineralöl, 73,1 mmol, 1,1 eq) zugegeben. Zur entstandenen Lösung werden nach beendeter Gasentwicklung (ca. 15 min) 13,7 ml Bromessigsäure-tert-Butylester (89,7 mmol, 1,35 eq) zugesetzt. Man rührt noch 15 Minuten bei 0 °C und anschließend bei Raumtemperatur über Nacht. Die Reaktionsmischung wird abfiltriert und das Filtrat zur Trockne eingeengt. Der Rückstand wird in 5 ml Ethylacetat aufgenommen und mit ca. 50 ml Diethylether versetzt und die resultierende Suspension über Nacht im Kühlschrank ausgefällt. Die Kristalle werden abgesaugt und mit wenig Ether gewaschen.

Das Filtrat wird eingeengt und durch Chromatographie an Kieselgel gereinigt (Bett: 20x 6 cm, Laufmittel: Petrolether/Ethylacetat= 8/2).
SC: in 250 ml Fraktionen gesammelt, Produkt: Fraktion 10-25
DC-Kontrolle: Petrolether/Ethylacetat= 7/3, Rf = 0,46
Ausbeute: 19,3 g, 54,0 mmol, 81 %
ESI-MS: 359,1 [M+H]⁺

### 2-(3-(Benzyloxycarbonylamino)-2-oxopyridin-1(2H)-yl)acetic acid

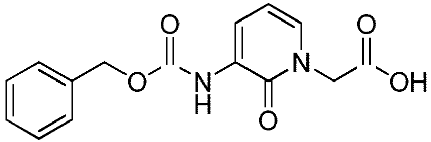

Summenformel: C15H14N2O5
Molekulargewicht: 302,28

4,00 g tert-Butyl 2-(3-(benzyloxycarbonylamino)-2-oxopyridin-1(2H)-yl)acetate (11,2 mmol) werden in 50 ml Dichlormethan gelöst und mit 50 ml Trifluoressigsäure versetzt. Bei Raumtemperatur wird für 3 h gerührt, bevor die volatilen Bestandteile i.Vak. entfernt werden. Nach Trocknen im Hochvakuum erhält man einen braunen Feststoff, der ohne Reinigung für die weitere Verwendung geeignet ist.
Ausbeute: 3,70 g, >100 %
ESI-MS: 303,2 [M+H]⁺

### Benzyl-1-(2-(2-ethylbutylamino)-2-oxoethyl)-2-oxo-1,2-dihydropyridin-3-ylcarbamate

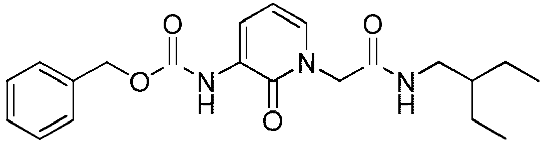

Summenformel: C21H27N3O4
Molekulargewicht: 385,46

Eine Mischung aus 3,70 g 2-(3-(benzyloxycarbonylamino)-2-oxopyridin-1(2H)-yl)acetic acid (~11,2 mmol), 3,58 g TBTU (11,2 mmol), 1,51 g HOBt (11,2 mmol) werden in 60 ml DMF gelöst. Durch Zusatz von 5,70 ml DIPEA (33,5 mmol, 3 eq) wird ein pH-Wert von ~10 eingestellt. Es wird 1,50 ml 2-Ethyl-butylamin (11,2 mmol) zugesetzt und die Mischung bei Raumtemperatur über Nacht gerührt. Das Lösungsmittel wird i. Vak. entfernt, der erhaltene Rückstand in 300 ml DCM aufgenommen und nacheinander mit 10%iger Citronensäure (3x75 ml), ges. NaHCO₃-Lsg. (3x75 ml) und ges. NaCl-Lsg. (75 ml) gewaschen. Die organische Phase wird über Na₂SO₄ getrocknet, abfiltriert und i. Vak. zur Trockne eingeengt. Man erhält einen hellbraunen Feststoff, der ohne weitere Reinigung zur Weiterverarbeitung geeignet ist.
Ausbeute: 5,22 g, >100%
ESI-MS: 386,3 [M+H]⁺

### 2-(3-Amino-2-oxopyridin-1(2H)-yl)-N-(2-ethylbutyl)acetamide (2a)

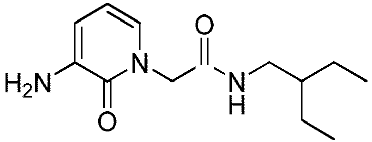

Summenformel: C13H21 N302
Molekulargewicht: 251,32

5,22 g Benzyl-1-(2-(2-ethylbutylamino)-2-oxoethyl)-2-oxo-1,2-dihydropyridin-3-yl-carbamate (2.4, ~11,2 mmol) werden unter Stickstoffatmosphäre in 60 ml Methanol gelöst. Zu dieser Lösung werden 500 mg Pd/C (10%) gegeben und unter Wasserstoffatmosphäre bei Atmosphärendruck für 2,5 h bei Raumtemperatur gerührt. Der Katalysator wird durch Filtration über Silcagel abgetrennt, bevor das Lösungsmittel i.Vak. entfernt wird. Man erhält ein dunkles Öl, das ohne weitere Reinigung zur Weiterverarbeitung geeignet ist.
Ausbeute: 3,62 g, >100%
ESI-MS: 252,2 [M+H]⁺

### Variante B

### tert-Butyl 2-(3-nitro-2-oxopyridin-1(2H)-yl)acetate

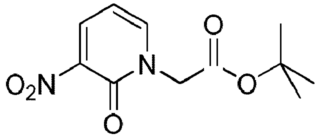

Summenformel: C11H14N205
Molekulargewicht: 254,24

3.00 g (21,0 mmol) 2-Hydroxy-3-nitropyridinin werden in 175 ml THF suspendiert (Argon-Atmosphäre) und bei 0 °C langsam 923 mg (23,1 mmol, 1,1 eq) NaH (60 %) zugegeben. Nach 30 min Rühren bei 0 °C werden 4,24 ml (28,3 mmol, 1,35 eq) tert-Butylbromoacetat zugetropft und ü.N. bei RT gerührt. Die Lösung wird auf 300 g Eis gegossen und der THF-Anteil i. Vak. entfernt. Der Rückstand wird mit Ethylacetat (2x200 ml) extrahiert, die org. Phase über Natriumsulfat getrocknet und das Lösungsmittel i.Vak. entfernt (Säule: 41*6,0 cm, Petrolether/Ethylacetat 1:9).
SC: in 50 ml Fraktionen gesammelt, Produkt: Fraktionen 7-16
DC-Kontrolle: Ethylacetat, R_{f} = 0,72
Ausbeute: 3,73 g, 14,7 mmol, 70 %
ESI-MS: 255,1 [M+H]⁺

### 2-(3-Nitro-2-oxopyridin-1(2H)-yl)acetic acid

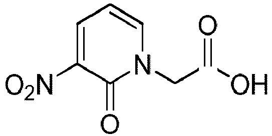

Summenformel: C7H6N2O5
Molekulargewicht: 198,13

3,73 g (14,7 mmol) tert-Butyl 2-(3-nitro-2-oxopyridin-1(2H)-yl)acetate werden in 10 ml Dichlormethan gelöst und mit 40 ml Trifluoressigsäure versetzt. Bei RT wird für 3 h gerührt, bevor die volatilen Bestandteile i. Vak. entfernt werden. Das Produkt wird ohne Reinigung weiter umgesetzt.
Ausbeute: 3,05 g, >100%
ESI-MS: 199,0 [M+H]⁺

### N-(2-Ethylbutyl)-2-(3-nitro-2-oxopyridin-1(2H)-yl)acetamide

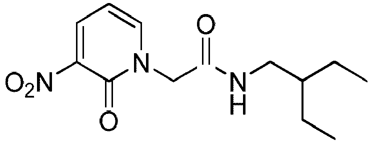

Summenformel: C13H19N3O4
Molekulargewicht: 281,31

Das Rohprodukt 2-(3-Nitro-2-oxopyridin-1(2H)-yl)acetic acid (~14,7 mmol), 4,71 g (14,7 mmol) TBTU, 1,98 g (14,7 mmol) HOBt und 4,99 ml (29,3 mmol, 2 eq) DIPEA werden in 60 ml DMF gelöst (Argon-Atmosphäre). Es werden 1,97 ml (14,7 mmol) 2-Ethyl-Butylamin (→ pH = 6) und ein weiteres eq DIPEA (→ pH = 8) zugegeben. Nach 1 h wird ein halbes eq DIPEA nachtitriert und ü.N. bei RT gerührt. Zeigt die DC-Kontrolle unvollständigen Umsatz wird weitere 3 h bei 45 °C gerührt. Das Lösungsmittel wird i.Vak. entfernt und der Rückstand in 500 ml DCM/MeOH (8/2) aufgenommen. Es wird nacheinander mit 10%iger Citronensäure (3x100 ml), ges. NaHCO₃-Lsg. (3x100 ml) und ges. NaCl-Lsg. (75 ml) gewaschen. Die org. Phase wird über Natriumsulfat getrocknet und das Lösungsmittel i.Vak. entfernt. Das Rohprodukt (3,5 g) wird mit 10 ml Ethylacetat versetzt, 30 ml PE (40-60) zugegeben und 15 min bei RT gerührt. Der Feststoff wird abgesaugt, mit etwas PE nachgewaschen und getrocknet.
Ausbeute: 2,90 g, 10,9 mmol, 70 %
ESI-MS: 282,2 [M+H]⁺

### 2-(3-amino-2-oxopyridin-1 (2H)-yl)-N-(2-ethylbutyl)acetamide

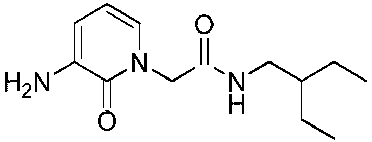

Summenformel: C13H21 N302
Molekulargewicht: 251,32

100 mg (0,36 mmol) N-(2-Ethylbutyl)-2-(3-nitro-2-oxopyridin-1 (2H)-yl)acetamide werden unter Stickstoffatmosphäre in 7 ml Methanol suspendiert. Zu dieser Lösung werden 10 mg Pd/C (10%) gegeben und unter Wasserstoffatmosphäre bei Atmosphärendruck für 2 h bei Raumtemperatur gerührt. Der Katalysator wird durch Filtration über Silicagel abgetrennt, bevor das Lösungsmittel i. Vak. entfernt wird. Man erhält einen grün-grauen Feststoff, der ohne Reinigung zur Weiterverarbeitung geeignet ist.
Ausbeute: 92 mg, >100 %
ESI-MS: 252,2 [M+H]⁺

### 1.3 Darstellung von Pyridinon-Inhibitoren

### (S,E)-Methyl 6-(tert-butoxycarbonylamino)-7-(1-(2-(2-ethylbutylamino)-2-oxoethyl)-2-oxo-1,2-dihydropyridin-3-ylamino)-7-oxohept-2-enoate

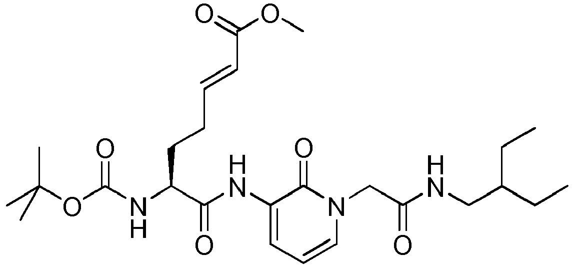

Summenformel: C26H40N4O7
Molekulargewicht: 520,62

Es wird eine Lösung aus 3,36 g 2-(3-Amino-2-oxopyridin-1 (2H)-yl)-N-(2-ethylbutyl)acetamide (2a, ~10,4 mmol) in 20 ml DMF vorgelegt. Dazu wird eine Lösung aus 2,97 g (S,E)-2-(tert-Butoxycarbonylamino)-7-ethoxy-7-oxohept-5-encarbonsäure (1a1, 10,4 mmol), 3,93 g HATU (10,4 mmol) und 3,52 ml DIPEA (20,7 mmol, 2 eq) in 40 ml DMF gegeben. Durch sukzessiven Zusatz von DIPEA wird der pH-Wert auf ca. 7 eingestellt. Das Reaktionsgemisch wird bei 40 °C für 2,5 Stunden sowie bei Raumtemperatur über Nacht gerührt, bevor das Lösungsmittel i. Vak. entfernt wird. Der erhaltene braune Rückstand wird in 250 ml Ethylacetat aufgenommen und nacheinander mit 10%iger Zitronensäure (3x75 ml), ges. NaHCO₃-Lsg. (3x75 ml) und ges. NaCl-Lsg. (75 ml) gewaschen. Die organische Phase wird über Na₂SO₄ getrocknet, abfiltriert und i. Vak. zur Trockne eingeengt. Der Rückstand wird durch Chromatographie an Kieselgel gereinigt (Bett: 13 x 6 cm, Laufmittel: Toluol/Aceton = 7/3).
SC: 150 ml Vorlauf, in 40 ml Fraktionen gesammelt, Produkt: Fraktion 6-15 DC-Kontrolle: DCM/MeOH = 97/3, Rf = 0,40
Ausbeute: 3,34 g, 6,42 mmol, 62 %
ESI-MS: 543,4 [M+Na]⁺

### (S,E)-Methyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate A63 (ZED1227)

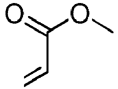

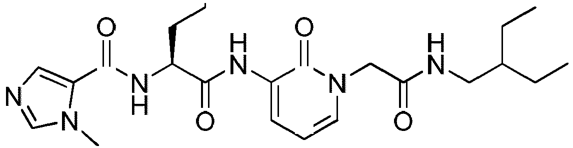

Summenformel: C26H36N6O6
Molekulargewicht: 528,60

3,14 g (S,E)-Methyl 6-(tert-butoxycarbonylamino)-7-(1-(2-(2-ethylbutylamino)-2-oxoethyl)-2-oxo-1,2-dihydropyridin-3-ylamino)-7-oxohept-2-enoate (3.1, 6,03 mmol) werden in einer Mischung aus 25 ml Dichlormethan und 35 ml TFA gelöst und für 3 Stunden bei Raumtemperatur gerührt, bevor die volatilen Bestandteile i. Vak. entfernt werden. Das erhaltene braune Öl wird im Hochvakuum getrocknet und in 10 ml DMF gelöst und 1,03 ml DIPEA (6,03 mmol) zugegeben. Dazu wird eine Lösung aus 2,29 g HATU (6,03 mmol) und 1,03 ml DIPEA (6,03 mmol) in 30 ml DMF gegeben. Durch sukzessiven Zusatz von DIPEA wird der pH-Wert auf ca. 7 eingestellt. Es wird über Nacht bei Raumtemperatur gerührt. Der Rückstand wird in 200 ml Ethylacetat aufgenommen und nacheinander mit 10%iger Citronensäure, ges. NaHCO₃-Lsg. und ges. NaCl-Lsg. (je 75 ml) gewaschen. Die organische Phase wird über Na₂SO₄ getrocknet, abfiltriert und i. Vak. zur Trockne eingeengt. Der Rückstand wird durch Chromatographie an Kieselgel gereinigt (Bett: 12 x 6 cm, Laufmittel:
DCM/MeOH = 97/3, nach 2 Litern 95/5).
SC: 1000 ml Vorlauf, in 50 ml Fraktionen gesammelt, Produkt: Fraktion 43-66 DC-Kontrolle: DCM/MeOH = 97/3, Rf = 0,30
Ausbeute: 1,42 g, 2,69 mmol, 45 %
ESI-MS: 551,3 [M+Na]⁺
¹H-NMR (DMSO-d6, 500 MHz): δ[ppm] = 9,29 (s, 1H), 8,63 (d, 1H), 8,21 (dd, 1H), 8,04 (t, 1H), 7,75 (d, 2H), 7,33 (dd, 1H), 6,93 (dt, 1H, J = 15,63; 6,93), 6,25 (t, 1H), 5,86 (d, 1H, J = 15,69), 4,58 (s, 2H), 3,79 (s, 3H), 3,62 (s, 3H), 3,01 (t, 2H), 2,33 (m, 2H), 2,03 (m, 1H), 1,90 (m, 1H), 1,26 (m, 5H), 0,83 (t, 6H)

### 2. Darstellung der Beispielverbindung A29 (ZED1098)

### Benzyl 1-(2-(isopentylamino)-2-oxoethyl)-2-oxo-1,2-dihydropyridin-3-ylcarbamate (ZED1020)

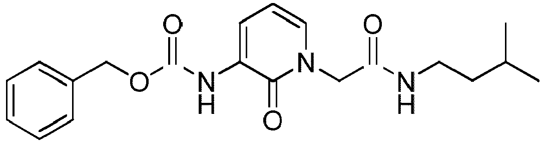

Summenformel: C20H25N3O4
Molekulargewicht: 371,43

Eine Mischung aus 900 mg 2-(3-(benzyloxycarbonylamino)-2-oxopyridin-1(2H)-yl)acetic acid (~2,32 mmol), 744 mg TBTU (2,32 mmol), 313 mg HOBt (2,32 mmol) werden in 25 ml DMF gelöst. Durch Zusatz von 985 µl DIPEA (5,79 mmol, 2,5 eq) wird ein pH-Wert von ~9 eingestellt. Es wird 539 µl lospentylamin (4,63 mmol) zugesetzt und die Mischung bei Raumtemperatur für 2,5 h gerührt. Das Lösungsmittel wird i. Vak. entfernt, der erhaltene Rückstand in 100 ml DCM aufgenommen und nacheinander mit 10%iger Citronensäure (3x75 ml), ges. NaHCO₃-Lsg. (3x75 ml) und ges. NaCl-Lsg. (75 ml) gewaschen. Die organische Phase wird über Na₂SO₄ getrocknet, abfiltriert und i. Vak. zur Trockne eingeengt. Man erhält einen bräunlichen Feststoff, der ohne weitere Reinigung zur Weiterverarbeitung geeignet ist.
Ausbeute: 573 mg, 1,54 mmol, 67 %
ESI-MS: 372,3 [M+H]⁺

### 2-(3-Amino-2-oxopyridin-1(2H)-yl)-N-isopentylacetamide (ZED1022)

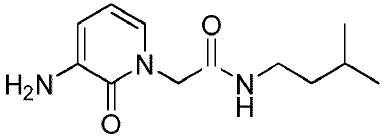

Summenformel: C12H19N3O2
Molekulargewicht: 237,30

573 mg Benzyl 1-(2-(isopentylamino)-2-oxoethyl)-2-oxo-1,2-dihydropyridin-3-ylcarbamate (ZED1020), 1,54 mmol) werden unter Stickstoffatmosphäre in 100 ml Ethanol gelöst. Zu dieser Lösung werden 50 mg Pd/C (10%) gegeben und unter Wasserstoffatmosphäre bei Atmosphärendruck für 1 h bei Raumtemperatur gerührt. Der Katalysator wird durch Filtration über Silcagel abgetrennt, bevor das Lösungsmittel i.Vak. entfernt wird. Man erhält ein grünes Öl, das ohne weitere Reinigung zur Weiterverarbeitung geeignet ist.
Ausbeute: 381 mg, >100 %
ESI-MS: 238,3 [M+H]⁺

### (S,E)-methyl 6-(tert-butoxycarbonylamino)-7-(1-(2-(isopentylamino)-2-oxoethyl)-2-oxo-1,2-dihydropyridin-3-ylamino)-7-oxohept-2-enoate (ZED1096)

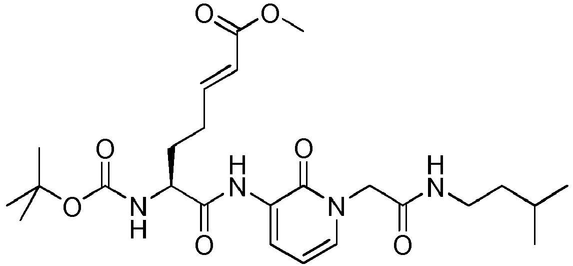

Summenformel: C25H38N4O7
Molekulargewicht: 506,59

Es wird eine Lösung aus 381 mg 2-(3-Amino-2-oxopyridin-1 (2H)-yl)-N-isopentylacetamide (ZED1022, ~1,54 mmol) in 5 ml DMF vorgelegt. Dazu wird eine Lösung aus 443 mg (S,E)-2-(tert-Butoxycarbonylamino)-7-ethoxy-7-oxohept-5-encarbonsäure (1a1, 1,54 mmol), 586 mg HATU (1,54 mmol) und 524 µl DIPEA (3,08 mmol, 2 eq) in 5 ml DMF gegeben. Durch sukzessiven Zusatz von DIPEA wird der pH-Wert auf ca. 7 eingestellt. Das Reaktionsgemisch wird bei 40 °C für 2,5 h sowie bei Raumtemperatur über Nacht gerührt, bevor das Lösungsmittel i. Vak. entfernt wird. Der erhaltene Rückstand wird in 75 ml Ethylacetat aufgenommen und nacheinander je dreimal mit 10%iger Zitronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Der bläuliche Rückstand wird ohne Reinigung weiter verarbeitet.
Ausbeute: 556 mg, 1,10 mmol, 72 %
ESI-MS: 507,3 [M+H]⁺

### (S,E)-methyl 6-acetamido-7-(1-(2-(isopentylamino)-2-oxoethyl)-2-oxo-1,2-dihydro-pyridin-3-ylamino)-7-oxohept-2-enoate (A29) (ZED1098)

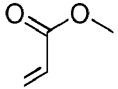

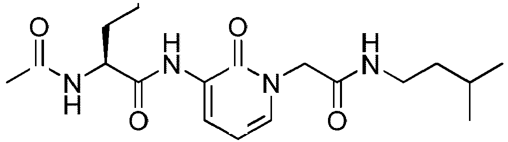

Summenformel: C22H32N4O6
Molekulargewicht: 448,51

556 mg (S,E)-methyl 6-(tert-butoxycarbonylamino)-7-(1-(2-(isopentylamino)-2-oxoethyl)-2-oxo-1,2-dihydropyridin-3-ylamino)-7-oxohept-2-enoate (ZED1096, 1,10 mmol) werden in einer Mischung aus 15 ml Dichlormethan und 15 ml TFA gelöst und für 1 h bei Raumtemperatur gerührt, bevor die volatilen Bestandteile i. Vak. entfernt werden. Das erhaltene Öl wird im Hochvakuum getrocknet, in 20 ml DMF gelöst und 412 µl DIPEA (2,42 mmol) sowie 125 µl Essigsäureanhydrid (1,32 mmol) zugegeben. Durch sukzessiven Zusatz von DIPEA wird der pH-Wert auf ca. 7 eingestellt. Es wird 3 h bei Raumtemperatur gerührt, bevor das Lösungsmittel i. Vak. entfernt wird. Der Rückstand wird mittels präparativer HPLC (35 % ACN in Wasser, 50 ml/min, Gradient 1 % pro min) gereinigt.
Ausbeute: 238 mg, 0,53 mmol, 48 %
471,4 [M+Na]⁺
¹H-NMR (DMSO-d6, 500 MHz): δ[ppm] = 9,22 (s, 1H), 8,35 (d, 1H), 8,18 (dd, 1H), 8,11 (t, 1H), 7,75 (d, 1H), 7,32 (dd, 1H), 6,90 (dt, 1H, J = 15,64; 6,85), 6,24 (t, 1H), 5,85 (d, 1H, J = 15,64), 4,58 (s, 2H), 4,41 (m, 1H), 3,63 (s, 3H), 3,09 (t, 2H), 2,27 (m, 2H), 1,90 (m, 1H), 1,89 (s, 3H), 1,73 (m, 1H), 1,59 (m, 1H), 1,31 (m, 2H), 0,86 (d, 6H)

### 3. Darstellung der Beispielverbindung (A61) (ZED1219)

### tert-Butyl 2-(3-amino-2-oxopyridin-1(2H)-yl)acetate (ZED1095)

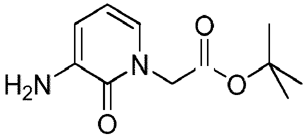

Summenformel: C11H16N203
Molekulargewicht: 224,26

2,00 g tert-Butyl 2-(3-(benzyloxycarbonylamino)-2-oxopyridin-1(2H)-yl)acetate werden unter Stickstoffatmosphäre in 70 ml Ethanol gelöst. Zu dieser Lösung werden 200 mg Pd/C (10%) gegeben und unter Wasserstoffatmosphäre bei Atmosphärendruck für 1,5 h bei Raumtemperatur gerührt. Der Katalysator wird durch Filtration über Silcagel abgetrennt, bevor das Lösungsmittel i. Vak. entfernt wird. Das Rohprodukt wird ohne weitere Reinigung zur Weiterverarbeitung verwendet.
Ausbeute: 1,35 g, >100%
ESI-MS: 225,1 [M+H]⁺

### (S,E)-Methyl 7-(1-(2-tert-butoxy-2-oxoethyl)-2-oxo-1,2-dihydropyridin-3-yl-amino)-6-(tert-butoxycarbonylamino)-7-oxohept-2-enoate (ZED1209)

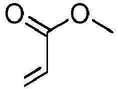

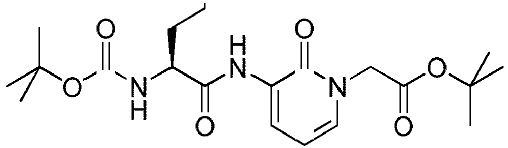

Summenformel: C24H35N3O8
Molekulargewicht: 493,55

Es wird eine Lösung aus 292 mg tert-Butyl 2-(3-amino-2-oxopyridin-1(2H)-yl)acetate (ZED1095, 1,30 mmol) in 5 ml DMF vorgelegt. Dazu wird eine Lösung aus 2,97 g (S,E)-2-(tert-butoxycarbonylamino)-7-methoxy-7-oxohept-5-enoic acid (1a1, 1,30 mmol), 494 mg HATU (1,30 mmol) und 442 µl DIPEA (2 eq) in 5 ml DMF gegeben. Durch sukzessiven Zusatz von DIPEA wird der pH-Wert auf ca. 7 eingestellt. Das Reaktionsgemisch wird bei RT über Nacht gerührt, bevor das Lösungsmittel i. Vak. entfernt wird. Der erhaltene Rückstand wird in 75 ml Ethylacetat aufgenommen und nacheinander je dreimal mit 10%iger Citronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Man erhält ein blaues Gel, das ohne weitere Reinigung zur Weiterverarbeitung geeignet ist.
Ausbeute: 590 mg, 1,20 mmol, 92 %
ESI-MS: 494,2 [M+H]⁺

### (S,E)-2-(3-(2-(tert-butoxycarbonylamino)-7-methoxy-7-oxohept-5-enamido)-2-oxopyridin-1(2H)-yl)acetic acid (ZED1211)

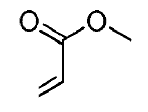

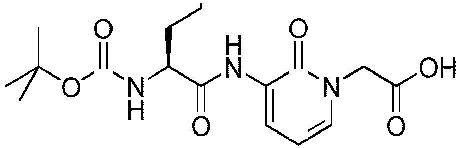

Summenformel: C20H27N3O8
Molekulargewicht: 437,44

590 mg (S,E)-Methyl 7-(1-(2-tert-butoxy-2-oxoethyl)-2-oxo-1,2-dihydropyridin-3-yl-amino)-6-(tert-butoxycarbonylamino)-7-oxohept-2-enoate (ZED1209, 1,20 mmol) werden in 10 ml Dichlormethan gelöst und zur Lösung 10 ml Trifluoressigsäure zugesetzt. Bei Raumtemperatur wird für 1 h gerührt. Das Lösungsmittel wird i. Vak. entfernt und der Rückstand im Hochvakuum getrocknet. Das erhaltene Öl wird ohne Reinigung weiter umgesetzt.

Dazu wird das Öl in 15 ml DMF aufgenommen und mit 173 µl DIPEA versetzt. Durch sukzessiven Zusatz von DIPEA wird der pH-Wert auf ca. 7 eingestellt. Es werden 285 mg Boc-OSu (1,1 eq) zugegeben und bei Raumtemperatur über Nacht gerührt. Das Lösungsmittel wird i. Vak. entfernt und der Rückstand wird mittels präparativer HPLC (30 % ACN in Wasser, 8 ml/min, Gradient 1 % pro min) gereinigt.
Ausbeute: 201 mg, 0,46 mmol, 38 %
ESI-MS: 438,2 [M+H]⁺

### (S,E)-methy 7-(1-(2-((2S,3R)-1-tert-butoxy-3-methyl-1-oxopentan-2-ylamino)-2-oxoethyl)-2-oxo-1,2-dihydropyridin-3-ylamino)-6-(tert-butoxycarbonylamino)-7-oxohept-2-enoate (ZED1215)

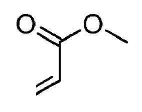

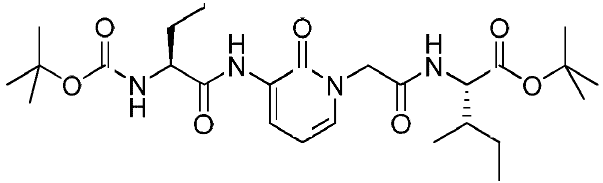

Summenformel: C30H46N4O9
Molekulargewicht: 606,71

Es wird eine Lösung aus 129 mg H-Ile-OtBu*HCl (0,58 mmol, 1,25 eq) in 4 ml DMF vorgelegt. Dazu wird eine Lösung aus 201 mg (S,E)-2-(3-(2-(tert-butoxycarbonylamino)-7-methoxy-7-oxohept-5-enamido)-2-oxopyridin-1 (2H)-yl)acetic acid (ZED1211, 0,46 mmol), 129 mg HATU (0,46 mmol) und 176 µl DIPEA (2,25 eq) in 4 ml DMF gegeben. Durch sukzessiven Zusatz von DIPEA wird der pH-Wert auf ca. 7 eingestellt. Das Reaktionsgemisch wird bei RT über Nacht gerührt, bevor das Lösungsmittel i. Vak. entfernt wird. Der erhaltene Rückstand wird in 75 ml Ethylacetat aufgenommen und nacheinander je dreimal mit 10%iger Citronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Man erhält ein Öl, das ohne weitere Reinigung zur Weiterverarbeitung geeignet ist.
Ausbeute: 310 mg, >100 %
ESI-MS: 607,3 [M+H]⁺

### (2S,3R)-2-(2-(3-((S,E)-2-benzamido-7-methoxy-7-oxohept-5-enamido)-2-oxopyridin-1(2H)-yl)acetamido)-3-methylpentanoic acid (A61) (ZED1219)

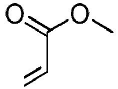

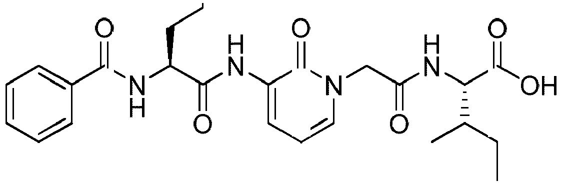

Summenformel: C28H34N4O8
Molekulargewicht: 554,59

155 mg (S,E)-methyl 7-(1-(2-((2S,3R)-1-tert-butoxy-3-methyl-1-oxopentan-2-ylamino)-2-oxoethyl)-2-oxo-1,2-dihydropyridin-3-ylamino)-6-(tert-butoxycarbonylamino)-7-oxohept-2-enoate (ZED1215, 0,46 mmol) werden in einer Mischung aus 10 ml Dichlormethan und 10 ml TFA gelöst und für 1,5 h bei RT gerührt, bevor die volatilen Bestandteile i. Vak. entfernt und im Hochvakuum getrocknet werden.

137 mg (0,23 mmol) des erhaltenen Öls werden in 2,5 ml DMF gelöst. Dazu wird eine Lösung aus 87 mg HATU (0,23 mmol), 23 mg Benzoesäure (0,23 mmol) und 78 µl DIPEA (2 eq) in 2 ml DMF gegeben. Durch sukzessiven Zusatz von DIPEA wird der pH-Wert auf ca. 7 eingestellt. Es wird über Nacht bei Raumtemperatur gerührt, bevor das Lösungsmittel i. Vak. entfernt wird. Der Rückstand wird mittels präparativer HPLC (30 % ACN in Wasser, 8 ml/min, Gradient 1 % pro min) gereinigt.
Ausbeute: 74 mg, 0,13 mmol, 58 %
ESI-MS: 577,4 [M+Na]⁺
¹H-NMR (DMSO-d6, 500 MHz): δ[ppm] = 12,64 (br, 1H), 9,33 (s, 1H), 8,83 (d, 1H), 8,41 (d, 1H), 8,22 (dd, 1H), 7,89 (m, 2H), 7,51 (m, 3H), 7,33 (dd, 1H), 6,94 (dt, 1H, J = 15,68; 6,83), 6,24 (t, 1H), 5,86 (d, 1H, J = 15,74), 4,69 (m, 3H), 4,21 (m, 1H), 3,61 (s, 3H), 2,34 (m, 2H), 2,00 (m, 2H), 1,77 (m, 1H), 1,43 (m, 1H), 1,19 (m, 1H), 0,86 (m, 6H)

### 4. Darstellung der Beispielverbindung (A58) (ZED1213)

### (S,E)-7-tert-Butyl 1-ethyl 6-(bis(tert-butoxycarbonyl)amino)hept-2-enedioate (ZED724)

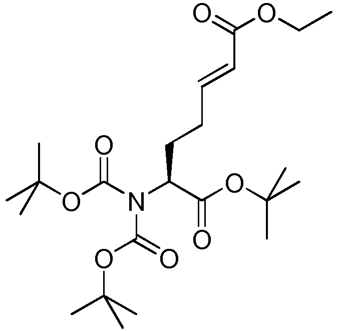

Summenformel: C23H39NO8
Molekulargewicht: 457,56 1,06 g Boc₂-Glu(H)-OtBu (2,74 mmol) werden in 27 ml trockenem Benzol vorgelegt und unter Argonatmosphäre bei Raumtemperatur eine Lösung aus 1,00 g (Ethoxycarbonylmethylen)-triphenylphosphoran (2,74 mmol) in 13 ml Benzol zugetropft. Nach Rühren über Nacht wird das Lösungsmittel i. Vak. entfernt. Der erhaltene Rückstand wird durch Chromatographie an Kieselgel gereinigt (Säule: 25*6,0 cm, Dichlormethan/Methanol 99,5:0,5).
SC: in 50 ml Fraktionen gesammelt, Produkt: Fraktionen 17-55
DC-Kontrolle: Dichlormethan/Methanol 99,5:0,5, R_{f} = 0,23
Ausbeute: 972 mg, 2,72 mmol, 78 %
ESI-MS: 480,3 [M+Na]⁺

### (S,E)-2-(tert-butoxycarbonylamino)-7-ethoxy-7-oxohept-5-enoic acid (ZED775)

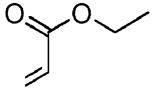

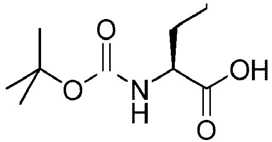

Summenformel: C14H23NO6
Molekulargewicht: 301,34

### 972 mg (S,E)-7-tert-Butyl 1-ethyl 6-(bis(tert-butoxycarbonyl)amino)hept-2-enedioate (ZED724, 2,12 mmol) werden in 15 ml Dichlormethan gelöst und zur Lösung 15 ml Trifluoressigsäure zugesetzt. Bei Raumtemperatur wird für 2 h gerührt. Das Lösungsmittel wird i. Vak. entfernt und der Rückstand im Hochvakuum getrocknet. Das erhaltene Öl wird ohne Reinigung weiter umgesetzt.

Dazu wird das Öl in 30 ml DMF aufgenommen und mit 361 µl DIPEA versetzt. Durch sukzessiven Zusatz von DIPEA wird der pH-Wert auf ca. 7 eingestellt. Es werden 502 mg Boc-OSu (2,33 mmol, 1,1 eq) zugegeben und bei Raumtemperatur über Nacht gerührt. Das Lösungsmittel wird i. Vak. entfernt und der Rückstand durch Chromatographie an Kieselgel gereinigt (Säule: 23*6,0 cm, Dichlormethan/Methanol 9:1). Man erhält ein farbloses Öl.
SC: in 50 ml Fraktionen gesammelt, Produkt: Fraktionen 35-80
DC-Kontrolle: Dichlormethan/Methanol 9:1, R_{f} = 0,21
Ausbeute: 249 mg, 0,83 mmol, 39 %
ESI-MS: 302,2 [M+H]⁺

### (S,E)-Ethyl 6-(tert-butoxycarbonylamino)-7-(1-(2-(2-ethylbutylamino)-2-oxoethyl)-2-oxo-1,2-dihydropyridin-3-ylamino)-7-oxohept-2-enoate (ZED1105)

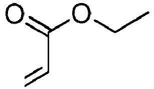

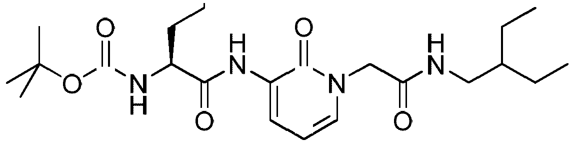

Summenformel: C27H42N4O7
Molekulargewicht: 534,64

Es wird eine Lösung aus 101 mg 2-(3-amino-2-oxopyridin-1(2H)-yl)-N-(2-ethylbutyl)acetamide (2a, 0,40 mmol) in 2,5 ml DMF vorgelegt. Dazu wird eine Lösung aus 121 mg (S,E)-2-(tert-butoxycarbonylamino)-7-ethoxy-7-oxohept-5-enoic acid (ZED775, 0,40 mmol), 152 mg HATU (0,40 mmol) und 136 µl DIPEA (2 eq) in 2,5 ml DMF gegeben. Durch sukzessiven Zusatz von DIPEA wird der pH-Wert auf ca. 7 eingestellt. Das Reaktionsgemisch wird bei 40 °C für 1,5 Stunden und anschließend bei RT über Nacht gerührt, bevor das Lösungsmittel i. Vak. entfernt wird. Der erhaltene Rückstand wird in 75 ml Ethylacetat aufgenommen und nacheinander je dreimal mit 10%iger Citronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Man erhält ein Gel, das ohne weitere Reinigung zur Weiterverarbeitung geeignet ist.
Ausbeute: 155 mg, 0,29 mmol, 73 %
ESI-MS: 535,3 [M+H]⁺

### (S,E)-Ethyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (A58) (ZED1213)

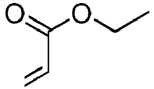

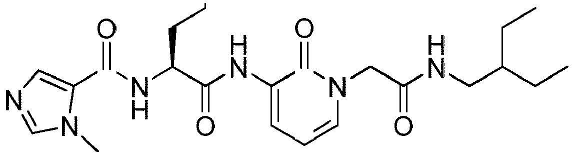

Summenformel: C27H38N6O6
Molekulargewicht: 542,63

155 mg (S,E)-Methyl 6-(tert-butoxycarbonylamino)-7-(1-(2-(2-ethylbutylamino)-2-oxo-ethyl)-2-oxo-1,2-dihydropyridin-3-ylamino)-7-oxohept-2-enoate (ZED1223, 0,29 mmol) werden in einer Mischung aus 10 ml Dichlormethan und 10 ml TFA gelöst und für 1 h bei RT gerührt, bevor die volatilen Bestandteile i. Vak. entfernt werden.

Das erhaltene grünliche Öl wird im Hochvakuum getrocknet und in 2 ml DMF gelöst. Dazu wird eine Lösung aus 110 mg HATU (0,29 mmol), 38 mg 1-Methyl-1H-imidazol-5-carbonsäure (0,29 mmol) und 99 µl DIPEA (2 eq) in 2 ml DMF gegeben. Durch sukzessiven Zusatz von DIPEA wird der pH-Wert auf ca. 7 eingestellt. Es wird über Nacht bei Raumtemperatur gerührt, bevor das Lösungsmittel i. Vak. entfernt wird. Der Rückstand wird mittels präparativer HPLC (30 % ACN in Wasser, 8 ml/min, Gradient 1 % pro min) gereinigt.
Ausbeute: 64 mg, 0,12 mmol, 41 %
ESI-MS: 543,4 [M+H]⁺
¹H-NMR (DMSO-d6, 500 MHz): δ[ppm] = 9,47 (s, 1H), 8,99 (d, 1H), 8,94 (s, 1H), 8,19 (m, 2H), 8,06 (d, 1H), 7,34 (dd, 1H), 6,92 (dt, 1H, J = 15,63; 6,83), 6,25 (t, 1H), 5,85 (d, 1H, J = 15,66), 4,73 (m, 1H), 4,59 (s, 2H), 4,08 (q, 2H), 3,95 (s, 3H), 3,02 (t, 2H), 2,34 (m, 2H), 2,02 (m, 1H), 1,89 (m, 1H), 1,27 (m, 5H), 1,19 (t, 3H), 0,83 (t, 6H)

### 5. Darstellung der Beispielverbindung (A6) (ZED1029)

### (S,E)-7-tert-butyl 1-isopropyl 6-(bis(tert-butoxycarbonyl)amino)hept-2-enedioate (ZED855)

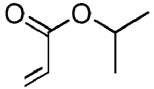

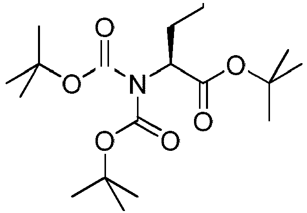

Summenformel: C24H41NO8
Molekulargewicht: 471,58

951 mg Boc₂-Glu(H)-OtBu (2,45 mmol) werden in 20 ml trockenem Benzol vorgelegt und unter Argonatmosphäre bei Raumtemperatur ein Lösung aus 889 mg (Isopropoxycarbonylmethylen)-triphenylphosphoran (2,45 mmol) in 10 ml Benzol zugetropft. Nach Rühren über Nacht wird das Lösungsmittel i. Vak. entfernt. Der erhaltene Rückstand wird durch Chromatographie an Kieselgel gereinigt (Säule: 39*3,2 cm, Dichlormethan/Methanol 99,5:0,5).
SC: in 50 ml Fraktionen gesammelt, Produkt: Fraktionen 9-20
DC-Kontrolle: Dichlormethan/Methanol 99,5:0,5, R_{f} = 0,32
Ausbeute: 961 mg, 2,04 mmol, 83 %
ESI-MS: 472,3 [M+H]⁺

### (S,E)-2-(tert-butoxycarbonylamino)-7-isopropoxy-7-oxohept-5-enoic acid (ZED902)

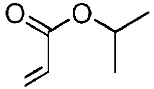

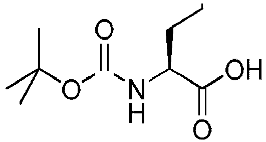

Summenformel: C15H25NO6
Molekulargewicht: 315,36

518 mg (S,E)-7-tert-butyl 1-isopropyl 6-(bis(tert-butoxycarbonyl)amino)hept-2-enedioate (ZED855, 1,10 mmol) werden in 10 ml Dichlormethan gelöst und zur Lösung 10 ml Trifluoressigsäure zugesetzt. Bei Raumtemperatur wird für 2 h gerührt. Das Lösungsmittel wird i. Vak. entfernt und der Rückstand im Hochvakuum getrocknet. Das erhaltene Öl wird ohne Reinigung weiter umgesetzt.

Dazu wird das Öl in 2,5 ml DMF aufgenommen und mit 185 µl DIPEA versetzt. Durch sukzessiven Zusatz von DIPEA wird der pH-Wert auf ca. 7 eingestellt. Es werden 259 mg Boc-OSu (1,21 mmol, 1,1 eq) zugegeben und bei Raumtemperatur über Nacht gerührt. Das Lösungsmittel wird i. Vak. entfernt und der Rückstand in 75 ml Ethylacetat aufgenommen, zweimal mit 10%iger Citronensäure- und einmal mit gesättigter NaCl-Lsg. gewaschen. Nach Trocknen der organischen Phase über Na₂SO₄ wird das Lösungsmittel i. Vak. entfernt. Der Rückstand wird mittels präparativer HPLC (40 % ACN in Wasser, 8 ml/min, Gradient 1 % pro min) gereinigt.
Ausbeute: 267 mg, 0,85 mmol, 77 %
ESI-MS: 316,2 [M+H]⁺

### (S,E)-Isopropyl 6-(tert-butoxycarbony)amino)-7-(1-(2-(isopentylamino)-2-oxoethyl)-2-oxo-1,2-dihydropyridin-3-ylamino)-7-oxohept-2-enoate (ZED1024)

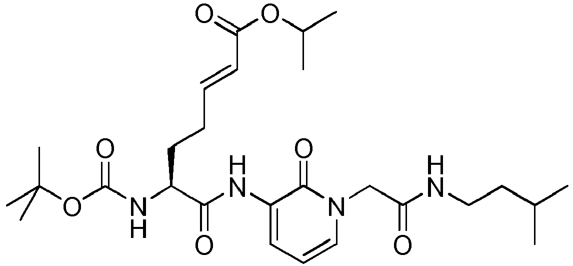

Summenformel: C27H42N4O7
Molekulargewicht: 534,64

Es wird eine Lösung aus 214 mg 2-(3-Amino-2-oxopyridin-1 (2H)-yl)-N-isopentylacetamide (ZED1022, 0,90 mmol) in 10 ml DMF vorgelegt. Dazu wird eine Lösung aus 284 mg (S,E)-2-(tert-butoxycarbonylamino)-7-isopropoxy-7-oxohept-5-enoic acid (ZED902, 0,90 mmol), 342 mg HATU (0,90 mmol) und 306 µl DIPEA (2 eq) in 10 ml DMF gegeben. Durch sukzessiven Zusatz von DIPEA wird der pH-Wert auf ca. 7 eingestellt. Das Reaktionsgemisch wird bei 40 °C für 2,5 h sowie bei Raumtemperatur über Nacht gerührt, bevor das Lösungsmittel i. Vak. entfernt wird. Der erhaltene Rückstand wird in 75 ml Ethylacetat aufgenommen und nacheinander je dreimal mit 10%iger Zitronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Der Rückstand wird mittels präparativer HPLC (50 % ACN in Wasser, 8 ml/min, Gradient 1 % pro min) gereinigt.
Ausbeute: 295 mg, 0,55 mmol, 61 %
ESI-MS: 535,4 [M+H]⁺

### (S,E)-isopropyl 6-acetamido-7-(1-(2-(isopentylamino)-2-oxoethyl)-2-oxo-1,2-di-hydropyridin-3-ylamino)-7-oxohept-2-enoate (A6) (ZED1029)

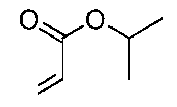

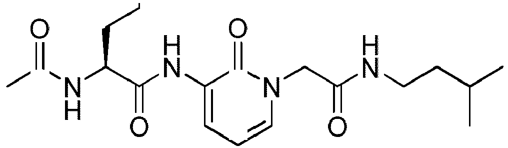

Summenformel: C24H36N4O6
Molekulargewicht: 476,57

171 mg (S,E)-Isopropyl 6-(tert-butoxycarbonylamino)-7-(1-(2-(isopentylamino)-2-oxo-ethyl)-2-oxo-1,2-dihydropyridin-3-ylamino)-7-oxohept-2-enoate (ZED1024, 0,32 mmol) werden in einer Mischung aus 5 ml Dichlormethan und 5 ml TFA gelöst und für 1 h bei Raumtemperatur gerührt, bevor die volatilen Bestandteile i. Vak. entfernt werden. Das erhaltene Öl wird im Hochvakuum getrocknet, in 10 ml DMF gelöst und 113 µl DIPEA (2 eq) sowie 36 µl Essigsäureanhydrid (0,39 mmol) zugegeben. Durch sukzessiven Zusatz von DIPEA wird der pH-Wert auf ca. 7 eingestellt. Es wird 3 h bei Raumtemperatur gerührt, bevor das Lösungsmittel i. Vak. entfernt wird. Der Rückstand wird mittels präparativer HPLC (40 % ACN in Wasser, 8 ml/min, Gradient 1 % pro min) gereinigt.
Ausbeute: 111 mg, 0,23 mmol, 73 %
ESI-MS: 477,3 [M+H]⁺
¹H-NMR (CDCl₃, 500 MHz): δ[ppm] = 8,96 (s, 1H), 8,41 (dd, 1H), 7,14 (dd, 1H), 6,88 (dt, 1H, J = 15,64; 6,86), 6,79 (s, 1H), 6,33 (t, 1H), 5,80 (d, 1H, J = 15,66), 5,17 (s, 1H), 5,01 (m, 1H), 4,58 (m, 2H), 4,41 (s, 1H), 3,24 (m, 2H), 2,31 (m, 2H), 2,09 (m, 1H), 1,86 (s, 3H), 1,83 (m, 1H), 1,56 (m, 1H), 1,37 (m, 2H), 1,24 (d, 6H), 0,88 (d, 6H)

### 6. Darstellung der Beispielverbindung (A77) (ZED1393)

### (S,E)-tert-butyl 2-(bis(tert-butoxycarbonyl)amino)-6-(methylsulfonyl)hex-5-enoate (ZED865)

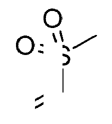

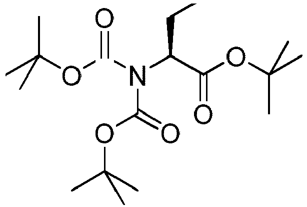

Summenformel: C21H37NO8S
Molekulargewicht: 463,59

10 mg NaH (60 %, 0,26 mmol) werden unter Argonatmosphäre vorgelegt. Eine Lösung aus 59 mg Diethyl-methylsulfonylmethylphosphonat (0,26 mmol) in 3 ml DMF wird zugegeben und bei RT gerührt bis die Gasentwicklung beendet ist. Eine Lösung aus 100 mg Boc₂-Glu(H)-OtBu (0,26 mmol) in 0,5 ml DMF wird zugetropft und bei RT über Nacht gerührt. Das Lösungsmittel wird i. Vak. entfernt. Der erhaltene Rückstand wird durch Chromatographie an Kieselgel gereinigt (Säule: 29*2,3 cm, Dichlormethan/Methanol 99:1).
SC: in 50 ml Fraktionen gesammelt, Produkt: Fraktionen 6-9
DC-Kontrolle: Dichlormethan/Methanol 99:1, R_{f} = 0,29
Ausbeute: 89 mg, 0,19 mmol, 74 %
ESI-MS: 464,2 [M+H]⁺

### (S,E)-2-(tert-butoxycarbonylamino)-6-(methylsulfonyl)hex-5-enoic acid (ZED1021)

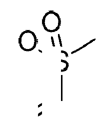

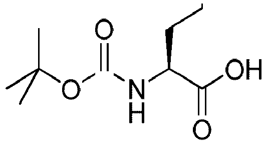

Summenformel: C12H21 N06S
Molekulargewicht: 307,36

356 mg (S,E)-tert-butyl 2-(bis(tert-butoxycarbonyl)amino)-6-(methylsulfonyl)hex-5-enoate (ZED865, 0,77 mmol) werden in 10 ml Dichlormethan gelöst und zur Lösung 10 ml Trifluoressigsäure zugesetzt. Bei Raumtemperatur wird für 1 h gerührt. Das Lösungsmittel wird i. Vak. entfernt und der Rückstand im Hochvakuum getrocknet. Das erhaltene Öl wird ohne Reinigung weiter umgesetzt.

Dazu wird das Öl in 5 ml DMF aufgenommen und mit 131 µl DIPEA versetzt. Durch sukzessiven Zusatz von DIPEA wird der pH-Wert auf ca. 7 eingestellt. Es werden 182 mg Boc-OSu (0,85 µmol, 1,1 eq) zugegeben und bei Raumtemperatur über Nacht gerührt. Das Lösungsmittel wird i. Vak. entfernt und der Rückstand mittels präparativer HPLC (5 % ACN in Wasser, 8 ml/min, Gradient 1 % pro min) gereinigt.
Ausbeute: 148 mg, 0,48 mmol, 63 %
ESI-MS: 308,1 [M+H]⁺

### (S,E)-tert-Butyl 1-(1-(2-(isopentylamino)-2-oxoethyl)-2-oxo-1,2-dihydropyridin-3-ylamino)-6-(methylsulfonyl)-1-oxohex-5-en-2-ylcarbamate (ZED1025)

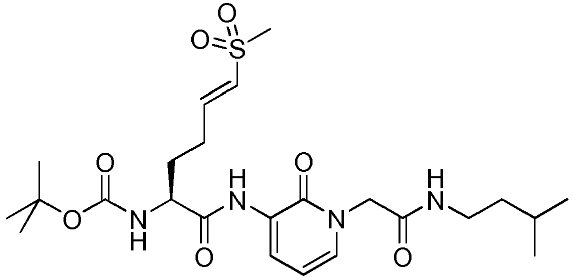

Summenformel: C24H38N4O7S
Molekulargewicht: 526,65

Es wird eine Lösung aus 160 mg 2-(3-Amino-2-oxopyridin-1 (2H)-yl)-N-isopentylacetamide (ZED1022, 0,69 mmol) in 5 ml DMF vorgelegt. Dazu wird eine Lösung aus 212 mg (S,E)-2-(tert-butoxycarbonylamino)-6-(methylsulfonyl)hex-5-enoic acid (ZED1021, 0,69 mmol), 260 mg HATU (0,69 mmol) und 234 µl DIPEA (2 eq) in 5 ml DMF gegeben. Durch sukzessiven Zusatz von DIPEA wird der pH-Wert auf ca. 7 eingestellt. Das Reaktionsgemisch wird bei 40 °C für 2,5 h sowie bei Raumtemperatur über Nacht gerührt, bevor das Lösungsmittel i. Vak. entfernt wird. Der erhaltene Rückstand wird in 75 ml Ethylacetat aufgenommen und nacheinander je dreimal mit 10%iger Zitronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Der Rückstand wird ohne Reinigung weiter verarbeitet.
Ausbeute: 251 mg, 0,48 mmol, 69 %
ESI-MS: 527,3 [M+H]⁺

### (S,E)-2-acetamido-N-(1-(2-(isopentylamino)-2-oxoethyl)-2-oxo-1,2-dihydropyridin-3-yl)-6-(methylsulfonyl)hex-5-enamide (A77) (ZED1393)

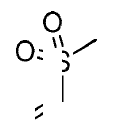

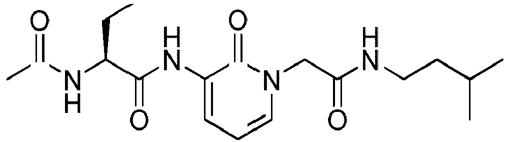

Summenformel: C21H32N4O6S
Molekulargewicht: 468,57

251 mg (S,E)-tert-Butyl 1-(1-(2-(isopentylamino)-2-oxoethyl)-2-oxo-1,2-dihydropyridin-3-ylamino)-6-(methylsulfonyl)-1-oxohex-5-en-2-ylcarbamate (ZED1025, 0,48 mmol) werden in einer Mischung aus 10 ml Dichlormethan und 10 ml TFA gelöst und für 1 h bei Raumtemperatur gerührt, bevor die volatilen Bestandteile i. Vak. entfernt werden. Das erhaltene Öl wird im Hochvakuum getrocknet, in 10 ml DMF gelöst und 163 µl DIPEA (2 eq) sowie 54 µl Essigsäureanhydrid (0,58 mmol) zugegeben. Durch sukzessiven Zusatz von DIPEA wird der pH-Wert auf ca. 7 eingestellt. Es wird 3 h bei Raumtemperatur gerührt, bevor das Lösungsmittel i. Vak. entfernt wird. Der Rückstand wird mittels präparativer HPLC (30 % ACN in Wasser, 8 ml/min, Gradient 1 % pro min) gereinigt.
Ausbeute: 130 mg, 0,28 mmol, 58 %
ESI-MS: 469,2 [M+H]⁺
¹H-NMR (CDCl₃, 500 MHz): δ[ppm] = 8,98 (s, 1H), 8,41 (dd, 1H), 7,11 (dd, 1H), 6,86 (dt, 1H, J = 15,63; 6,89), 6,80 (s, 1H), 6,33 (t, 1H), 5,78 (d, 1H, J = 15,67), 5,11 (s, 1H), 4,56 (m, 2H), 4,43 (s, 1H), 3,27 (m, 2H), 2,87 (s, 3H), 2,30 (m, 2H), 2,09 (m, 1H), 1,87 (s, 3H), 1,84 (m, 1H), 1,58 (m, 1H), 1,33 (m, 2H), 0,87 (d, 6H)

### 7. Darstellung der Beispielverbindung (A78) (ZED1397)

### (S,E)-1-Benzyl 7-tert-butyl 6-(bis(tert-butoxycarbonyl)amino)hept-2-enedioate (ZED818)

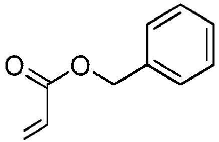

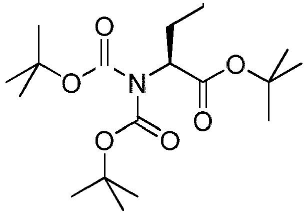

Summenformel: C28H41NO8
Molekulargewicht: 519,63

100 mg Boc₂-Glu(H)-OtBu (0,26 mmol) werden in 3 ml trockenem Benzol vorgelegt und unter Argonatmosphäre bei Raumtemperatur ein Lösung aus 109 mg Benzyl(triphenylphosphoranyliden)acetat (0,26 mmol) in 2 ml Benzol zugetropft. Nach Rühren über Nacht wird das Lösungsmittel i. Vak. entfernt. Der erhaltene Rückstand wird durch Chromatographie an Kieselgel gereinigt (Säule: 28*2,3 cm, Dichlormethan/Methanol 99:1).
SC: in 50 ml Fraktionen gesammelt, Produkt: Fraktionen 7-9
DC-Kontrolle: Dichlormethan/Methanol 99:1, R_{f} = 0,30
Ausbeute: 94 mg, 0,18 mmol, 70 %
ESI-MS: 520,2 [M+H]⁺

### (S,E)-7-(Benzyloxy)-2-(tert-butoxycarbonylamino)-7-oxohept-5-enoic acid (ZED1394)

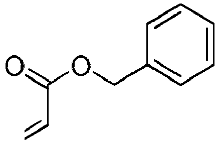

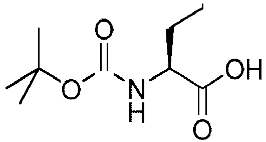

Summenformel: C19H25NO6
Molekulargewicht: 363,40

94 mg (S,E)-1-Benzyl 7-tert-butyl 6-(bis(tert-butoxycarbonyl)amino)hept-2-enedioate (ZED818, 0,18 mmol) werden in 2 ml Dichlormethan gelöst und zur Lösung 2 ml Trifluoressigsäure zugesetzt. Bei Raumtemperatur wird für 1,5 h gerührt. Das Lösungsmittel wird i. Vak. entfernt und der Rückstand im Hochvakuum getrocknet. Das erhaltene Öl wird ohne Reinigung weiter umgesetzt.

Dazu wird das Öl in 2 ml DMF aufgenommen und mit 61 µl DIPEA (2 eq) versetzt. Durch sukzessiven Zusatz von DIPEA wird der pH-Wert auf ca. 7 eingestellt. Es werden 43 mg Boc-OSu (0,20 mmol, 1,1 eq) zugegeben und bei Raumtemperatur über Nacht gerührt. Das Lösungsmittel wird i. Vak. entfernt und der Rückstand mittels präparativer HPLC (35 % ACN in Wasser, 8 ml/min, Gradient 1 % pro min) gereinigt.
Ausbeute: 60 mg, 0,16 mmol, 91 %
ESI-MS: 364,1 [M+H]⁺

### (S,E)-Benzyl 6-(tert-butoxycarbonylamino)-7-(1-(2-(isopentylamino)-2-oxoethyl)-2-oxo-1,2-dihydropyridin-3-ylamino)-7-oxohept-2-enoate (ZED1396)

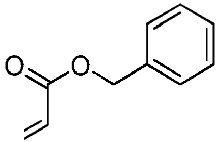

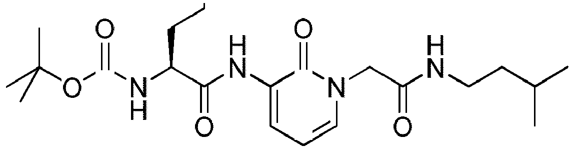

Summenformel: C31H42N4O7
Molekulargewicht: 582,69

Es wird eine Lösung aus 38 mg 2-(3-Amino-2-oxopyridin-1(2H)-yl)-N-iso-pentylacetamide (ZED1022, 0,16 mmol) in 2 ml DMF vorgelegt. Dazu wird eine Lösung aus 60 mg (S,E)-7-(Benzyloxy)-2-(tert-butoxycarbonylamino)-7-oxohept-5-enoic acid (ZED1394, 0,16 mmol), 61 mg HATU (0,16 mmol) und 54 µl DIPEA (2 eq) in 2 ml DMF gegeben. Durch sukzessiven Zusatz von DIPEA wird der pH-Wert auf ca. 7 eingestellt. Das Reaktionsgemisch wird bei 40 °C für 3 h sowie bei Raumtemperatur über Nacht gerührt, bevor das Lösungsmittel i. Vak. entfernt wird. Der erhaltene Rückstand wird in 40 ml Ethylacetat aufgenommen und nacheinander je dreimal mit 10%iger Zitronensäure, ges. NaHCO₃-Lsg. und ges. NaCl-Lsg. gewaschen. Die organische Phase wird über Na₂SO₄ getrocknet, abfiltriert und i. Vak. zur Trockne eingeengt. Der Rückstand wird ohne Reinigung weiter verarbeitet.
Ausbeute: 86 mg, 0,15 mmol, 92 %
ESI-MS: 583,3 [M+H]⁺

### (S,E)-Benzyl 6-acetamido-7-(1-(2-(isopentylamino)-2-oxoethyl)-2-oxo-1,2-dihydro-pyridin-3-ylamino)-7-oxohept-2-enoate A78 (ZED1397)

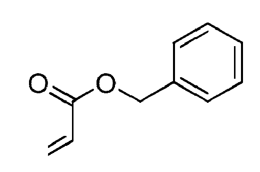

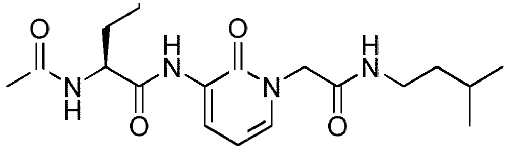

Summenformel: C28H36N4O6
Molekulargewicht: 524,61

86 mg (S,E)-Benzyl 6-(tert-butoxycarbonylamino)-7-(1-(2-(isopentylamino)-2-oxoethyl)-2-oxo-1,2-dihydropyridin-3-ylamino)-7-oxohept-2-enoate (ZED1396, 0,15 mmol) werden in einer Mischung aus 3 ml Dichlormethan und 3 ml TFA gelöst und für 1 h bei Raumtemperatur gerührt, bevor die volatilen Bestandteile i. Vak. entfernt werden. Das erhaltene Öl wird im Hochvakuum getrocknet, in 4 ml DMF gelöst und 51 µl DIPEA (2 eq) sowie 14 µl Essigsäureanhydrid (0,58 mmol) zugegeben. Durch sukzessiven Zusatz von DIPEA wird der pH-Wert auf ca. 7 eingestellt. Es wird 4 h bei Raumtemperatur gerührt, bevor das Lösungsmittel i. Vak. entfernt wird. Der Rückstand wird mittels präparativer HPLC (35 % ACN in Wasser, 8 ml/min, Gradient 1 % pro min) gereinigt.
Ausbeute: 49 mg, 0,09 mmol, 62 %
ESI-MS: 525,3 [M+H]⁺
¹H-NMR (CDCl₃, 500 MHz): δ[ppm] = 8,96 (s, 1H), 8,39 (dd, 1H), 7,37 (m, 5H), 7,10 (dd, 1H), 6,87 (dt, 1H, J = 15,64; 6,85), 6,80 (s, 1H), 6,32 (t, 1H), 5,80 (d, 1H, J = 15,68), 5,14 (s, 1H), 5,08 (s, 1H), 4,58 (m, 2H), 4,40 (s, 1H), 3,27 (m, 2H), 2,31 (m, 2H), 2,10 (m, 1H), 1,85 (s, 3H), 1,82 (m, 1H), 1,57 (m, 1H), 1,32 (m, 2H), 0,87 (d, 6H)

### 8. Ausführungsbeispiele erfindungsgemäßer Verbindungen

### (S,E)-Ethyl 6-(benzyloxycarbonylamino)-7-oxo-7-(2-oxo-1-(2-oxo-2-(2,4,6-trimethyl-phenethylamino)ethyl)-1,2-dihydropyridin-3-ylamino)hept-2-enoate (A1)

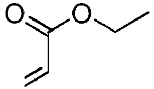

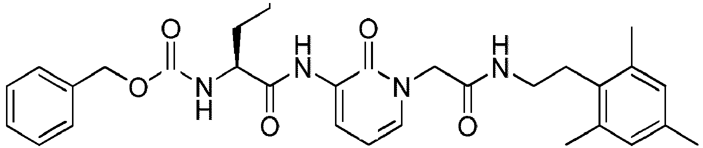

Summenformel: C35H42N4O7
Molekulargewicht: 630,73
ESI-MS: 583,3 [M+H]⁺

### (S,E)-Ethyl 6-(benzyloxycarbonylamino)-7-(1-(2-(isopentylamino)-2-oxoethyl)-6-methyl-2-oxo-1,2-dihydropyridin-3-ylamino)-7-oxohept-2-enoate (A2)

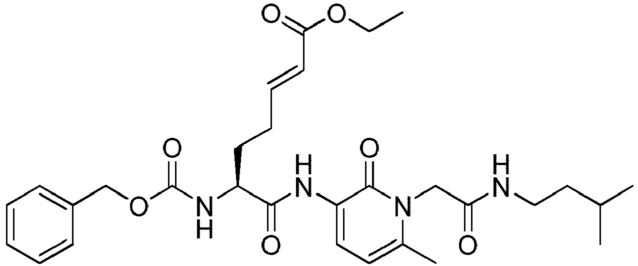

Summenformel: C30H40N4O7
Molekulargewicht: 568,66
ESI-MS: 569,3 [M+H]⁺

### (S,E)-Ethyl 6-(benzyloxycarbonylamino)-7-(1-(2-(isopentylamino)-2-oxoethyl)-2-oxo-1,2-dihydropyridin-3-ylamino)-7-oxohept-2-enoate (A3)

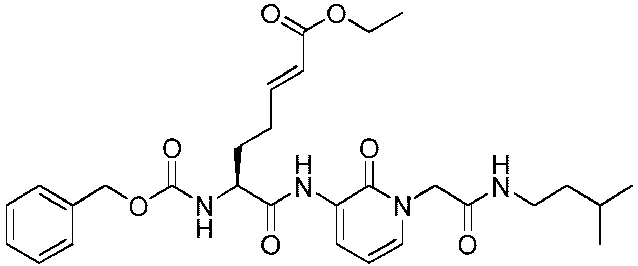

Summenformel: C29H38N4O7
Molekulargewicht: 554,63
ESI-MS: 555,3 [M+H]⁺

### (S,E)-Ethyl 6-acetamido-7-(1-(2-(isopentylamino)-2-oxoethyl)-6-methyl-2-oxo-1,2-dihydropyridin-3-ylamino)-7-oxohept-2-enoate (A4)

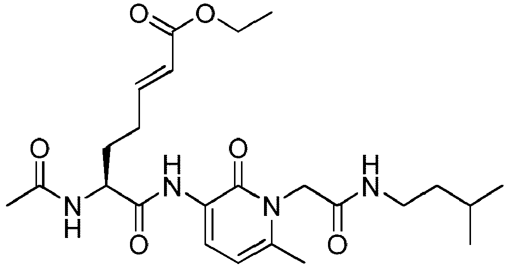

Summenformel: C24H36N4O6
Molekulargewicht: 476,57
ESI-MS: 555,3 [M+H]⁺
500-MHz-¹H-NMR-cosy (DMSO_{d6}): δ[ppm] = 8,19 (d, 1H, H-3), 7,88 (d, 1H, H-6), 7,47 (d, 1H, H-14), 7,37-7,31 (m, 5H, Aryl-H), 6,87 (dt, 1H, H-10, J_{10/9} = 6,6 Hz, J_{11/10} = 15,4 Hz), 5,81 (d, 1H, H-11 , J_{11/10} = 15,4 Hz), 5,02 (s, 2H, Benzyl-CH₂), 4,37 -4,28 (m, 2H, H-5, H-4), 4,28-4,20 (m, 1H, H-2), 4,10 (q, 2H, H-12ₐ, H-12_{b}), 4,08-4,00 (m, 1H, H-7), 3,73-3,67 (m, 1H, H-4cₐ), 3,61 (s, 3H, OMe), 3,60-3,52 (m, 1H, H-4c_{b}), 2,27-2,15 (m, 2H, H-9ₐ, H-9_{b}), 2,10-2,00 (m, 1H, H-4_{a/1}), 2,00-1,90 (m, 2H, H-4_{b/1}, Methin-H(Val)), 1,88-1,78 (m, 3H, H-4_{a/2}, H-4_{b/2}), 1,78-1,65 (m, 2H, H-8ₐ, Methin-H (Leu)), 1,65-1,60 (m, 1H, H-8_{b}), 1,58-1,50 (m, 1H, CH₂ₐ-Leu), 1,50-1,43 (m, 1H, CH_{2b}-Leu), 1,22 (t, 3H, CH₃-16), 0,89 (dd, 6H, 2xCH₃-Val), 0,84 (dd, 6H, 2xCH₃-Leu)

### 3-(2-(3-((S,E)-2-(Benzyloxycarbonylamino)-7-ethoxy-7-oxohept-5-enamido)-6-methyl-2-oxopyridin-1(2H)-yl)acetamido)-5-methylhexanoic acid (A5)

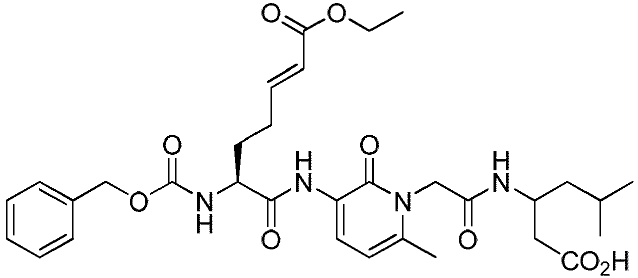

Summenformel: C32H42N4O9
Molekulargewicht: 626,70
ESI-MS: 627,3 [M+H]⁺

### (S,E)-Ethyl 6-acetamido-7-(1-(2-(isopentylamino)-2-oxoethyl)-2-oxo-1,2-dihydro-pyridin-3-ylamino)-7-oxohept-2-enoate (A7)

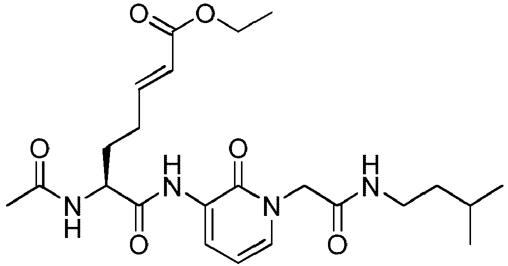

Summenformel: C23H34N4O6
Molekulargewicht: 462,54
ESI-MS: 463,3 [M+H]⁺

### (S,E)-Ethyl 7-(6-methyl-2-oxo-1-(2-oxo-2-(phenethylam ino)ethyl)-1,2-d ihydro-pyridin-3-ylamino)-6-(nicotinamido)-7-oxohept-2-enoate (A8)

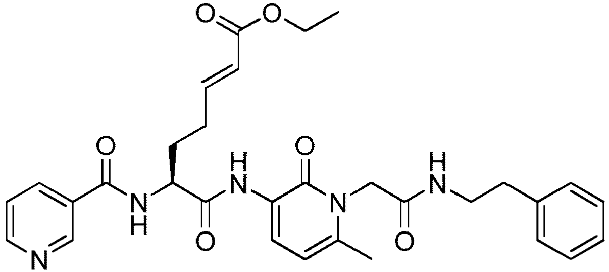

Summenformel: C31H35N5O6
Molekulargewicht: 573,64
ESI-MS: 574,4 [M+H]⁺

### (S,E)-ethyl 6-((4-chlorophenyl)methylsulfonamido)-7-(1-(2-(isopentylamino)-2-oxo-ethyl)-2-oxo-1,2-dihydropyridin-3-ylamino)-7-oxohept-2-enoate (A9)

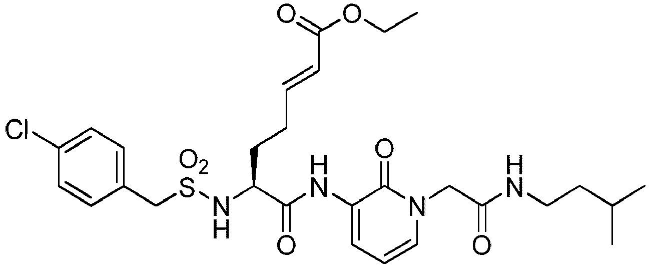

Summenformel: C28H37C1N4O7S
Molekulargewicht: 609,13
ESI-MS: 609,2 [M+H]⁺

### (S,E)-Ethyl 6-benzamido-7-(1-(2-(isopentylamino)-2-oxoethyl)-2-oxo-1,2-dihydro-pyridin-3-ylamino)-7-oxohept-2-enoate (A10)

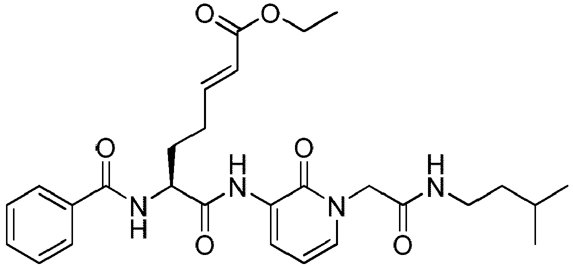

Summenformel: C28H36N4O6
Molekulargewicht: 524,61
ESI-MS: 525,3 [M+H]⁺

### (S,E)-Ethyl 6-(furan-3-carboxamido)-7-(1-(2-(isopentylamino)-2-oxoethyl)-2-oxo-1,2-dihydropyridin-3-ylamino)-7-oxohept-2-enoate (A11)

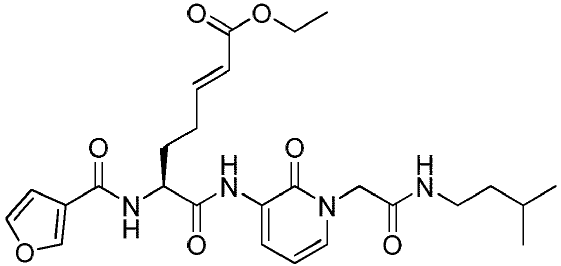

Summenformel: C26H34N4O7
Molekulargewicht: 514,57
ESI-MS: 515,2 [M+H]⁺

### (S,E)-Ethyl 7-(1-(2-(isopentylamino)-2-oxoethyl)-2-oxo-1,2-dihydropyridin-3-ylamino)-7-oxo-6-(thiophene-3-carboxamido)hept-2-enoate (A12)

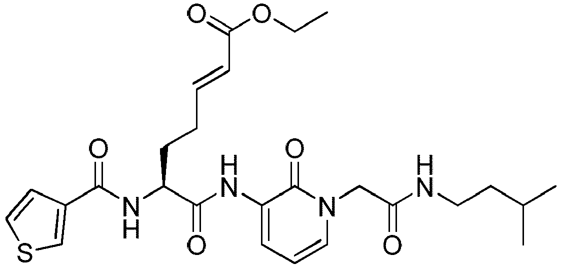

Summenformel: C26H34N4O6S
Molekulargewicht: 530,64
ESI-MS: 531,2 [M+H]⁺

### (S,E)-Ethyl 6-(furan-3-sulfonamido)-7-(1-(2-(isopentylamino)-2-oxoethyl)-2-oxo-1,2-dihydropyridin-3-ylamino)-7-oxohept-2-enoate (A13)

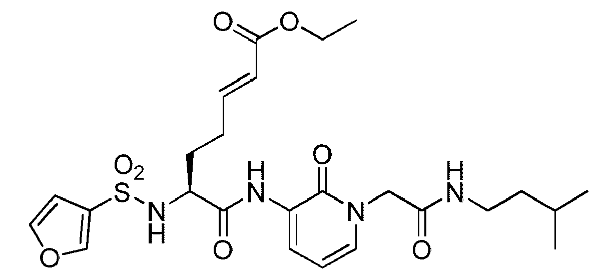

Summenformel: C25H34N4O8S
Molekulargewicht: 550,62
ESI-MS: 551,2 [M+H]⁺

### (S,E)-Ethyl 7-(1-(2-(isopentylamino)-2-oxoethyl)-2-oxo-1,2-dihydropyridin-3-ylamino)-7-oxo-6-(4-sulfamoylbenzamido)hept-2-enoate (A14)

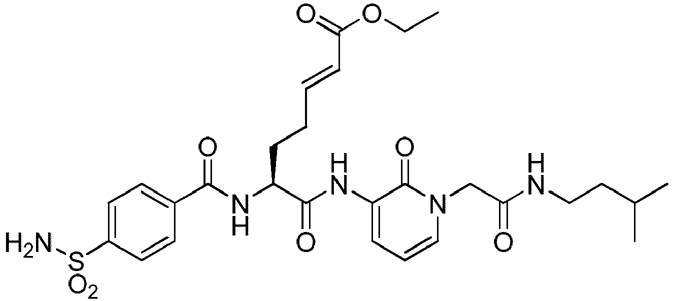

Summenformel: C28H37N5O8S
Molekulargewicht: 603,69
ESI-MS: 604,3 [M+H]⁺

### (S,E)-Ethyl 7-(1-(2-(isopentylamino)-2-oxoethyl)-2-oxo-1,2-dihydropyridin-3-ylamino)-6-(5-methylthiazole-4-carboxamido)-7-oxohept-2-enoate (A15)

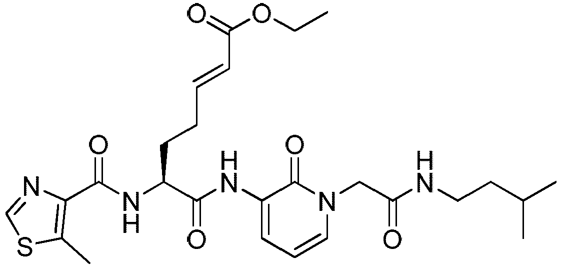

Summenformel: C26H35N5O6S
Molekulargewicht: 545,65
ESI-MS: 546,2 [M+H]⁺

### (S,E)-Ethyl 7-(1-(2-(isopentylamino)-2-oxoethyl)-2-oxo-1,2-dihydropyridin-3-ylamino)-6-(nicotinamido)-7-oxohept-2-enoate (A16)

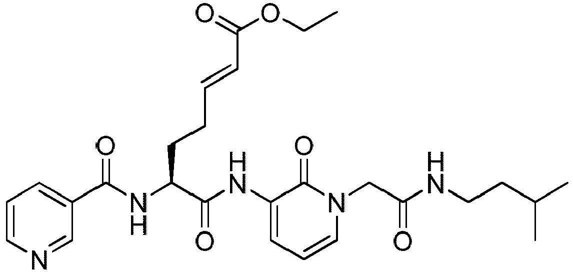

Summenformel: C27H35N5O6
Molekulargewicht: 525,60
ESI-MS: 526,3 [M+H]⁺

### (S,E)-Ethyl 6-(3,5-bis(trifluoromethyl)benzamido)-7-(1-(2-(isopentylamino)-2-oxoethyl)-2-oxo-1,2-dihydropyridin-3-ylamino)-7-oxohept-2-enoate (A17)

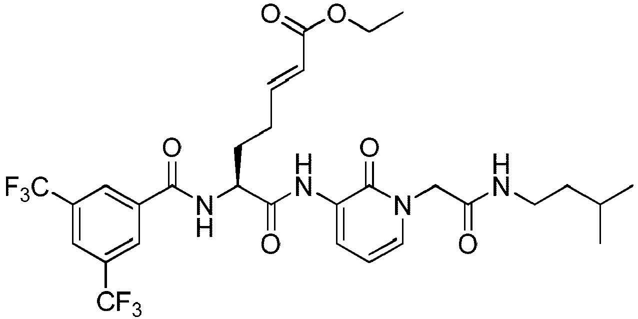

Summenformel: C30H34F6N4O6
Molekulargewicht: 660,60
ESI-MS: 661,3 [M+H]⁺

### (S,E)-Ethyl 7-(1-(2-(isopentylamino)-2-oxoethyl)-2-oxo-1,2-dihydropyridin-3-ylamino)-7-oxo-6-(4-(piperidin-1-yl)benzamido)hept-2-enoate (A18)

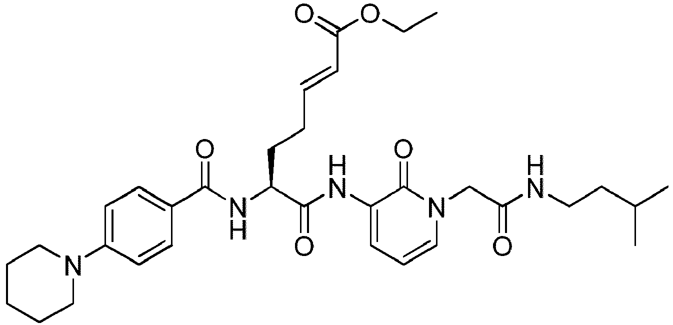

Summenformel: C33H45N5O6
Molekulargewicht: 607,74
ESI-MS: 608,3 [M+H]⁺

### (S,E)-Ethyl 7-(1-(2-(isopentylamino)-2-oxoethyl)-2-oxo-1,2-dihydropyridin-3-ylamino)-6-(1-methyl-1H-imidazole-5-carboxamido)-7-oxohept-2-enoate (A19)

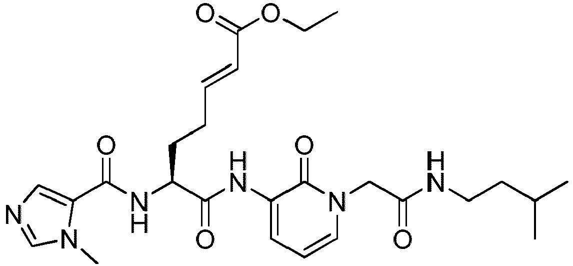

Summenformel: C26H36N6O6
Molekulargewicht: 528,60
ESI-MS: 529,3 [M+H]⁺

### (S,E)-Ethyl 7-(1-(2-(isopentylamino)-2-oxoethyl)-2-oxo-1,2-dihydropyridin-3-ylamino)-6-(4-(4-methylpiperazin-1-yl)benzamido)-7-oxohept-2-enoate (A20)

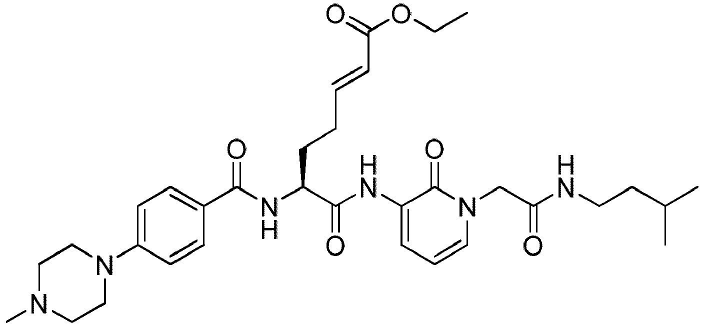

Summenformel: C33H46N6O6
Molekulargewicht: 622,75
ESI-MS: 623,4 [M+H]⁺

### (S,E)-Ethyl 7-(1-(2-(isopentylamino)-2-oxoethy))-2-oxo-1,2-dihydropyridin-3-ylamino)-6-(4-methyl-3,4-dihydro-2H-benzo[b][1,4]oxazine-7-carboxamido)-7-oxohept-2-enoate (A21)

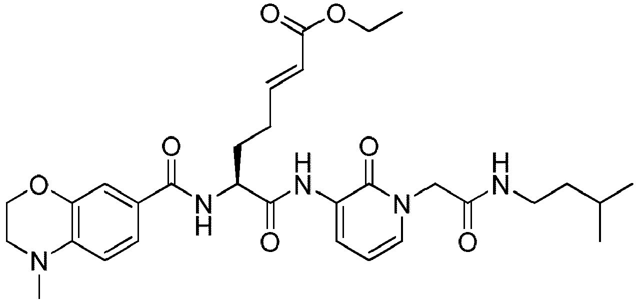

Summenformel: C31H41 N507
Molekulargewicht: 595,69
ESI-MS: 596,3 [M+H]⁺

### (S,E)-Ethyl 7-(1-(2-(isopentylamino)-2-oxoethyl)-2-oxo-1,2-dihydropyridin-3-ylamino)-7-oxo-6-(phenylsulfonamido)hept-2-enoate (A22)

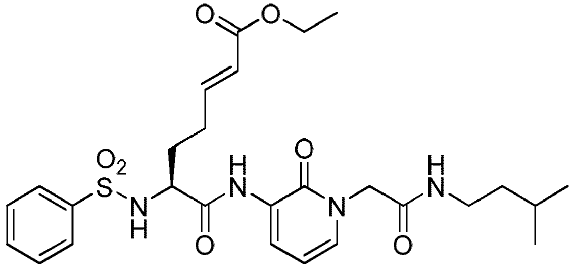

Summenformel: C27H36N4O7S
Molekulargewicht: 560,66
ESI-MS: 561,3 [M+H]⁺

### (S,E)-5-(N-(7-Ethoxy-1-(1-(2-(isopentylamino)-2-oxoethyl)-2-oxo-1,2-dihydropyridin-3-ylamino)-1,7-dioxohept-5-en-2-yl)sulfamoyl)-2-hydroxybenzoic acid (A23)

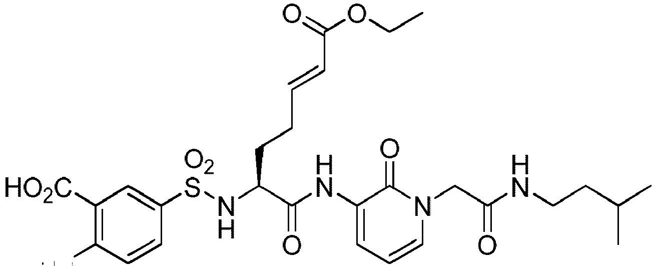

Summenformel: C28H36N4O10S
Molekulargewicht: 620,67
ESI-MS: 621,2 [M+H]⁺

### (S,E)-4-(7-Ethoxy-1-(1-(2-(isopentylamino)-2-oxoethyl)-2-oxo-1,2-dihydropyridin-3-ylamino)-1,7-dioxohept-5-en-2-ylamino)-4-oxobutanoic acid (A24)

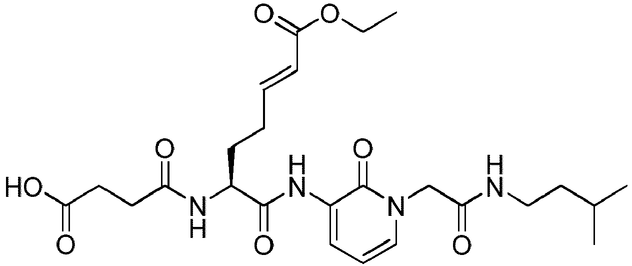

Summenformel: C25H36N4O8
Molekulargewicht: 520,58
ESI-MS: 521,2 [M+H]⁺

### (S,E)-Ethyl 6-acetamido-7-(1-(2-(2-(diethylamino)ethylamino)-2-oxoethyl)-2-oxo-1,2-dihydropyridin-3-ylamino)-7-oxohept-2-enoate (A25)

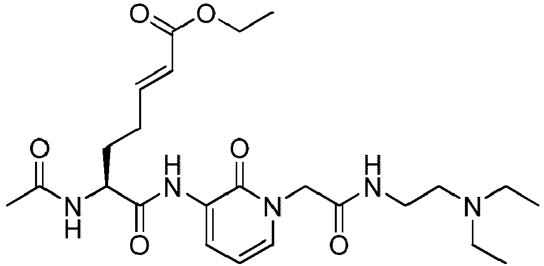

Summenformel: C24H37N5O6
Molekulargewicht: 491,58
ESI-MS: 492,3 [M+H]⁺

### (S,E)-4-(1-(1-(2-(2-(Diethylamino)ethylamino)-2-oxoethyl)-2-oxo-1,2-dihydropyridin-3-ylamino)-7-ethoxy-1,7-dioxohept-5-en-2-ylamino)-4-oxobutanoic acid (A26)

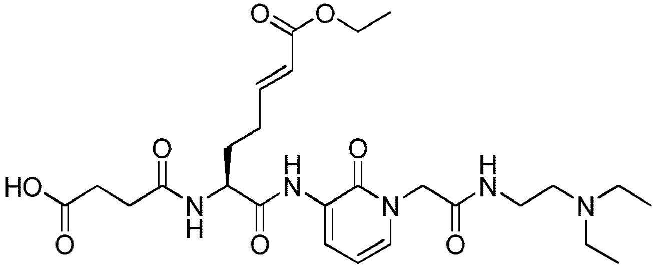

Summenformel: C26H39N5O8
Molekulargewicht: 549,62
ESI-MS: 550,3 [M+H]⁺

### (S,E)-Ethy) 6-acetamido-7-(1-(2-(2-(dimethylamino)ethylamino)-2-oxoethyl)-2-oxo-1,2-dihydropyridin-3-ylamino)-7-oxohept-2-enoate (A27)

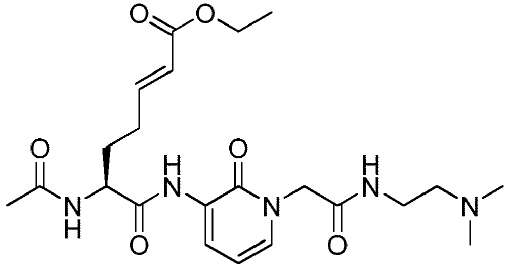

Summenformel: C22H33N5O6
Molekulargewicht: 463,53
ESI-MS: 464,2 [M+H]⁺

### (S,E)-Methyl 6-acetamido-7-(1-(2-(isopentylamino)-2-oxoethyl)-2-oxo-1,2-dihydro-pyridin-3-ylamino)-7-oxohept-2-enoate (A28)

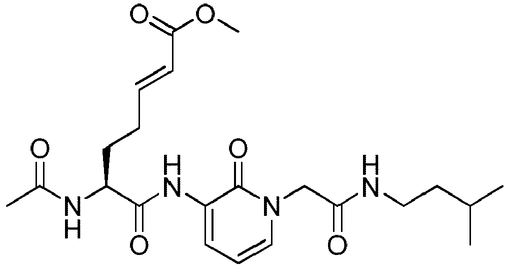

Summenformel: C22H32N4O6
Molekulargewicht: 448,51
ESI-MS: 449,2 [M+H]⁺

### (S,E)-4-(7-Ethoxy-1-(1-(2-(2-ethylbutylamino)-2-oxoethyl)-2-oxo-1,2-dihydropyridin-3-ylamino)-1,7-dioxohept-5-en-2-ylamino)-4-oxobutanoic acid (A30)

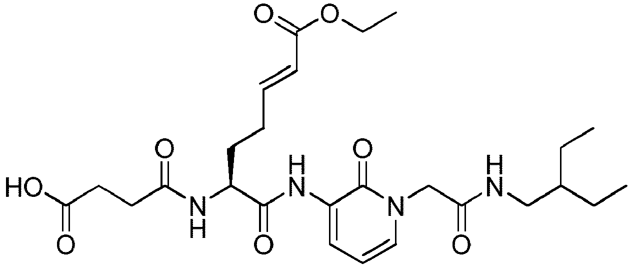

Summenformel: C26H38N4O8
Molekulargewicht: 534,60
ESI-MS: 535,3 [M+H]⁺

### (S,E)-Ethyl 6-acetam ido-7-(1-(2-((S)-1-methoxy-4-methyl-1-oxopentan-2-ylamino)-2-oxoethyl)-2-oxo-1,2-dihydropyridin-3-ylamino)-7-oxohept-2-enoate (A31)

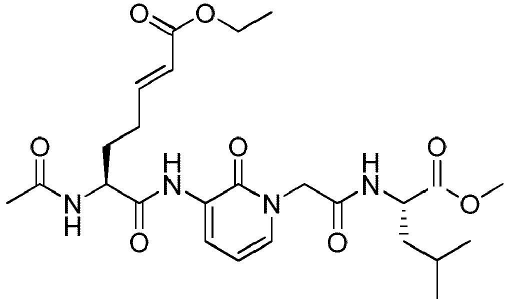

Summenformel: C25H36N4O8
Molekulargewicht: 520,58
ESI-MS: 521,3 [M+H]⁺

### 4-((S,E)-7-Ethoxy-1-(1-(2-((S)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A32)

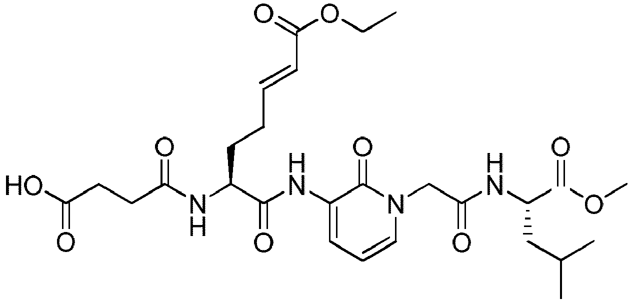

Summenformel: C27H38N4O10
Molekulargewicht: 578,61
ESI-MS: 579,3 [M+H]⁺

### (S,E)-Ethyl 6-acetamido-7-(1-(2-((R)-1-methoxy-4-methyl-1-oxopentan-2-ylamino)-2-oxoethyl)-2-oxo-1,2-dihydropyridin-3-ylamino)-7-oxohept-2-enoate (A33)

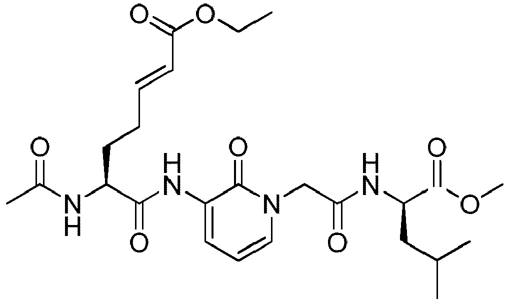

Summenformel: C25H36N4O8
Molekulargewicht: 520,58
ESI-MS: 521,3 [M+H]⁺

### 4-((S,E)-7-Ethoxy-1-(1-(2-((R)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A34)

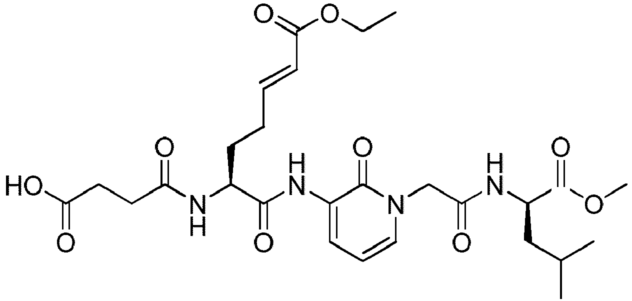

Summenformel: C27H38N4O10
Molekulargewicht: 578,61
ESI-MS: 579,3 [M+H]⁺

### (S,E)-ethyl 6-acetamido-7-(1-(2-((2S,3R)-1-methoxy-3-methyl-1-oxopentan-2-ylamino)-2-oxoethyl)-2-oxo-1,2-dihydropyridin-3-ylamino)-7-oxohept-2-enoate (A35)

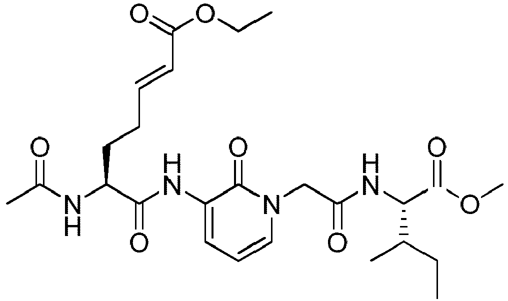

Summenformel: C25H36N4O8
Molekulargewicht: 520,58
ESI-MS: 521,3 [M+H]⁺

### 4-((S,E)-7-Ethoxy-1-(1-(2-((2S,3R)-1-methoxy-3-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A36)

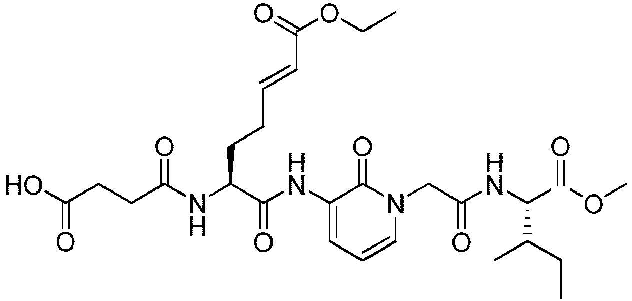

Summenformel: C27H38N4O10
Molekulargewicht: 578,61
ESI-MS: 579,3 [M+H]⁺

### (S,E)-4-(7-Ethoxy-1,7-dioxo-1-(2-oxo-1-(2-oxo-2-(4-(trifluoromethyl)benzylamino)ethyl)-1,2-dihydropyridin-3-ylamino)hept-5-en-2-ylamino)-4-oxobutanoic acid (A37)

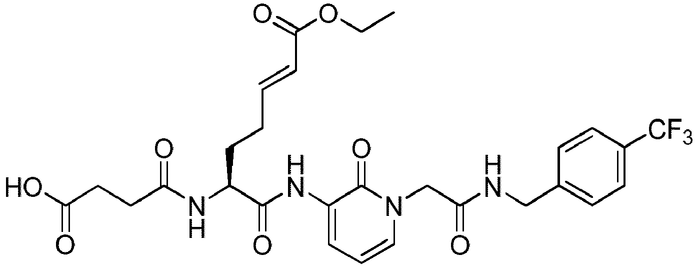

Summenformel: C28H31F3N4O8
Molekulargewicht: 608,56
ESI-MS: 609,2 [M+H]⁺

### (S,E)-4-(1-(1-(2-(3,3-Dimethylbutylamino)-2-oxoethyl)-2-oxo-1,2-dihydropyridin-3-ylamino)-7-ethoxy-1,7-dioxohept-5-en-2-ylamino)-4-oxobutanoic acid (A38)

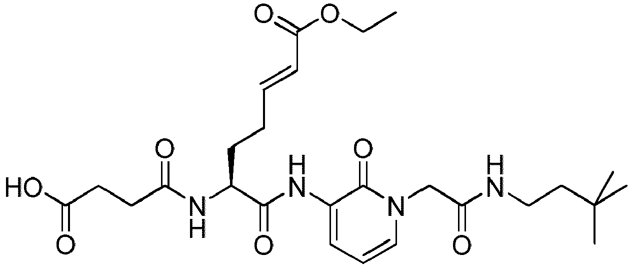

Summenformel: C26H38N4O8
Molekulargewicht: 534,60
ESI-MS: 535,3 [M+H]⁺

### (S,E)-Ethyl 6-acetamido-7-(1-(2-(3,3-dimethylbutylamino)-2-oxoethyl)-2-oxo-1,2-dihydropyridin-3-ylamino)-7-oxohept-2-enoate (A39)

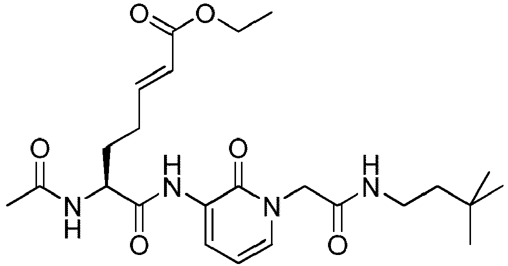

Summenformel: C24H36N4O6
Molekulargewicht: 476,57
ESI-MS: 477,3 [M+H]⁺

### (S,E)-Ethyl 6-acetamido-7-(1-(2-((S)-1-methoxy-4,4-dimethyl-1-oxopentan-2-ylamino)-2-oxoethyl)-2-oxo-1,2-dihydropyridin-3-ylamino)-7-oxohept-2-enoate (A40)

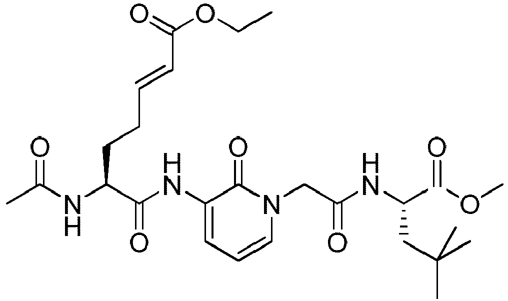

Summenformel: C26H38N4O8
Molekulargewicht: 534,60
ESI-MS: 535,3 [M+H]⁺

### 4-((S,E)-7-Ethoxy-1-(1-(2-((S)-1-methoxy-4,4-dimethyl-1-oxopentan-2-ylamino)-2-oxoethyl)-2-oxo-1,2-dihydropyridin-3-ylamino)-1,7-dioxohept-5-en-2-ylamino)-4-oxobutanoic acid (A41)

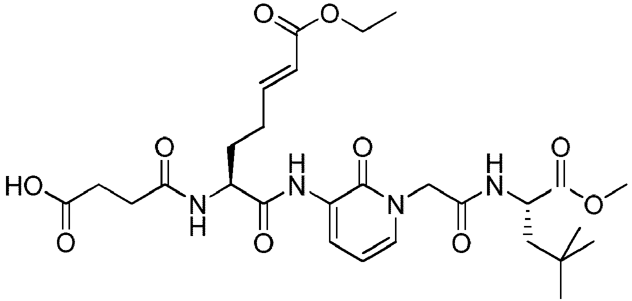

Summenformel: C28H40N4O10
Molekulargewicht: 592,64
ESI-MS: 593,3 [M+H]⁺

### (S,E)-ethyl 6-acetamido-7-(1-(2-(3-ethylpentylamino)-2-oxoethyl)-2-oxo-1,2-dihydropyridin-3-ylamino)-7-oxohept-2-enoate (A42)

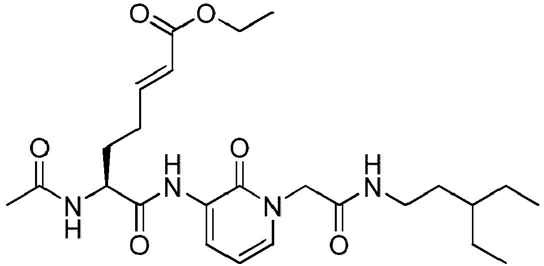

Summenformel: C25H38N4O6
Molekulargewicht: 490,59
ESI-MS: 491,3 [M+H]⁺

### (S,E)-4-(7-Ethoxy-1-(1-(2-(3-ethylpentylamino)-2-oxoethyl)-2-oxo-1,2-dihydropyridin-3-ylamino)-1,7-dioxohept-5-en-2-ylamino)-4-oxobutanoic acid (A43)

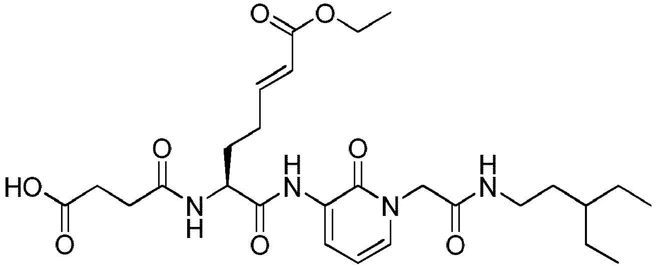

Summenformel: C27H40N4O8
Molekulargewicht: 548,63
ESI-MS: 549,3 [M+H]⁺

### (S,E)-4-(7-Ethoxy-1,7-dioxo-1-(2-oxo-1-(2-oxo-2-(2-(trifluoromethyl)benzylamino)ethyl)-1,2-dihydropyridin-3-ylamino)hept-5-en-2-ylamino)-4-oxobutanoic acid (A44)

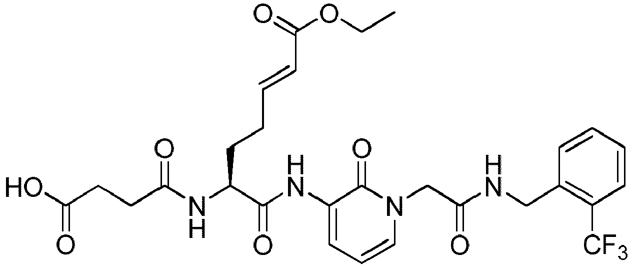

Summenformel: C28H31F3N4O8
Molekulargewicht: 608,56
ESI-MS: 609,2 [M+H]⁺

### (S,E)-4-(7-Ethoxy-1,7-dioxo-1-(2-oxo-1-(2-oxo-2-(4-(trifluoromethyl)piperidin-1-yl)ethyl)-1,2-dihydropyridin-3-ylamino)hept-5-en-2-ylamino)-4-oxobutanoic acid (A45)

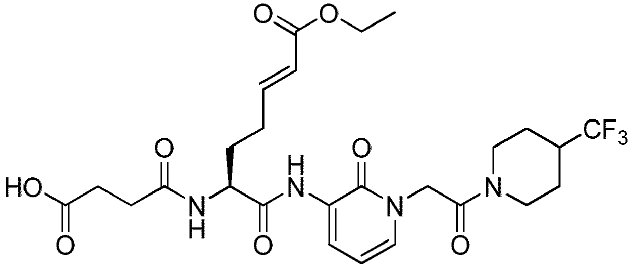

Summenformel: C26H33F3N4O8
Molekulargewicht: 586,56
ESI-MS: 587,2 [M+H]⁺

### (S,E)-Ethyl 6-acetamido-7-oxo-7-(2-oxo-1-(2-oxo-2-(2-(pyrrolidin-1-yl)ethylamino)ethyl)-1,2-dihydropyridin-3-ylamino)hept-2-enoate (A46)

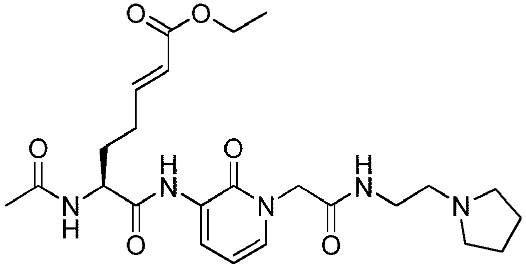

Summenformel: C24H35N5O6
Molekulargewicht: 489,56
ESI-MS: 490,3 [M+H]⁺

### (S,E)-4-(7-Ethoxy-1,7-dioxo-1-(2-oxo-1-(2-oxo-2-(2-(thiophen-2-yl)ethylamino)ethyl)-1,2-dihydropyridin-3-ylamino)hept-5-en-2-ylamino)-4-oxobutanoic acid (A47)

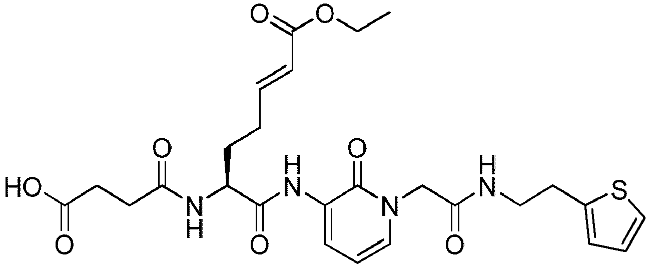

Summenformel: C26H32N4O8S
Molekulargewicht: 560,62
ESI-MS: 561,2 [M+H]⁺

### (S,E)-Ethyl 6-acetamido-7-(1-(2-((1-ethylpiperidin-4-yl)methylamino)-2-oxoethyl)-2-oxo-1,2-dihydropyridin-3-ylamino)-7-oxohept-2-enoate (A48)

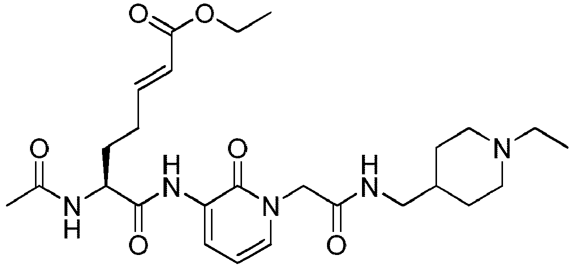

Summenformel: C26H39N5O6
Molekulargewicht: 517,62
ESI-MS: 518,3 [M+H]⁺

### (S,E)-4-(7-Ethoxy-1,7-dioxo-1-(2-oxo-1-(2-oxo-2-(2-(2-oxoimidazolidin-1-yl)ethylamino)ethyl)-1,2-dihydropyridin-3-ylamino)hept-5-en-2-ylamino)-4-oxobutanoic acid (A49)

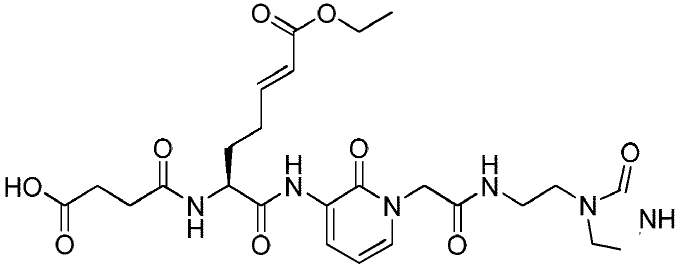

Summenformel: C25H34N6O9
Molekulargewicht: 562,57
ESI-MS: 563,2 [M+H]⁺

### (6S,E)-Ethyl 6-acetamido-7-(1-(2-(2-methylbutylamino)-2-oxoethyl)-2-oxo-1,2-dihydropyridin-3-ylamino)-7-oxohept-2-enoate (A50)

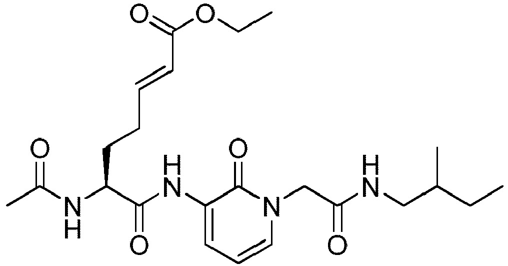

Summenformel: C23H34N4O6
Molekulargewicht: 462,54
ESI-MS: 463,3 [M+H]⁺

### 4-((2S,E)-7-Ethoxy-1-(1-(2-(2-methylbutylamino)-2-oxoethyl)-2-oxo-1,2-dihydropyridin-3-ylamino)-1,7-dioxohept-5-en-2-ylamino)-4-oxobutanoic acid (A51)

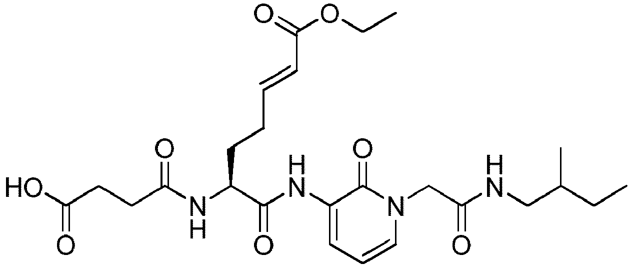

Summenformel: C25H36N4O8
Molekulargewicht: 520,58
ESI-MS: 521,3 [M+H]⁺

### (6S,E)-Ethyl 6-acetamido-7-(1-(2-(3-methylpiperidin-1-yl)-2-oxoethyl)-2-oxo-1,2-dihydropyridin-3-ylamino)-7-oxohept-2-enoate (A52)

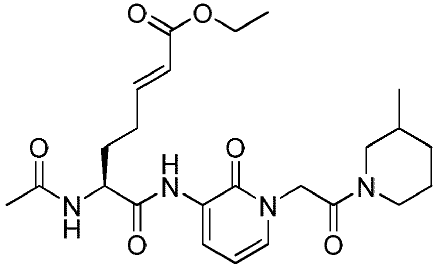

Summenformel: C24H34N4O6
Molekulargewicht: 474,55
ESI-MS: 475,3 [M+H]⁺

### (2S,3R)-2-(2-(3-((S,E)-7-Ethoxy-2-(1-methyl-1H-imidazole-5-carboxamido)-7-oxohept-5-enamido)-2-oxopyridin-1(2H)-yl)acetamido)-3-methylpentanoic acid (A53)

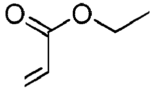

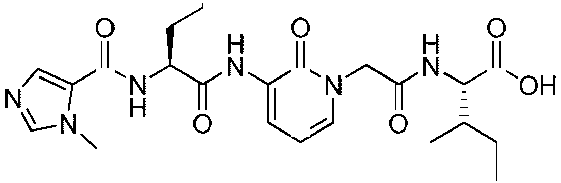

Summenformel: C27H36N6O8
Molekulargewicht: 572,61
ESI-MS: 573,3 [M+H]⁺

### (S,E)-Ethyl 6-acetamido-7-(1-(2-(isobutylamino)-2-oxoethyl)-2-oxo-1,2-dihydropyridin-3-ylamino)-7-oxohept-2-enoate (A54)

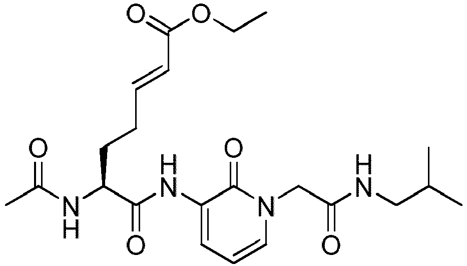

Summenformel: C22H32N4O6
Molekulargewicht: 448,51
ESI-MS: 449,2 [M+H]⁺

### (6S,E)-Ethyl 6-acetamido-7-(1-(2-(3-methylbutan-2-ylamino)-2-oxoethyl)-2-oxo-1,2-dihydropyridin-3-ylamino)-7-oxohept-2-enoate (A55)

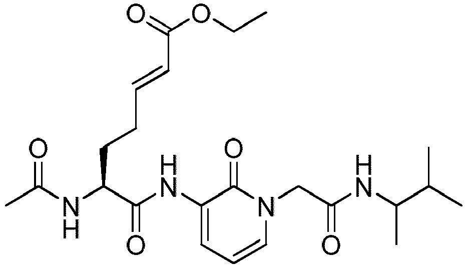

Summenformel: C23H34N4O6
Molekulargewicht: 462,54
ESI-MS: 463,3 [M+H]⁺

### (S,E)-Ethyl 7-(1-(2-(isopentylamino)-2-oxoethyl)-2-oxo-1,2-dihydropyridin-3-ylamino)-7-oxo-6-(3-ureidopropanamido)hept-2-enoate (A56)

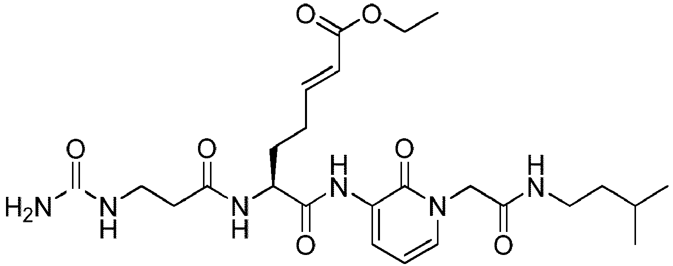

Summenformel: C25H38N6O7
Molekulargewicht: 534,61
ESI-MS: 535,3 [M+H]⁺

### (S,E)-Ethyl 6-acetamido-7-(1-(2-((S)-1-methoxy-3-methyl-1-oxobutan-2-ylamino)-2-oxoethyl)-2-oxo-1,2-dihydropyridin-3-ylamino)-7-oxohept-2-enoate (A57)

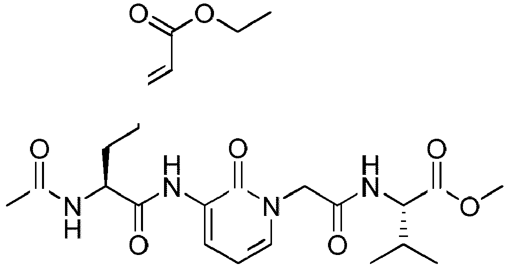

Summenformel: C24H34N4O8
Molekulargewicht: 506,55
ESI-MS: 507,2 [M+H]⁺

### (25,3R)-2-(2-(3-((S,E)-2-Benzamido-7-ethoxy-7-oxohept-5-enamido)-2-oxopyridin-1(2H)-yl)acetamido)-3-methylpentanoic acid (A59)

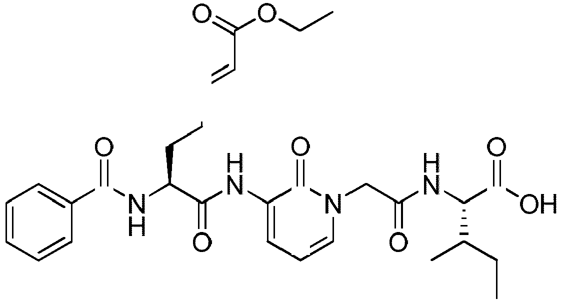

Summenformel: C29H36N4O8
Molekulargewicht: 568,62
ESI-MS: 569,3 [M+H]⁺

### (2S,3R)-2-(2-(3-((S,E)-7-Methoxy-2-(1-methyl-1H-imidazole-5-carboxamido)-7-oxohept-5-enamido)-2-oxopyridin-1(2H)-yl)acetamido)-3-methylpentanoic acid (A60)

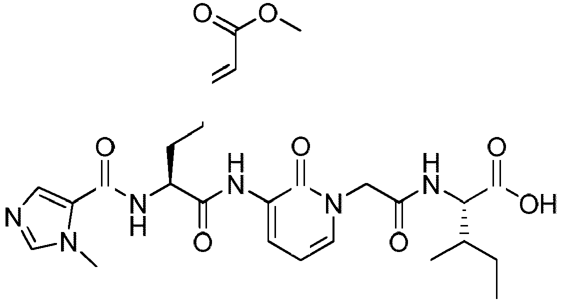

Summenformel: C26H34N6O8
Molekulargewicht: 558,58
ESI-MS: 559,3 [M+H]⁺

### (S,E)-Ethyl 6-acetamido-7-(1-(2-(2-ethylbutylamino)-2-oxoethyl)-2-oxo-1,2-dihydropyridin-3-ylamino)-7-oxohept-2-enoate (A62)

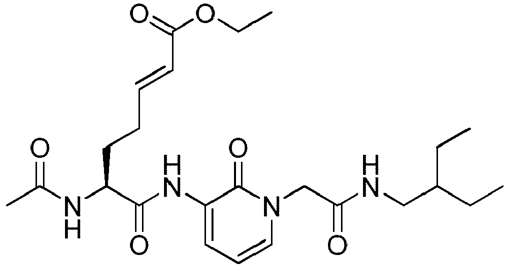

Summenformel: C24H36N4O6
Molekulargewicht: 476,57
ESI-MS: 477,3 [M+H]⁺

### (6S,E)-Methyl 6-acetamido-7-(1-(2-(3-methylpiperidin-1-yl)-2-oxoethyl)-2-oxo-1,2-dihydropyridin-3-ylamino)-7-oxohept-2-enoate (A64)

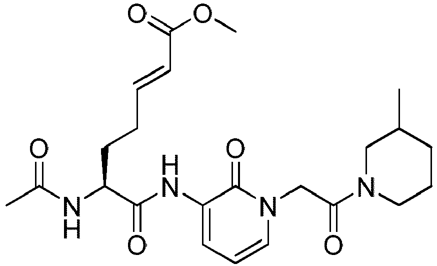

Summenformel: C23H32N4O6
Molekulargewicht: 460,52
ESI-MS: 560,3 [M+H]⁺

### (S,E)-Methyl 6-acetamido-7-oxo-7-(2-oxo-1-(2-oxo-2-(4-(trifluoromethyl)piperidin-1-yl)ethyl)-1,2-dihydropyridin-3-ylamino)hept-2-enoate (A65)

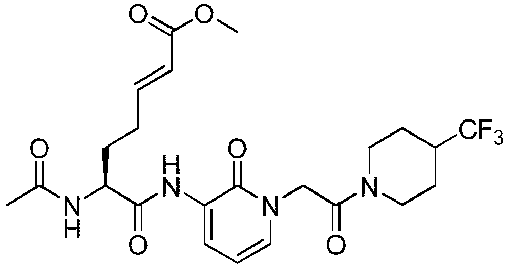

Summenformel: C23H29F3N4O6
Molekulargewicht: 514,49
ESI-MS: 515,2 [M+H]⁺

### (6S,E)-Methyl 6-acetamido-7-oxo-7-(2-oxo-1-(2-oxo-2-(3-(trifluoromethyl)piperidin-1-yl)ethyl)-1,2-dihydropyridin-3-ylamino)hept-2-enoate (A66)

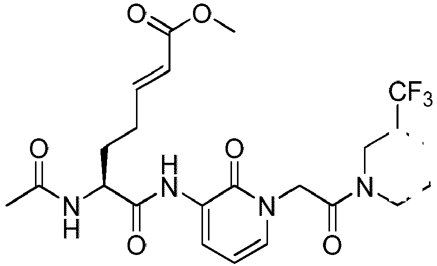

Summenformel: C23H29F3N4O6
Molekulargewicht: 514,49
ESI-MS: 515,2 [M+H]⁺

### (S,E)-Methyl 7-(1-(2-(2-ethylbutylamino)-2-oxoethyl)-2-oxo-1,2-dihydropyridin-3-ylamino)-6-(nicotinamido)-7-oxohept-2-enoate (A67)

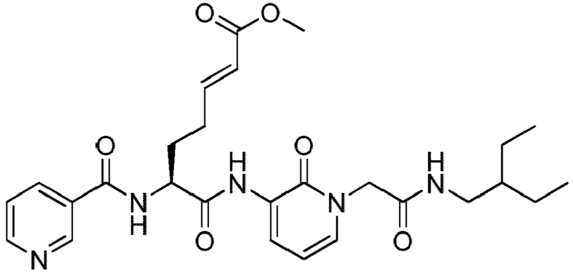

Summenformel: C27H35N5O6
Molekulargewicht: 525,60
ESI-MS: 526,3 [M+H]⁺

**(6S,E)-Methyl 6-(2-aminopropanamido)-7-(1-(2-(2-ethylbutylamino)-2-oxoethyl)-2-oxo-1,2-dihydropyridin-3-ylamino)-7-oxohept-2-enoate 2,2,2-trifluoroacetate (A68)** 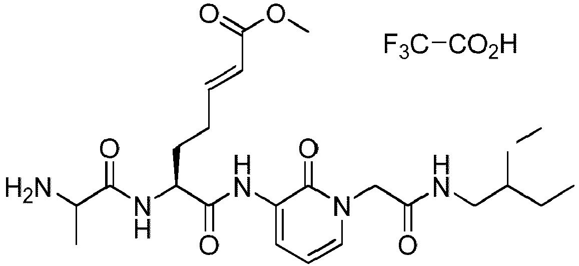 Summenformel: C26H38F3N5O8
Molekulargewicht: 605,60
ESI-MS: 492,3 [M+H]⁺

### (S,E)-Methyl 6-(2-aminoacetamido)-7-(3-((2-(2-ethylbutylamino)-2-oxoethyl)(methyl)amino)-3-oxoprop-1-en-2-ylamino)-7-oxohept-2-enoate 2,2,2-trifluoroacetate (A69)

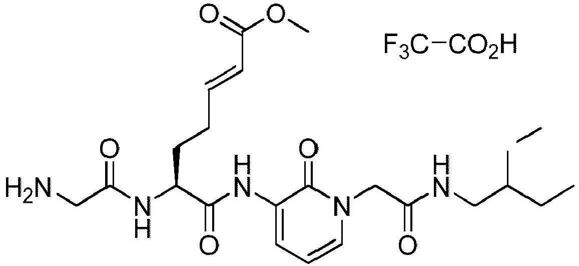

Summenformel: C25H36F3N5O8
Molekulargewicht: 591,58
ESI-MS: 478,3 [M+H]⁺

### (S,E)-Methyl 6-(2-aminobenzamido)-7-(1-(2-(2-ethylbutylamino)-2-oxoethyl)-2-oxo-1,2-dihydropyridin-3-ylamino)-7-oxohept-2-enoate 2,2,2-trifluoroacetate (A70)

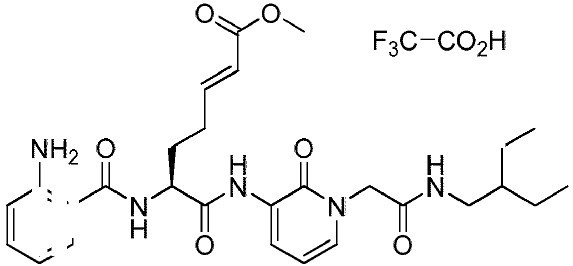

Summenformel: C30H38F3N5O8
Molekulargewicht: 653,65
ESI-MS: 540,3 [M+H]⁺

### (S,E)-Methyl 6-(3',6'-bis(dimethylamino)-3-oxo-3H-spiro[isobenzofuran-1,9'-xanthene]-5-ylcarboxamido)-7-(1-(2-(2-ethylbutylamino)-2-oxoethyl)-2-oxo-1,2-dihydropyridin-3-ylamino)-7-oxohept-2-enoate (A71)

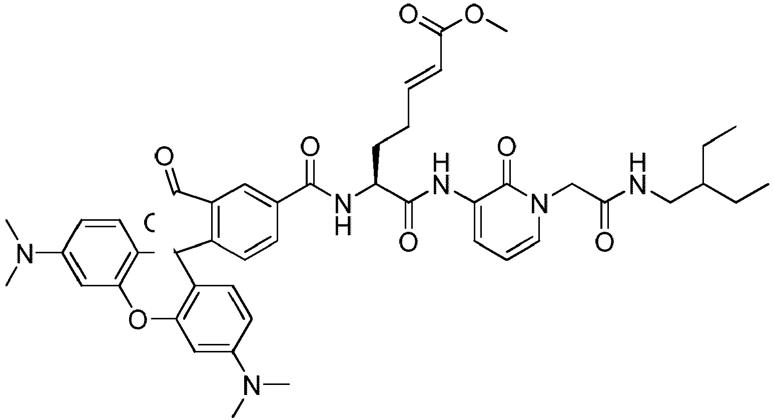

Summenformel: C46H52N6O9
Molekulargewicht: 832,94
ESI-MS: 833,4 [M+H]⁺

### (S,E)-Ethyl 6-acetamido-7-(1-(2-(isopentylamino)-2-oxoethyl)-2-oxo-1,2-dihydropyridin-3-ylamino)-7-oxohept-2-enoate (A72)

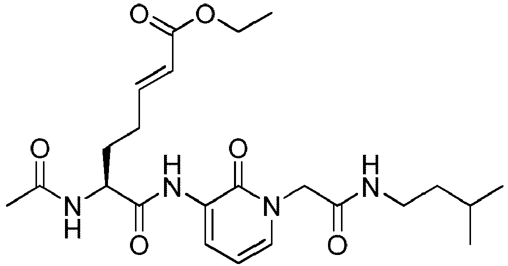

Summenformel: C23H34N4O6
Molekulargewicht: 462,54
ESI-MS: 463,3 [M+H]⁺

### (S,E)-Methyl 6-acetamido-7-(1-(2-(isopentylamino)-2-oxoethyl)-2-oxo-1,2-dihydropyridin-3-ylamino)-7-oxohept-2-enoate (A73)

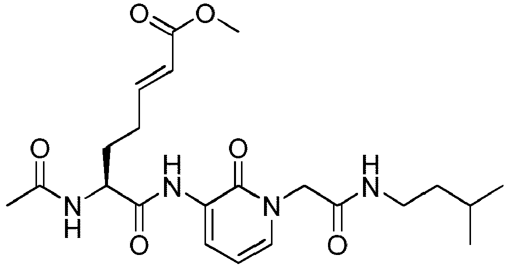

Summenformel: C22H32N4O6
Molekulargewicht: 448,51
ESI-MS: 449,2 [M+H]⁺

### (S,E)-Ethyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (A74)

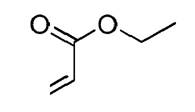

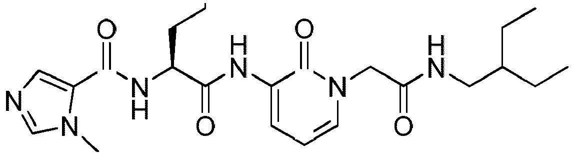

Summenformel: C27H38N6O6
Molekulargewicht: 542,63
ESI-MS: 543,3 [M+H]⁺

### (25,3R)-2-(2-(3-((S,E)-2-Benzamido-7-methoxy-7-oxohept-5-enamido)-2-oxopyridin-1(2H)-yl)acetamido)-3-methylpentanoic acid (A75)

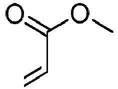

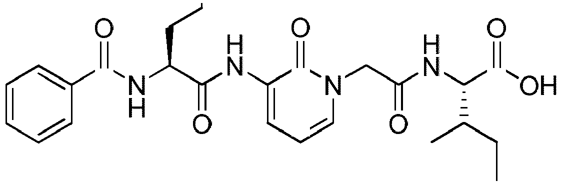

Summenformel: C28H34N4O8
Molekulargewicht: 554,59
ESI-MS: 555,2 [M+H]⁺

### (R,E)-Methyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (A76)

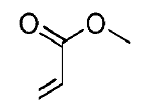

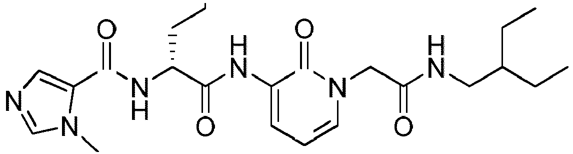

Summenformel: C26H36N6O6
Molekulargewicht: 528,60
ESI-MS: 529,3 [M+H]⁺

### (S,E)-Methyl 6-acetamido-7-(1-(2-(2-ethylbutylamino)-2-oxoethyl)-2-oxo-1,2-dihydropyridin-3-ylamino)-7-oxohept-2-enoate (A79)

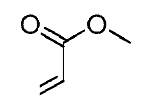

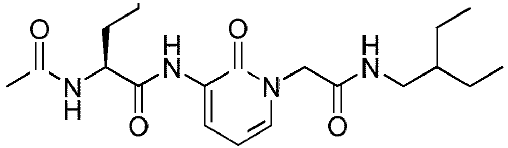

Summenformel: C23H34N4O6
Molekulargewicht: 462,54
ESI-MS: 463,3 [M+H]⁺

### 9. Inhibitorische Wirkung der erfindungsgemäßen Verbindungen

### Generelle Methode zur Inaktivierung von humaner Gewebetransglutaminase

250 µg lyophilisierte His-getagte rekombinante humane Gewebetransglutaminase (His₆-rh-TG2, Zedira Produkt T002) werden durch Zusatz von 150 µl Wasser rekonstituiert. (resultierender Puffer 50 mM NaH₂PO₄, 150 mM NaCl, pH = 8,0).

Eine 10 mM Inhibitor-Stammlösung in DMSO wird hergestellt und jeweils mit Puffer (50mM Tris-HCl, 10 mM CaCl₂, 5 mM DTT, pH = 7,5) auf das zwanzigfache der im Inhibitionsansatz gewünschten Konzentration verdünnt (mind. jedoch 1/50 Verdünnung resultierend 2% DMSO-Konzentration.).

900 µl einer Assay-Lösung zusammengesetzt aus 55,56 mM Tris, 11,11 mM CaCl₂, 0,11 % PEG₈₀₀₀, 5,56 mM DTT, 5,56 mM Glycinmethylester und 50 µM Abz-APE(CAD-DNP)QEA-OH, (Zedira Produkt A102; Patentnummer: EP 1781807B1), pH = 7,5 werden in einer Küvette vorgelegt und in der Messzelle eines Spektrophotometers auf 37°C temperiert. Zu dieser Lösung werden 50 µl der jeweiligen Inhibitorlösung zugesetzt (resultierend eine Konzentration von weniger als 0,2% DMSO im Ansatz). 7 µl der oben rekonstituierten Transglutaminase-Lösung werden mit 51 µl Puffer (50 mM Tris, 100 mM NaCl, 5 mM DTT, pH = 7,5 verdünnt. 50µl dieser Enzymlösung (10µg His₆-rhTG2) werden zu der Assay-Lösung, enthaltend die jeweilige Inhibitorkonzentration, zugesetzt. Es wird 5 min bei 37 °C inkubiert bevor die Messung gestartet wird. (λ_{exc} = 313 nm und λₑₘ = 418 nm, t = 15 bis 30 min).

Die vergleichende Bestimmung verschiedener Transglutaminasen zur Ermittlung der Selektivität erfolgt mit Casein und Dansylcadaverin als Substraten. Hierzu wird der Kit T036 (Zedira, Darmstadt; Lit.: Lorand et al., Anal. Biochem. 1971, 44:221-31) sowie die Transglutaminaseprodukte T009 (Transglutaminase 1), T012 (Transglutaminase 3), T021 (Transglutaminase 6) und T027 (plasma Transglutaminase; Factor XIII) verwendet. Deshalb sind jeweils zwei unterschiedliche IC₅₀-Werte für die Verbindungen angegeben.

Die Auswertung der resultierenden Enzymaktivität erfolgt über die Steigung der durch Zunahme der Fluoreszenz erhaltenen Geraden.

Um die nicht inhibierte Enzymaktivität zu bestimmen, wird DMSO statt Inhibitor-Stammlösung verwendet. IC₅₀-Werte werden bestimmt, indem die resultierende Enzymaktivität gegen den Logarithmus der Inhibitorkonzentration aufgetragen wird. IC₅₀ ist definiert als die Inhibitorkonzentration bei der 50% Enzymaktivität resultiert.

Die inhibitorische Aktivität der erfindungsgemäßen Verbindungen hinsichtlich der Gewebetransglutaminase (TG2) wird mittels der IC₅₀-Wert in nachfolgender Tabelle wiedergegeben.

| Verbindung | IC₅₀ TG2 | Verbindung | IC₅₀ TG2 |
|---|---|---|---|
| A1 | 100 nM | A40 | 131 nM |
| A2 | 100 nM | A41 | 166 nM |
| A3 | 96 nM | A42 | 147 nM |
| A4 | 119 nM | A43 | 145 nM |
| A5 | 130 nM | A44 | 240 nM |
| A6 | 532 nM | A45 | 1,23 µM |
| A7 | 126 nM | A46 | 3,3 µM |
| A8 | 251 nM | A47 | 480 nM |
| A9 | 708 nM | A48 | 1,7 µM |
| A10 | 73 nM | A49 | 1,8 µM |
| A11 | 104 nM | A50 | 119 nM |
| A12 | 84 nM | A51 | 166 nM |
| A13 | 83 nM | A52 | 0,7 µM |
| A14 | 137 nM | A53 | 60 nM |
| A15 | 92 nM | A54 | 191 nM |
| A16 | 104 nM | A55 | 153 nM |
| A17 | 63 nM | A56 | 139 nM |
| A18 | 175 nM | A57 | 84 nM |
| A19 | 80 nM | A58 | 76 nM |
| A20 | 202 nM | A59 | 43 nM |
| A21 | 111 nM | A60 | 54 nM |
| A22 | 133 nM | A61 | 25 nM |
| A23 | 475 nM | A62 | 93 nM |
| A24 | 173 nM | A63 | 45 nM |
| A25 | 4,8 µM | A64 | 141 nM |
| A26 | 5,4 µM | A65 | 132 nM |
| A27 | 5,2 µM | A66 | 310 nM |
| A28 | 32 nM | A67 | 55 nM |
| A29 | 32 nM | A68 | 53 nM |
| A30 | 116 nM | A69 | 71 nM |
| A31 | 124 nM | A70 | 81 nM |
| A32 | 194 nM | A71 | 97 nM |
| A33 | 493 nM | A72 | 1,15 µM |
| A34 | 640 nM | A73 | 157 nM |
| A35 | 55 nM | A74 | 554 nM |
| A36 | 88 nM | A75 | 136 nM |
| A37 | 340 nM | A76 | 54,7 µM |
| A38 | 175 nM | A77 | 469 nM |
| A39 | 115 nM | A78 | 627 nM |
| | | A79 | 34 nM |

Die Selektivität der inhibitorischen Aktivität ausgewählter erfindungsgemäßer Verbindungen hinsichtlich der Gewebetransglutaminase (TG2) wird anhand der IC₅₀-Werte gegenüber den Transglutaminasen TG1, TG6, TG3 und FXIII in nachfolgender Tabelle wiedergegeben. Zu beachten ist, dass hier gegen Casein als Substrat gemessen wurde, weshalb andere apparente Hemmwerte (IC₅₀-Wert) resultieren.

| **A7** | IC₅₀ | **A19** | IC₅₀ | **A24** | IC₅₀ |
|---|---|---|---|---|---|
| TG2 | 3,6 µM | TG2 | 2,39 µM | TG2 | 7,5 µM |
| TG1 | 28,8 µM | TG1 | 96,0 µM | TG1 | 91,9 µM |
| TG6 | 51,0 µM | TG6 | 54,3 µM | TG6 | 38,5 µM |
| TG3 | 84,1 µM | TG3 | 128 µM | TG3 | 119 µM |
| FXIII | 105 µM | FXIII | 81,1 µM | FXIII | 133 µM |
| | | | | | |

| **A29** | IC₅₀ | **A30** | IC₅₀ | **A35** | IC₅₀ |
|---|---|---|---|---|---|
| TG2 | 0,4 µM | TG2 | 3,26 µM | TG2 | 1,0 µM |
| TG1 | 28,1 µM | TG1 | 113 µM | TG1 | 87,1 µM |
| TG6 | 83,3 µM | TG6 | 32,8 µM | TG6 | 21,3 µM |
| TG3 | 70,9 µM | TG3 | 69,0 µM | TG3 | 95,8 µM |
| FXIII | 67,4 µM | FXIII | 90,9 µM | FXIII | 70,7 µM |
| | | | | | |

| **A42** | IC₅₀ | **A53** | IC₅₀ | **A58** | IC₅₀ |
|---|---|---|---|---|---|
| TG2 | 7,1 µM | TG2 | 833 nM | TG2 | 1,54 µM |
| TG1 | 86,0 µM | TG1 | 68,9 µM | TG1 | 97,5 µM |
| TG6 | 36,7 µM | TG6 | 26,2 µM | TG6 | 66,0 µM |
| TG3 | 104 µM | TG3 | 72,6 µM | TG3 | 109 µM |
| FXIII | 109 µM | FXIII | 77,7 µM | FXIII | 120 µM |
| | | | | | |

| **A59** | IC₅₀ | **A60** | IC₅₀ | **A61** | IC₅₀ |
|---|---|---|---|---|---|
| TG2 | 461 nM | TG2 | 112 nM | TG2 | 79 nM |
| TG1 | 37,1 µM | TG1 | 31,1 µM | TG1 | 15,3 µM |
| TG6 | 9,3 µM | TG6 | 11,5 µM | TG6 | 4,3 µM |
| TG3 | 41,1 µM | TG3 | 42,3 µM | TG3 | 17,6 µM |
| FXIII | 79,6 µM | FXIII | 73,2 µM | FXIII | 63,4 µM |
| | | | | | |

| **A62** | IC₅₀ | **A63** | IC₅₀ | **A64** | IC₅₀ |
|---|---|---|---|---|---|
| TG2 | 1,96 µM | TG2 | 218 nM | TG2 | 3,0 µM |
| TG1 | 133 µM | TG1 | 51,8 µM | TG1 | 122 µM |
| TG6 | 47,0 µM | TG6 | 31,5 µM | TG6 | 109 µM |
| TG3 | 99,7 µM | TG3 | 73,9 µM | TG3 | 125 µM |
| FXIII | 122 µM | FXIII | 89,0 µM | FXIII | 120 µM |

| **A65** | IC₅₀ | **A79** | IC₅₀ | | |
|---|---|---|---|---|---|
| TG2 | 2,9 µM | TG2 | 397 nM | | |
| TG1 | 120 µM | TG1 | 80,7 µM | | |
| TG6 | 37,7 µM | TG6 | 47,8 µM | | |
| TG3 | 100 µM | TG3 | 59,6 µM | | |
| FXIII | 156 µM | FXIII | 78,2 µM | | |

### 10. Nachweis der Gewebetransglutaminase (TG2)-Inhibition

30 BALB/c Mäuse wurden in 4 Gruppen unterteilt: Kontrollgruppe (3 Tiere) und 3 Inhibitorgruppen (jeweils 9 Tiere). Nachdem die Tiere 6 Stunden ohne Nahrung gehalten wurde, wurden je 500 µl Inhibitor-Lösung (A63 (ZED1227), A29 (ZED1098) bzw. A61 (ZED1219)) bzw. Puffer oral via Schlundsonde verabreicht. Die Dosis pro Tier betrug 5 mg/kg Körpergewicht. Nach 30 min wurde den Tieren wieder Zugang zu Nahrung verschafft. Nach drei, 8 bzw. 24 Stunden wurden je 3 Mäuse pro Inhibitor-Gruppe geopfert und der Dünndarm präpariert.

Von den Kontroll-Tieren wurden nach 24 h die Dünndärme präpariert und kryokonserviert.

Mit dem Mikrotom wurden Kryoschnitte der Dünndarmpräparate angefertigt. Die Qualität der Schnitte wurde durch Hämatoxilin/Eosin-Färbung sichergestellt.

Die jeweiligen Methoden sind dem Fachmann bekannt.

Im Anschluss erfolgte das im Folgenden beschriebene Färbeprotokoll: Die Kryoschnitte wurden in Aceton (100%, eiskalt) für 10 min fixiert und im Anschluss mit 1 % BSA in 0,1 M Tris-HCL blockiert. Nach einem Waschschritt (1% BSA in PBS-Puffer) erfolgte die Inkubation mit 4 µg/ml des TG2-Substrats Biotinyl-TVQQEL (Zedira, Produktnummer B001) in Gegenwart von 5 mM CaCl₂ für 2 h bei Raumtemperatur. Die Reaktion wurde mit 25 mM EDTA gestoppt. Nach je einem weiteren Wasch-, Blockier, und Waschschritt wurde der Primärantikörper gegen die humane TG2 zugegeben (Zedira, Produktnummer A018, 25 µg/ml) und eine Stunde bei Raumtemperatur inkubiert. Nach vier Waschschritten wurde Streptavidin-FITC (2 µg/ml; Streptavidinfluoresceinisothiocyanat) und Cy3-gelabelter Ziege-anti-Kaninchen IgG-Antikörper (22,5 µg/ml) zugegeben und 40 min im Dunkeln bei Raumtemperatur inkubiert. TG2 war somit im Fluoreszenzmikroskop als Rotfärbung sichtbar (Cy3), während durch Transglutaminase-Aktivität eingebautes Biotinyl-TVQQEL durch Grünfärbung visualisiert wurde (FITC). Die Überlagerung beider Bilder führte zu einer Gelbfärbung, wenn aktive Transglutaminase vorlag.

Bei den auswertbaren Schnitten der Kontrollmäuse lag keine Inhibierung der TG2 vor, d. h. bis hin zu den Spitzen der Dünndarmzotten konnte aktive TG2 nachgewiesen werden. Im Gegensatz dazu nahm die Aktivität der TG2 in den Schnitten von Mäusen, die Inhibitor verabreicht bekamen, deutlich ab. Insbesondere bei Schnitten von Mäusen, die nach 24 h geopfert wurden, war die TG2-Inhibierung bis über die gesamte Dünndarmschleimhaut nachweisbar.

Die Experimente zeigen, dass die getesteten Inhibitoren in der Lage sind, *in vivo* in der Dünndarmschleimhaut die TG2 zu inhibieren.

### 11. Ex vivo-Testung der Inhibitoren A63 (ZED1227) und A61 (ZED1219)

Schnitte (ca. 5 -7 µM dick) einer Kontrollmaus aus Bsp. 7 wurden nach Fixierung für 30 min mit 0,2 mg/ml; 0,02 mg/ml; 0,002 mg/ml und 0,0 mg/ml Inhibitor (A63 (ZED1227) bzw. A61 (ZED1219)) vorinkubiert. Im Anschluss wurde das in Bsp. 10 beschriebene Färbeprotokoll durchgeführt. In den Kontroll-Schnitten mit 0,0 mg/ml Inhibitor war eine Gelbfärbung im Bereich der Dünndarmschleimhaut zu sehen, die auf aktive TG2 zurückzuführen ist. Nach Zugabe von Inhibitor konnte mit 0,002 µg/ml Inhibitor noch aktive Transglutaminase nachgewiesen werden. Bei den höheren Inhibitor-Konzentrationen war die TG2 vollständig inhibiert.

Die Ergebnisse zeigen, dass sowohl A63 (ZED1227) als auch A61 (ZED1219) in der Lage sind, TG2 in Gewebeschnitten zu inhibieren.

### 12. Bestimmung der Zelltoxizität von Transglutaminase-Inhibitoren

Huh7 (humane Hepatom-Zelllinie) und CaCo2 (humane Coloncarcinom-Zelllinie) wurden auf 96 well-Platten in Dulbecco's Modified Eagle's Medium (DMEM)/10% fötales Kälberserum (FKS) ausgesät und kultiviert. Die Methode ist dem Fachmann bekannt.

Nach einer Stunde wurden die Inhibitoren A63 (ZED1227), A29 (ZED1098) und A61 (ZED1219) in den Konzentrationen 0,1 uM bis 1 mM zugegeben. Die Bestimmung der Proliferation erfolgte mit Hilfe des Cell Proliferation ELISA, BrdU (Roche 11 647 229 001): 24 Stunden nach Inkubation der Zellen mit den Inhibitoren wurde dabei BrdU zugegeben. Die kolorimetrische Entwicklung des Tests erfolgte nach weiteren 18 Stunden laut Herstellerangaben. Die Extinktion wurde bei 450 nm bestimmt.

Zur Bestimmung der metabolischen Aktivität mit dem EZ4U-Assay (Biomedica BI-5000) wurde das Tetrazolium-Substrat 48 Stunden nach Zugabe der Inhibitoren zu den Zellen gegeben. Die Umsetzungskapazität der Zellen wurde über einen Zeitraum von 4 Stunden stündlich bei 450 nm gegen einen Referenzfilter von 630 nm gemessen.

Als Referenz wurde in beiden Versuchen DMEM ohne Inhibitor zugegeben, als Positiv-Kontrolle Cycloheximid (2,5 µg/ml) und Camptothecin (0,2 µg/ml). Währen die beiden Positiv-Kontrollen zu einer signifikanten Reduktion der Proliferation und der metabolischen Aktivität sowohl der CaCo2-als auch der Huh7-Zellen führten, konnte mit den getesteten Inhibitoren A63 (ZED1227), A29 (ZED1098) und A61 (ZED1219) bis zur höchsten gemessenen Konzentration von 1 mM keine Auswirkung auf metabolische Aktivität und Proliferation gemessen werden.

Die Inhibitoren A63 (ZED1227), A29 (ZED1098) und A61 (ZED1219) zeigen demnach keine cytotoxische Aktivität.

## Patentansprüche

1. Verbindungen der folgenden allgemeinen Formel (I): 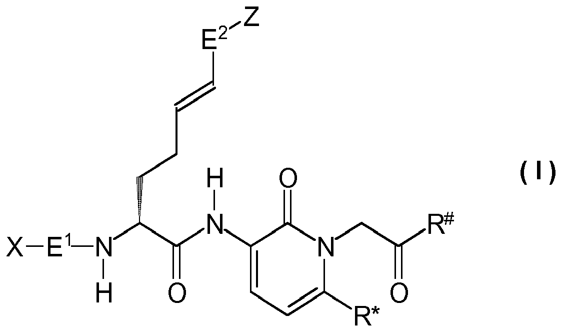 worin
**E¹**, **E²** unabhängig voneinander für -CO- oder -SO₂- stehen,
**R^{#}** einen der folgenden Reste bedeutet -NYY', -NH-CH₂-COOH, -NH-CH(CH₃)-COOH, -NH-CH(CH₂CH₂SCH₃)-COOH, -NH-CH(CH₂OH)-COOH, -NH-CH(CH₂SH)-COOH, -NH-CH(CH₂CONH₂)-COOH, -NH-CH(CH₂CH₂CONH₂)-COOH, 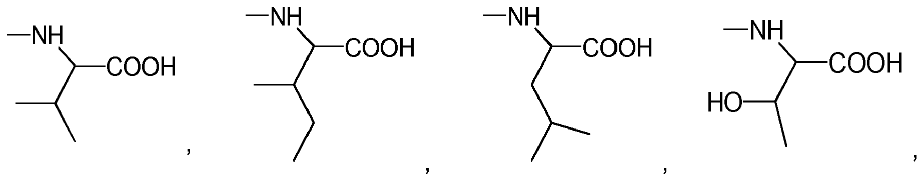 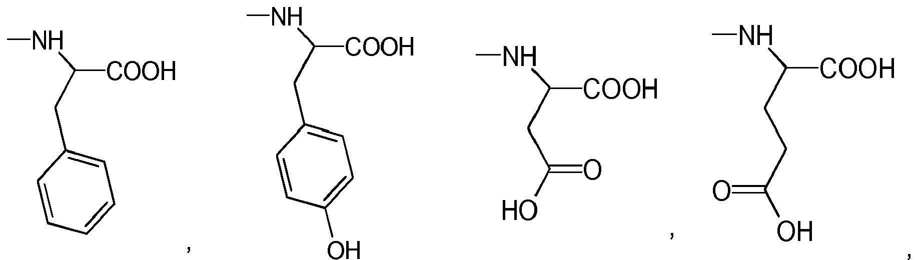 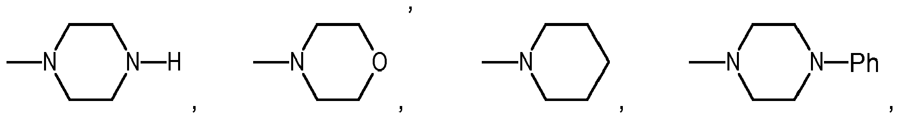 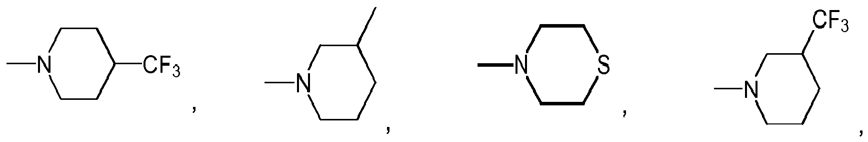 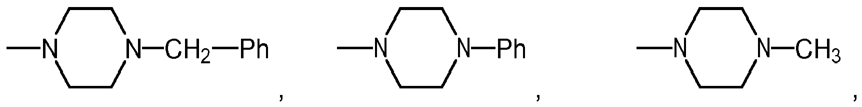 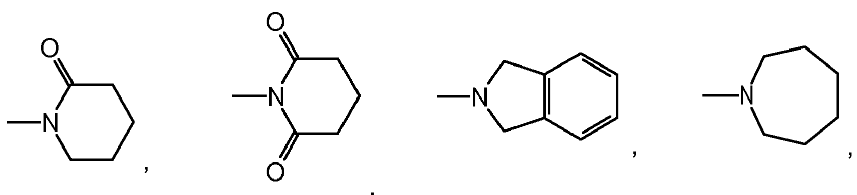 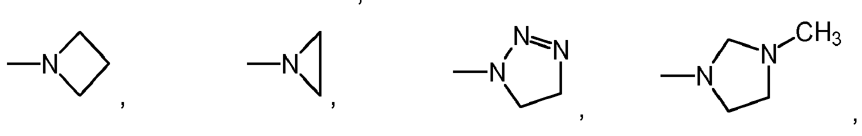 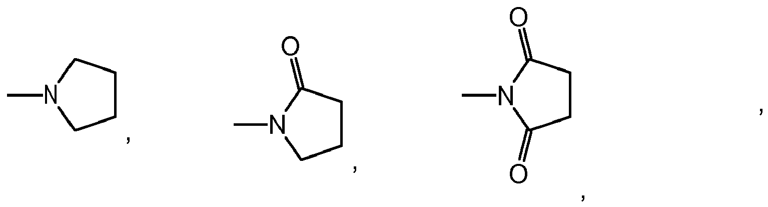 **Y** für einen der folgenden Reste steht: -CH₂R¹, -CHR¹-CH₂R², -CHR¹-CHR²-CH₂R³, -CHR¹-CHR²-CHR³-CH₂R⁴, -CHR¹-CHR²-CHR³-CHR⁴-CH₂R⁵, -CHR¹-CHR²-CHR³-CHR⁴-CHR⁵-CH₂R⁶
**Y'**, **R¹⁷** bis **R²⁰** unabhängig voneinander ausgewählt werden aus: -H, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂ und -C₂H₄-CH(CH₃)₂;
**X** bedeutet: -CH₂R⁷, -CHR⁷-CH₂R⁸, -O-CH₂R⁷, -O-CHR⁷-CH₂R⁸, 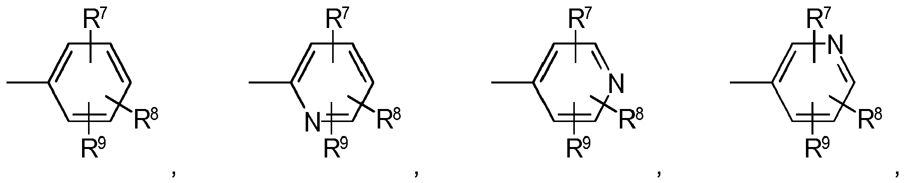 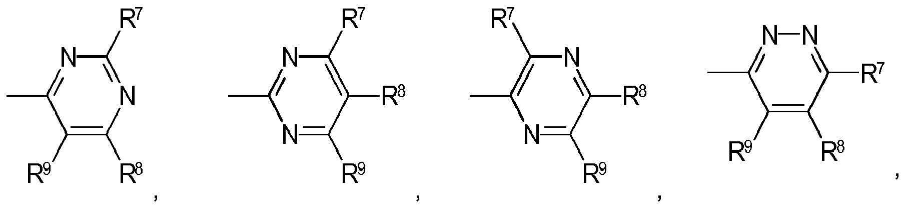 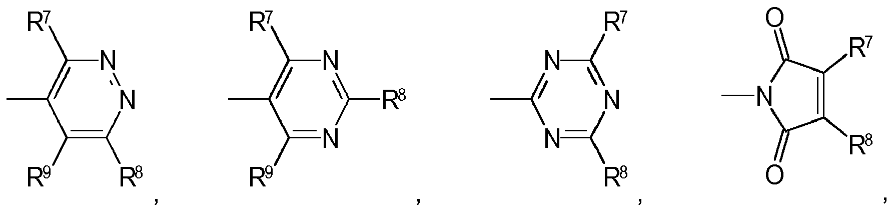 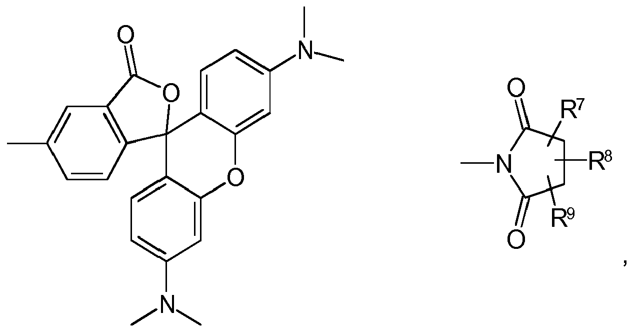 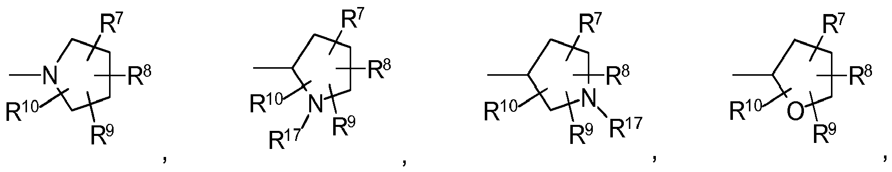 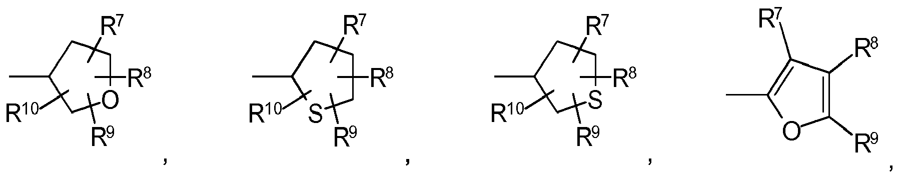 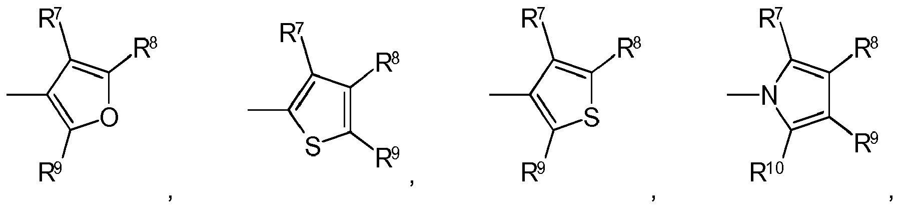 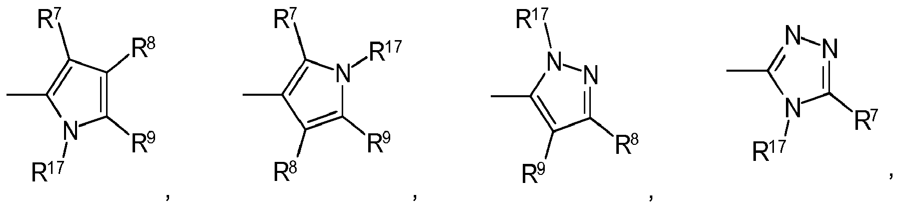 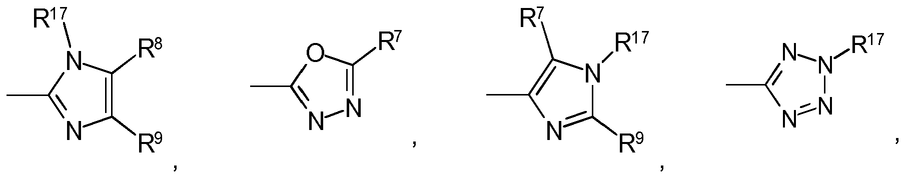 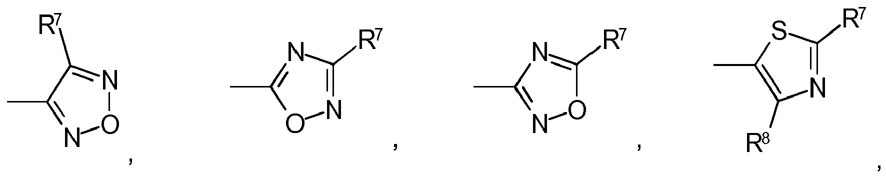 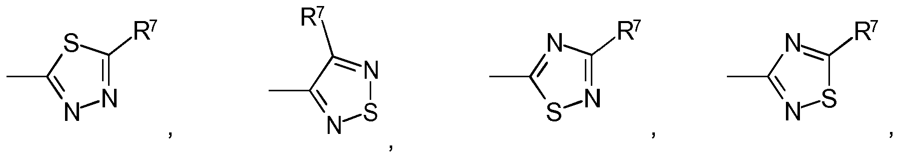 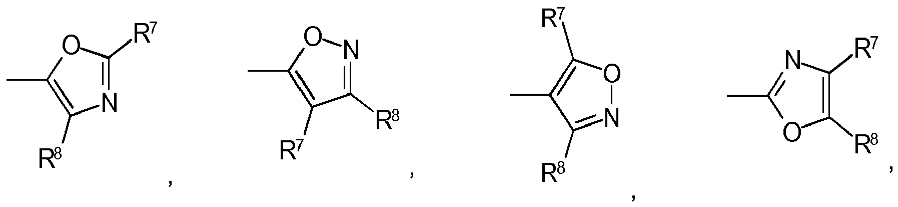 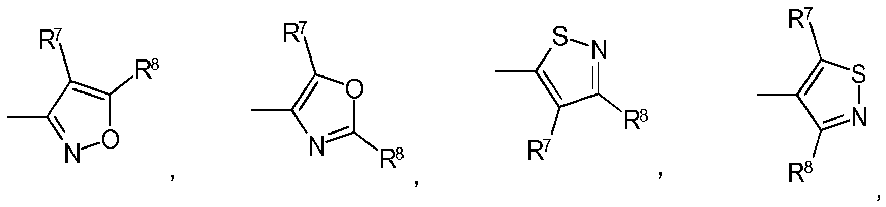 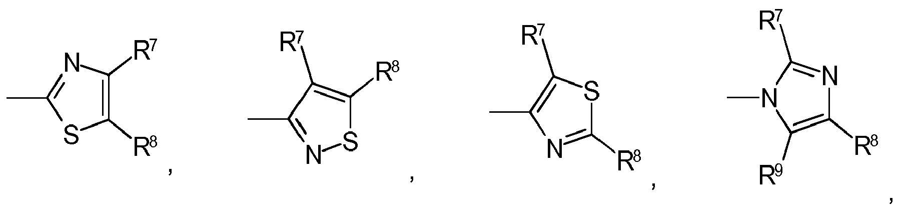 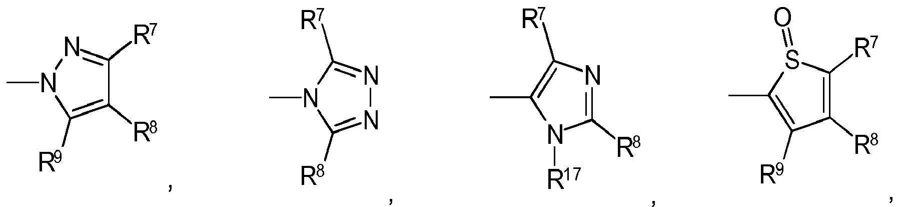 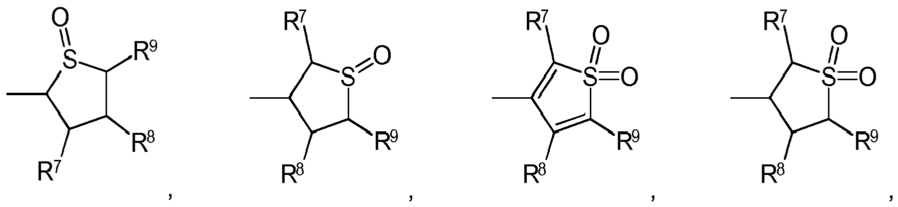 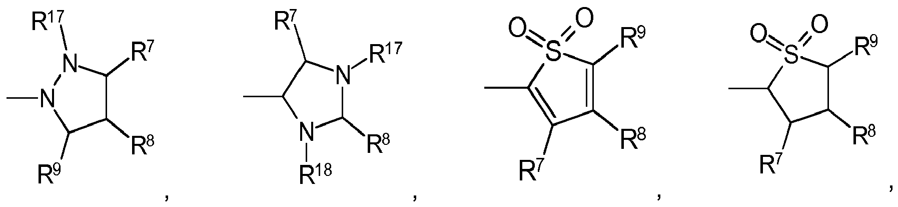 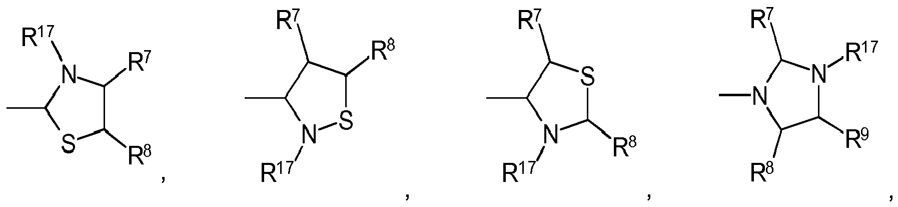 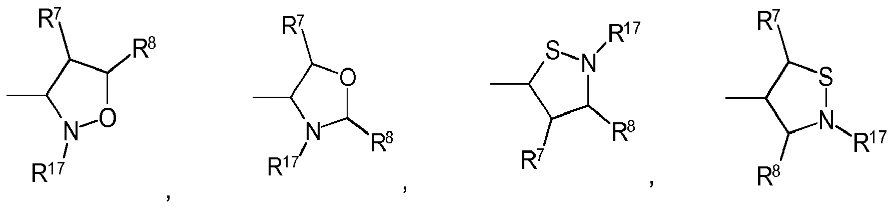 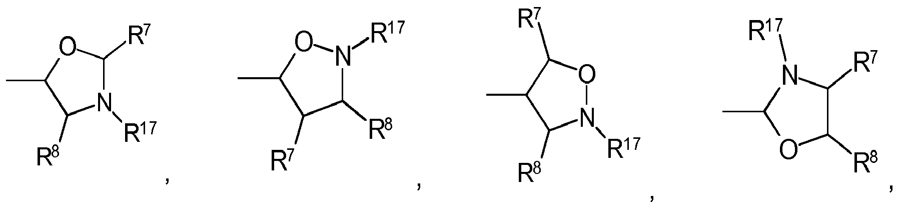 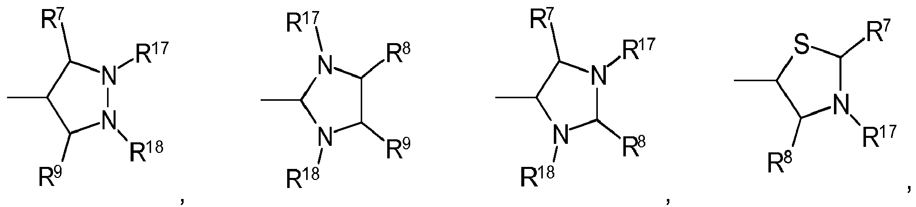 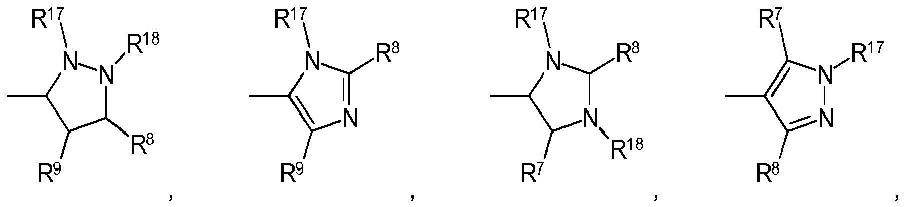 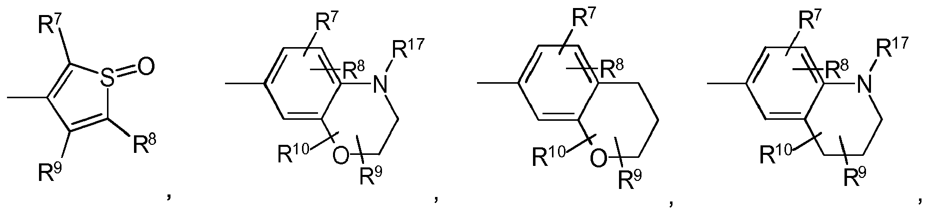 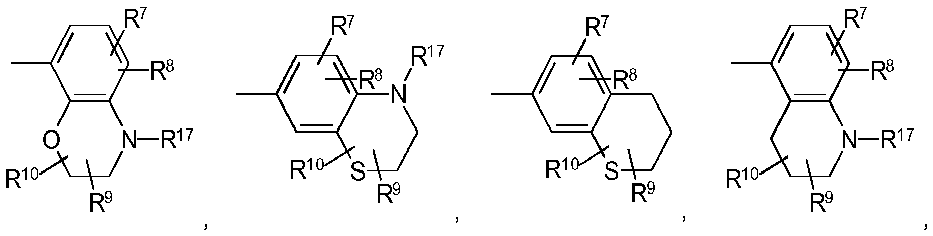 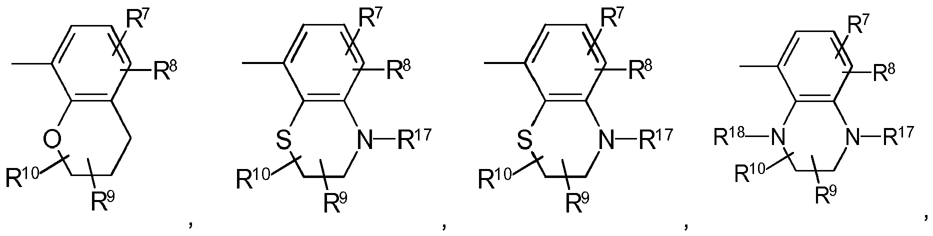 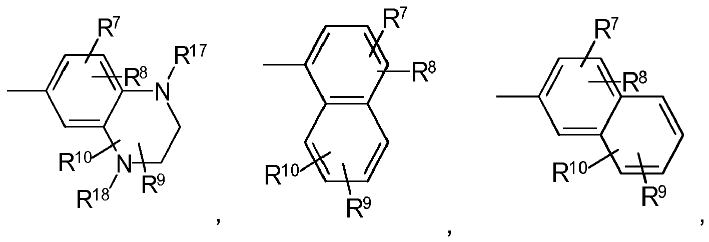 **Z** bedeutet: -CH₃, -C₂H₅, -C₃H₇, -CH(CH₃)₂, -C₄H₉, -Ph, -CH₂-Ph, -OCH₃, -OC₂H₅, -OC₃H₇, -OCH(CH₃)₂, -OC₄H₉, -OPh, -OCH₂-Ph, -OCH=CH₂ oder -OCH₂-CH=CH₂;
**R¹** bis **R¹⁰** unabhängig voneinander für folgende Gruppen stehen: -H, -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -SH, -NO₂, -F, -Cl, -Br, -I, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -OCF₃, -OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-CO-NH-cyclo-C₃H₅, -NH-CO-NH[CH(CH₃)₂], -NH-CO-NH[C(CH₃)₃], -NH-CO-N(CH₃)₂, -NH-CO-N(C₂H₅)₂, -NH-CO-N(C₃H₇)₂, -NH-CO-N(cyclo-C₃H₅)₂, -NH-CO-N[CH(CH₃)₂]₂, -NH-CO-N[C(CH₃)₃]₂, -NH-CS-NH₂, -NH-CS-NHCH₃, -NH-CS-NHC₂H5, -NH-CS-NHC₃H₇, -NH-CS-NH-cyclo-C₃H₅, -NH-CS-NH[CH(CH₃)₂], -NH-CS-NH[C(CH₃)₃], -NH-CS-N(CH₃)₂, -NH-CS-N(C₂H₅)2, -NH-CS-N(C₃H₇)₂, -NH-CS-N(cyclo-C₃H₅)₂, NH-CS-N[CH(CH₃)₂]₂, -NH-CS-N[C(CH₃)₃]₂, -NH₂*HOOCCF₃, -CH₂F, -CF₂Cl, -CHF₂, -CF₃, -CH₂Cl, -CH₂Br, -CH₂l, -CH₂-CH₂F, -CH₂-CHF₂, -CH₂-CF₃, -CH₂-CH₂Cl, -CH₂-CH₂Br, -CH₂-CH₂l, cyclo-C₃H₅, cyclo-C₄H₇, cyclo-C₅H₉, cyclo-C₆H₁₁, cyclo-C₇H₁₃, cyclo-C₈H₁₅, -Ph, -CH₂-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -C₇H₁₅, -C₈H₁₇, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH₂NH₂, -CH₂OH, -CH₂SH, -CH₂-CH₂NH₂, -CH₂-CH₂SH, -C₆H₄-OCH₃, -C₆H₄-OH, -CH₂-CH₂-OCH₃, -CH₂-CH₂OH, -CH₂-OCH₃, -CH₂-C₆H₄-OCH₃, CH₂-C₆H₄-OH, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C3H₇, 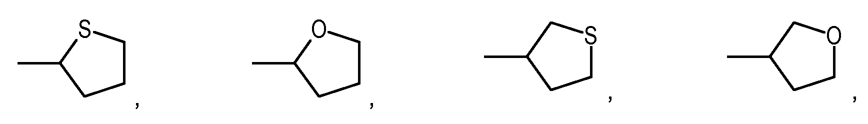 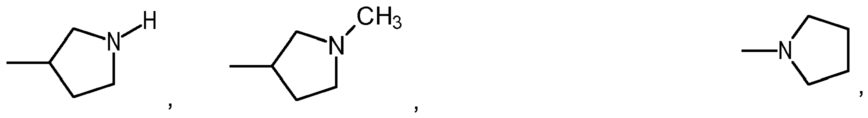 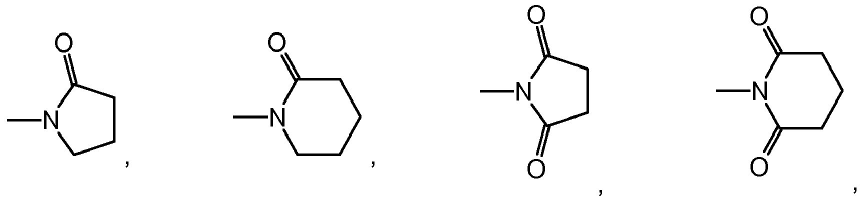 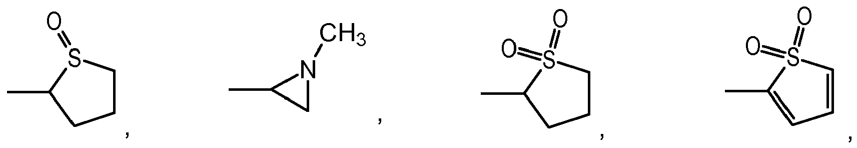 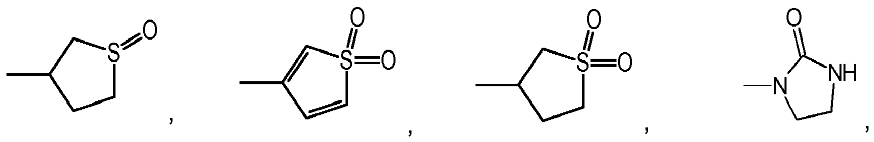 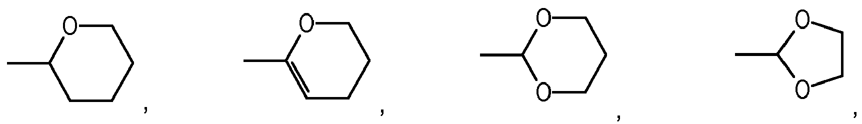 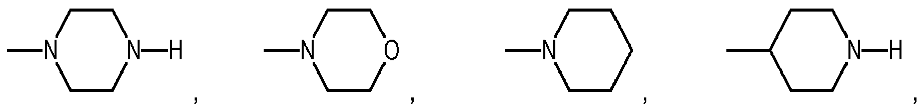 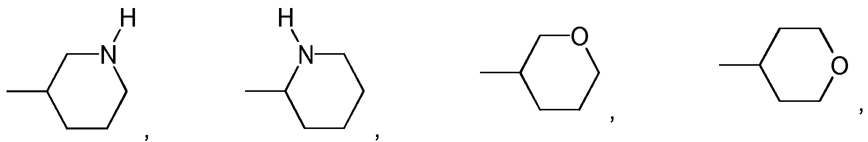 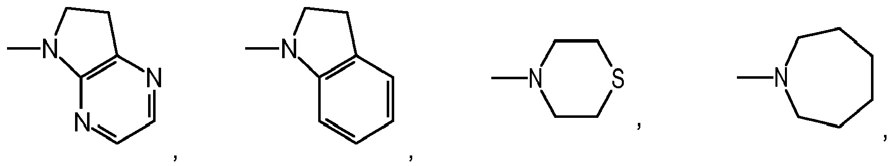 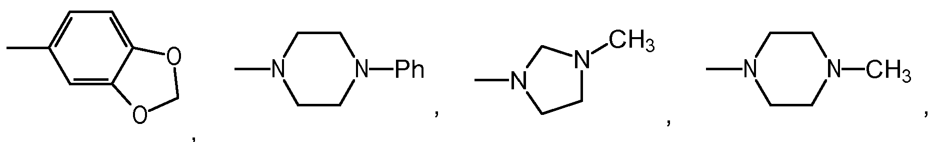 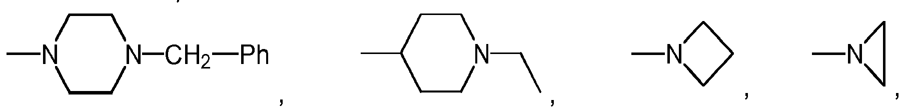 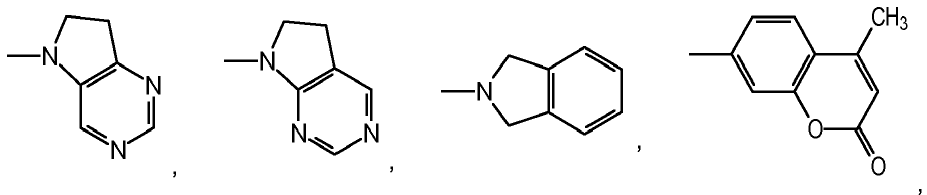 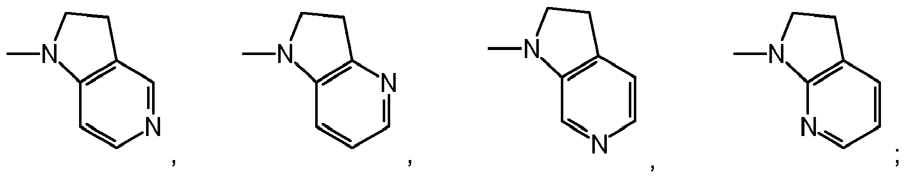 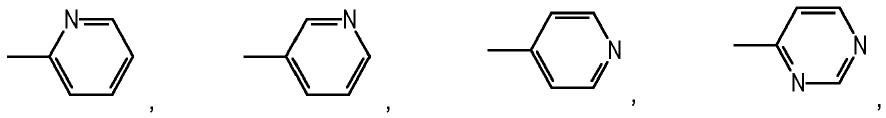 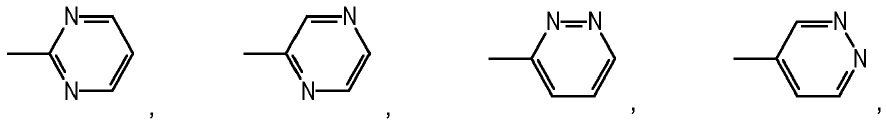 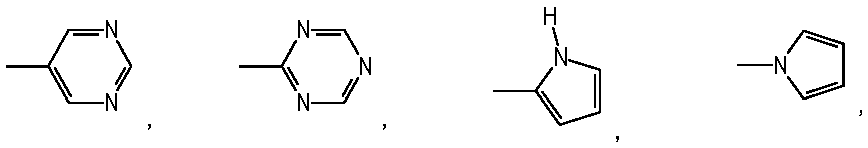 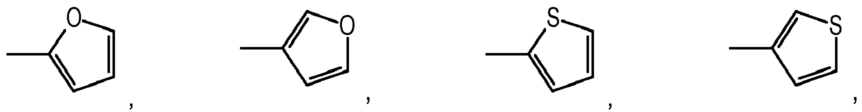 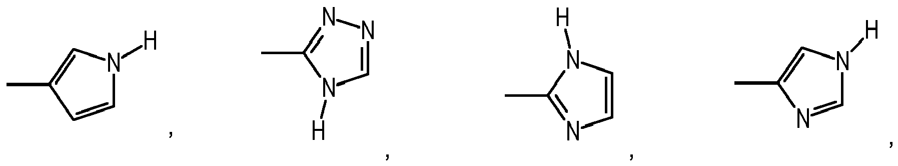 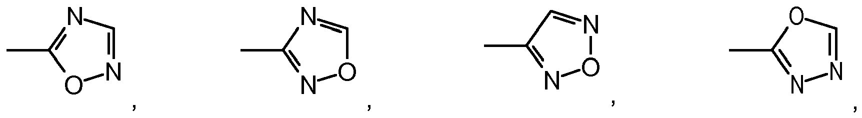 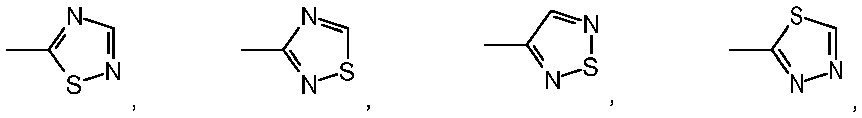 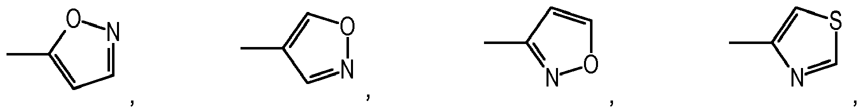 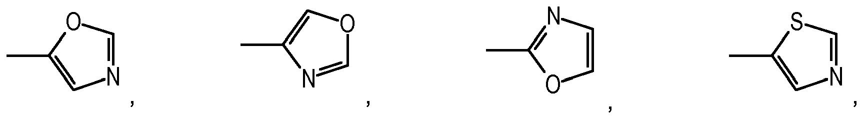 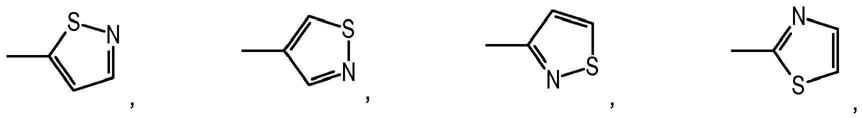 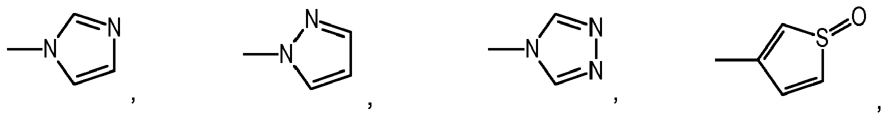 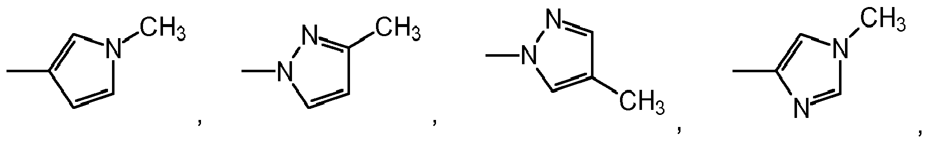 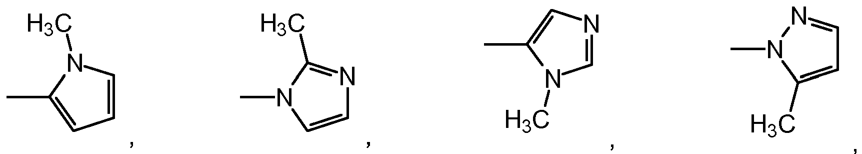 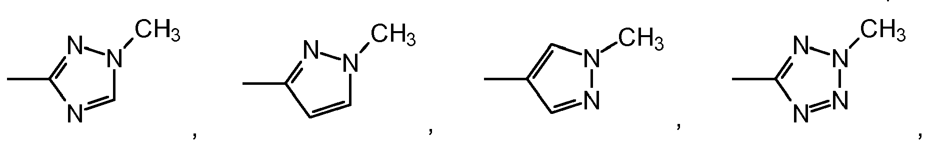 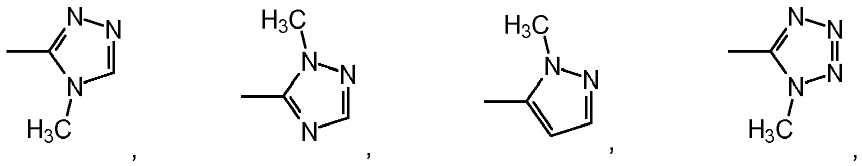 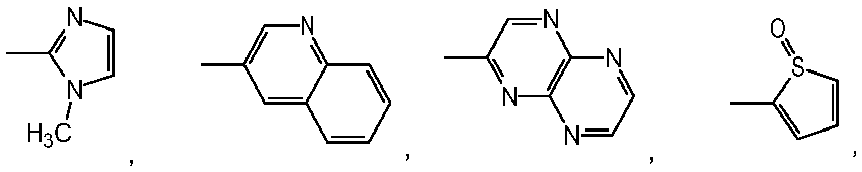 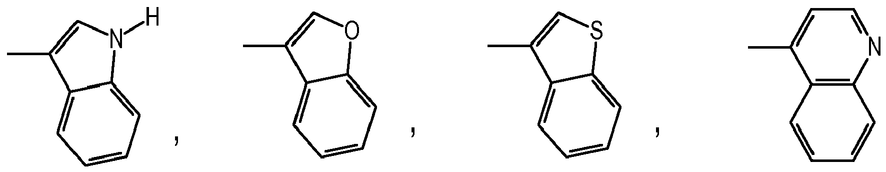 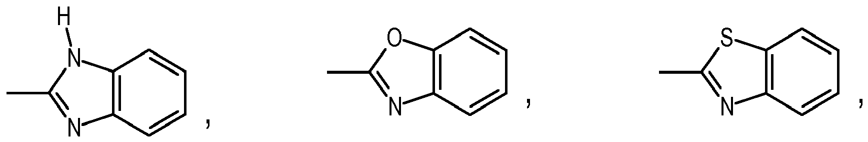 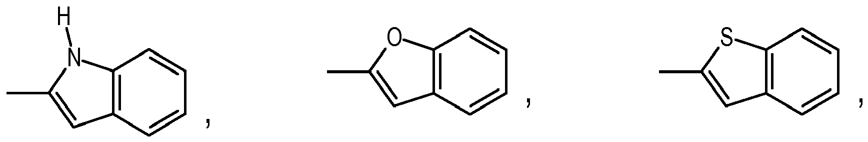 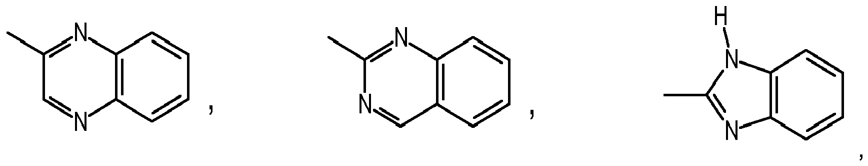 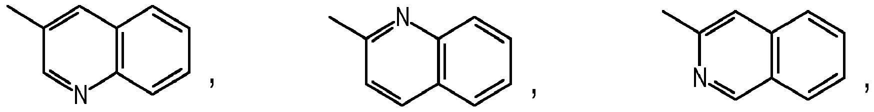 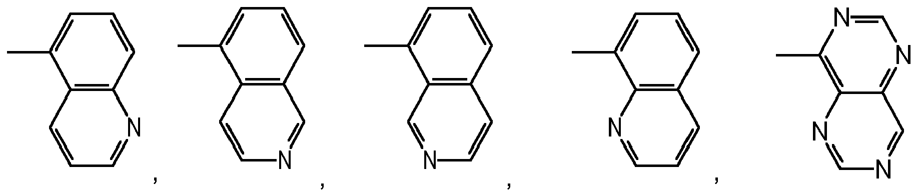 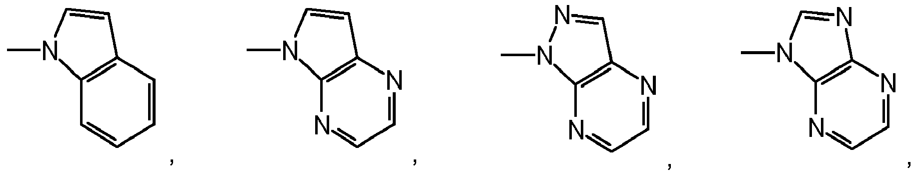 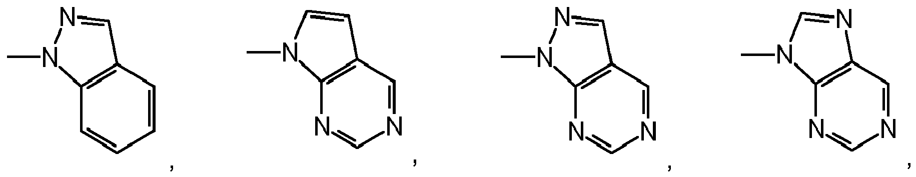 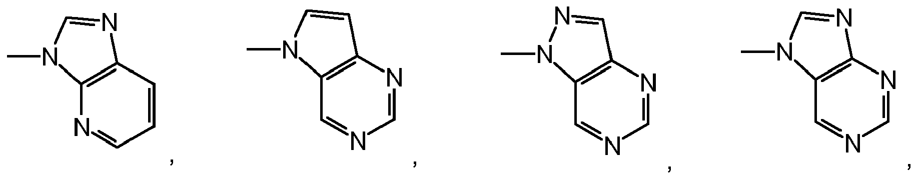 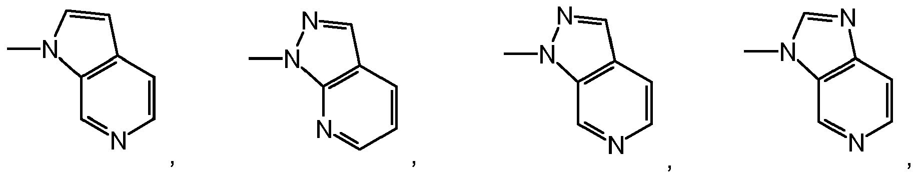 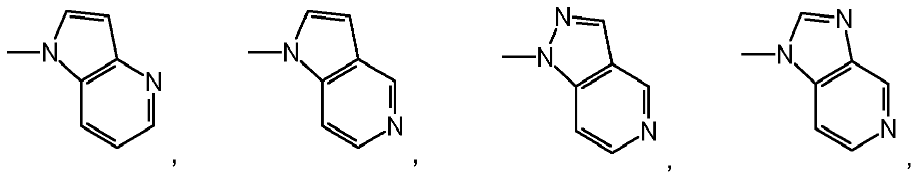 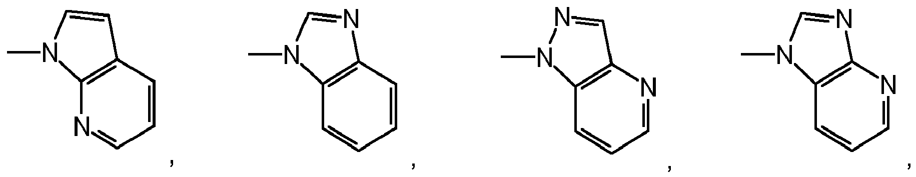 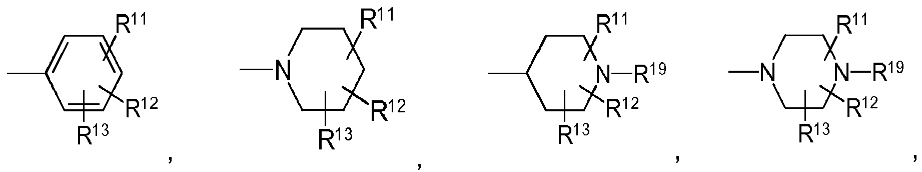 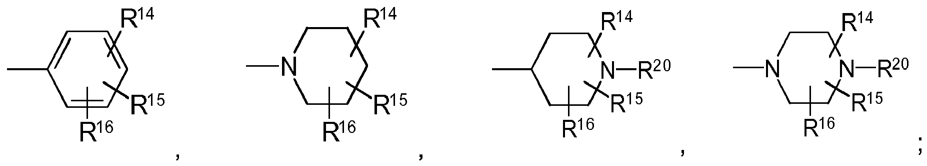 die Reste **R¹¹** bis **R¹⁶** unabhängig voneinander für folgende Gruppen stehen: -H, -NH₂, -OH, -OCH₃, -OC₂H₅, -OC₃H₇, -OCF₃, -CF₃, -F, -Cl, -Br, -I, -CH₃, -C₂H₅, -C₃H₇, -CH(CH₃)₂, -Ph und -CN;
sowie stereoisomere Formen, E/Z-Isomere, Enantiomere, Enantiomerengemische, Diastereomere, Diastereomerengemische, Racemate, Tautomere, Anomere, Keto-Enol-Formen, Betainformen, Prodrugs, Solvate, Hydrate als auch pharmakologisch verträgliche Salze der vorgenannten Verbindungen.

2. Verbindungen gemäß Anspruch 1, worin
**Z** für -OCH₃, -OCH₂CH₃, -OCH(CH₃)₂ oder -OCH₂-Ph steht,
**R^{#}** für-NHY steht,
**Y** die Bedeutung hat wie in Anspruch 1 definiert.

3. Verbindungen gemäß Anspruch 2, worin
**Y** für -CH₂R¹, -CHR¹-CH₂R², -CHR¹-CHR²-CH₂R³, -CHR¹-CHR²-CHR³-CH₂R⁴ oder -CHR¹-CHR²-CHR³-CHR⁴-CH₂R⁵ steht und
R¹ bis R⁵ unabhängig voneinander bedeuten: -COOH, -COOCH₃, -N(CH₃)₂, -N(C₂H₅)₂, -Ph, -CH₃, -C₂H₅, -CH₂-CH(CH₃)₂, -C(CH₃)₃,

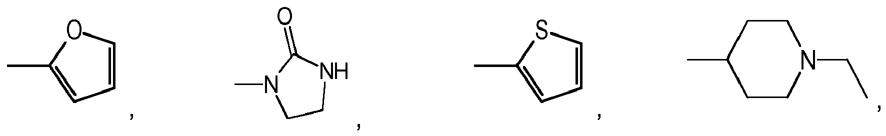

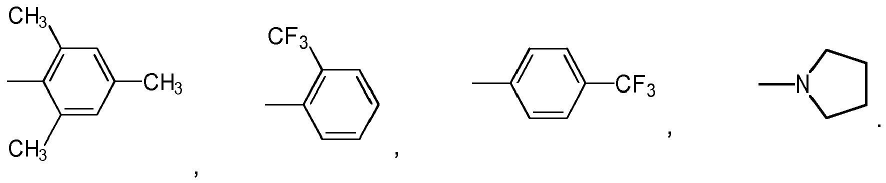

4. Verbindungen gemäß einem der Ansprüche 1 - 3, worin
Y bedeutet -CH₃, -C₂H₅, -C₃H₇, -C₄H₉, -C₅H₁₁, -C₂H₄-CH(CH₃)₂, 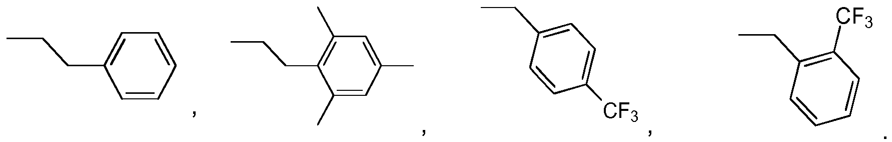 -CH(CH₃)-CH₂-CH(CH₃)₂, -CH₂-CH(C₂H₅)₂, -CH₂-CH(CH₃)-C₂H₅, -C₂H₄-C(CH₃)₃ oder -C₂H₄-CH(C₂H₅)₂,

5. Verbindungen gemäß einem der Ansprüche 1 - 4, worin
**X** für -CH₂R⁷, -CHR⁷-CH₂R⁸, 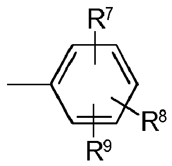 oder 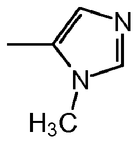 steht.

6. Verbindungen gemäß einem der vorherigen Ansprüche, worin
**Z** für -OCH₃, -OCH₂CH₃ oder -OCH₂-Ph steht,
**E¹** für -CO- oder -SO₂- steht,
**E²** für -CO- steht
R^{#} für eine der folgenden Reste steht: -NYY', 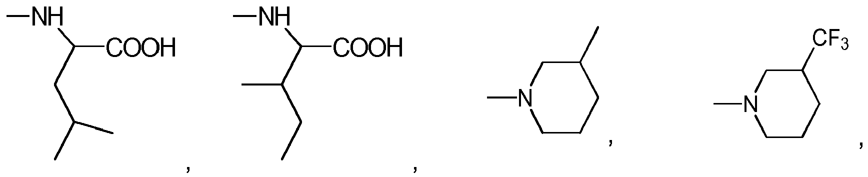 oder 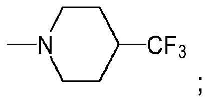 Y' für -H steht;
Y für -CH₂R¹, -CHR¹-CH2R², -CHR¹-CHR²-CH₂R³, -CHR¹-CHR²-CHR³-CH₂R⁴ oder -CHR¹-CHR²-CHR³-CHR⁴-CH₂R⁵ steht;
**R¹** bis **R⁵** unabhängig voneinander bedeuten: -H, -CH₃, -C₂H₅, -Ph, -CH₂-CH(CH₃)₂, -N(CH₃)₂, -N(C₂H₅)₂, -COOH, -COOCH₃, -C(CH₃)₃, 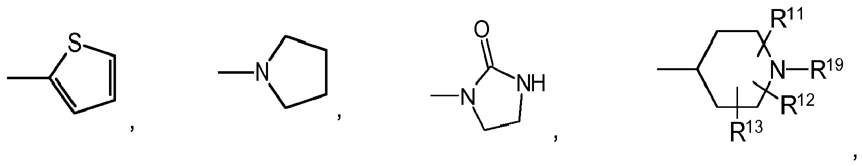 oder 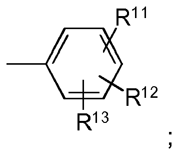 **R¹¹** bis **R¹³** unabhängig voneinander -H, -CH₃, -C₂H₅ oder -CF₃ bedeuten,
**R¹⁹** -C₂H₅ bedeutet,
**X** für -CH₂R⁷, -CHR⁷-CH₂R⁸, -O-CH₂R⁷, 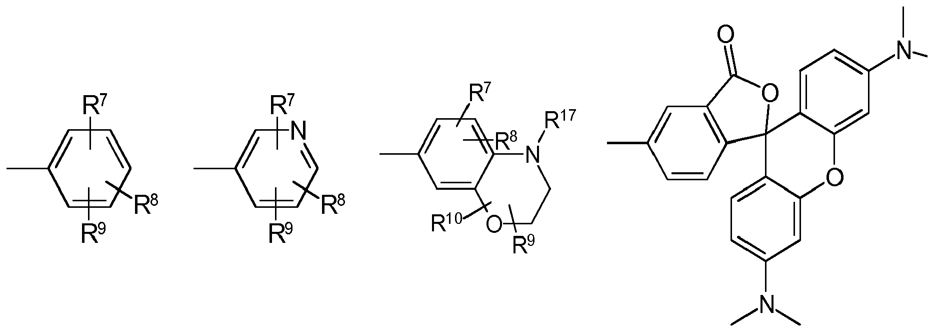 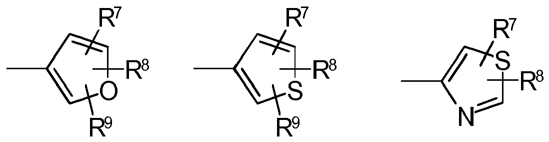 oder 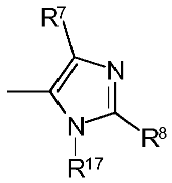 **R**⁷ bis **R**¹⁰ unabhängig voneinander bedeuten -H, -OH, -COOH, -SO₂NH₂, -CH₃, -CF₃, -NH-CO-NH₂, -NH₂*HOOCCF₃ oder -NH₂, 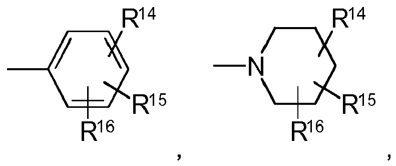 oder 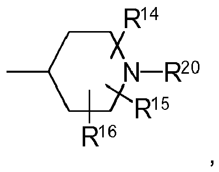 **R¹⁴** bis **R¹⁶** unabhängig voneinander -H, -Cl oder -CH₃, bedeuten, und
**R¹⁷** und **R²⁰** -CH₃ bedeuten.

7. Verbindungen gemäß Anspruch 1 der allgemeinen Formel (II): 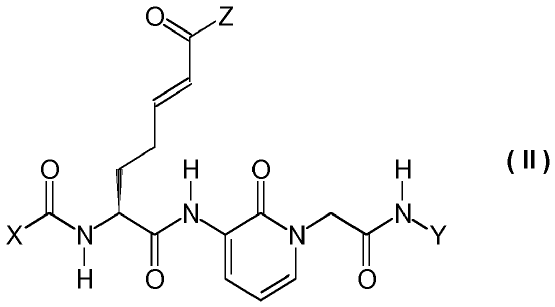 worin **X, Y** und **Z** die Bedeutung wie in Anspruch 1 haben.

8. Verbindungen gemäß Anspruch 1 ausgewählt aus der Gruppe bestehend aus:
(S,E)-Ethyl 6-(benzyloxycarbonylamino)-7-oxo-7-(2-oxo-1-(2-oxo-2-(2,4,6-trimethylphenethylamino)ethyl)-1,2-dihydropyridin-3-ylamino)hept-2-enoate
(S,E)-Ethyl 6-(benzyloxycarbonylamino)-7-(1-(2-(isopentylamino)-2-oxoethyl)-6-methyl-2-oxo-1,2-dihydropyridin-3-ylamino)-7-oxohept-2-enoate
(S,E)-Ethyl 6-(benzyloxycarbonylamino)-7-(1-(2-(isopentylamino)-2-oxoethyl)-2-oxo-1,2-d ihyd ropyrid in-3-ylam ino)-7-oxohept-2-enoate
(S,E)-Ethyl 6-acetamido-7-(1-(2-(isopentylamino)-2-oxoethyl)-6-methyl-2-oxo-1,2-d ihyd ropyrid in-3-ylam ino)-7-oxohept-2-enoate
3-(2-(3-((S,E)-2-(Benzyloxycarbonylamino)-7-ethoxy-7-oxohept-5-enamido)-6-methyl-2-oxopyridin-1 (2H)-yl)acetamido)-5-methylhexanoic acid
(S,E)-isopropyl 6-acetamido-7-(1-(2-(isopentylamino)-2-oxoethyl)-2-oxo-1,2-di-hydropyrid in-3-ylam ino)-7-oxohept-2-enoate
(S,E)-Ethyl 6-acetamido-7-(1-(2-(isopentylamino)-2-oxoethyl)-2-oxo-1,2-dihydro-pyridin-3-ylamino)-7-oxohept-2-enoate
(S,E)-Ethyl 7-(6-methyl-2-oxo-1-(2-oxo-2-(phenethylamino)ethyl)-1,2-dihydro-pyridin-3-ylamino)-6-(nicotinamido)-7-oxohept-2-enoate
(S,E)-ethyl 6-((4-chlorophenyl)methylsulfonamido)-7-(1-(2-(isopentylamino)-2-oxoethyl)-2-oxo-1,2-dihydropyridin-3-ylamino)-7-oxohept-2-enoate
(S,E)-Ethyl 6-benzamido-7-(1-(2-(isopentylamino)-2-oxoethyl)-2-oxo-1,2-dihydro-pyridin-3-ylamino)-7-oxohept-2-enoate
(S,E)-Ethyl 6-(furan-3-carboxamido)-7-(1-(2-(isopentylamino)-2-oxoethyl)-2-oxo-1,2-dihydropyridin-3-ylamino)-7-oxohept-2-enoate
(S,E)-Ethyl 7-(1-(2-(isopentylamino)-2-oxoethyl)-2-oxo-1,2-dihydropyridin-3-ylamino)-7-oxo-6-(thiophene-3-carboxamido)hept-2-enoate
(S,E)-Ethyl 6-(furan-3-sulfonamido)-7-(1-(2-(isopentylamino)-2-oxoethyl)-2-oxo-1,2-dihydropyridin-3-ylamino)-7-oxohept-2-enoate
(S,E)-Ethyl 7-(1-(2-(isopentylamino)-2-oxoethyl)-2-oxo-1,2-dihydropyridin-3-ylamino)-7-oxo-6-(4-sulfamoylbenzamido)hept-2-enoate
(S,E)-Ethyl 7-(1-(2-(isopentylamino)-2-oxoethyl)-2-oxo-1,2-dihydropyridin-3-ylamino)-6-(5-methylthiazole-4-carboxamido)-7-oxohept-2-enoate
(S,E)-Ethyl 7-(1-(2-(isopentylamino)-2-oxoethyl)-2-oxo-1,2-dihydropyridin-3-ylamino)-6-(nicotinamido)-7-oxohept-2-enoate
(S,E)-Ethyl 6-(3,5-bis(trifluoromethyl)benzamido)-7-(1-(2-(isopentylamino)-2-oxoethyl)-2-oxo-1,2-dihydropyridin-3-ylamino)-7-oxohept-2-enoate
(S,E)-Ethyl 7-(1-(2-(isopentylamino)-2-oxoethyl)-2-oxo-1,2-dihydropyridin-3-ylamino)-7-oxo-6-(4-(piperidin-1-yl)benzamido)hept-2-enoate
(S,E)-Ethyl 7-(1-(2-(isopentylamino)-2-oxoethyl)-2-oxo-1,2-dihydropyridin-3-ylamino)-6-(1-methyl-1H-imidazole-5-carboxamido)-7-oxohept-2-enoate
(S,E)-Ethyl 7-(1-(2-(isopentylamino)-2-oxoethyl)-2-oxo-1,2-dihydropyridin-3-ylamino)-6-(4-(4-methylpiperazin-1-yl)benzamido)-7-oxohept-2-enoate
(S,E)-Ethyl 7-(1-(2-(isopentylamino)-2-oxoethyl)-2-oxo-1,2-dihydropyridin-3-ylamino)-6-(4-methyl-3,4-dihydro-2H-benzo[b][1,4]oxazine-7-carboxamido)-7-oxohept-2-enoate
(S,E)-Ethyl 7-(1-(2-(isopentylamino)-2-oxoethyl)-2-oxo-1,2-dihydropyridin-3-ylamino)-7-oxo-6-(phenylsulfonamido)hept-2-enoate
(S,E)-5-(N-(7-Ethoxy-1-(1-(2-(isopentylamino)-2-oxoethyl)-2-oxo-1,2-dihydropyridin-3-ylamino)-1,7-dioxohept-5-en-2-yl)sulfamoyl)-2-hydroxybenzoic acid
(S,E)-4-(7-Ethoxy-1-(1-(2-(isopentylamino)-2-oxoethyl)-2-oxo-1,2-dihydropyridin-3-ylamino)-1,7-dioxohept-5-en-2-ylamino)-4-oxobutanoic acid
(S,E)-Ethyl 6-acetamido-7-(1-(2-(2-(diethylamino)ethylamino)-2-oxoethyl)-2-oxo-1,2-dihydropyridin-3-ylamino)-7-oxohept-2-enoate
(S,E)-4-(1-(1-(2-(2-(Diethylamino)ethylamino)-2-oxoethyl)-2-oxo-1,2-dihydropyridin-3-ylamino)-7-ethoxy-1,7-dioxohept-5-en-2-ylamino)-4-oxobutanoic acid
(S,E)-Ethyl 6-acetamido-7-(1-(2-(2-(dimethylamino)ethylamino)-2-oxoethyl)-2-oxo-1,2-dihydropyridin-3-ylamino)-7-oxohept-2-enoate
(S,E)-Methyl 6-acetamido-7-(1-(2-(isopentylamino)-2-oxoethyl)-2-oxo-1,2-dihydropyridin-3-ylamino)-7-oxohept-2-enoate
(S,E)-methyl 6-acetamido-7-(1-(2-(isopentylamino)-2-oxoethyl)-2-oxo-1,2-dihydropyridin-3-ylamino)-7-oxohept-2-enoate
(S,E)-4-(7-Ethoxy-1-(1-(2-(2-ethylbutylamino)-2-oxoethyl)-2-oxo-1,2-dihydropyridin-3-ylamino)-1,7-dioxohept-5-en-2-ylamino)-4-oxobutanoic acid
(S,E)-Ethyl 6-acetamido-7-(1-(2-((S)-1-methoxy-4-methyl-1-oxopentan-2-ylamino)-2-oxoethyl)-2-oxo-1,2-dihydropyridin-3-ylamino)-7-oxohept-2-enoate
4-((S,E)-7-Ethoxy-1-(1-(2-((S)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid
(S,E)-Ethyl 6-acetamido-7-(1-(2-((R)-1-methoxy-4-methyl-1-oxopentan-2-ylamino)-2-oxoethyl)-2-oxo-1,2-dihydropyridin-3-ylamino)-7-oxohept-2-enoate
4-((S,E)-7-Ethoxy-1-(1-(2-((R)-1-methoxy-4-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid
(S,E)-ethyl 6-acetamido-7-(1-(2-((2S,3R)-1-methoxy-3-methyl-1-oxopentan-2-ylamino)-2-oxoethyl)-2-oxo-1,2-dihydropyridin-3-ylamino)-7-oxohept-2-enoate
4-((S,E)-7-Ethoxy-1-(11-(2-((2S,3R)-1-methoxy-3-methyl-1-oxopentan-2-ylamino)-2-oxoethyl)-2-oxo-1,2-dihydropyridin-3-ylamino)-1,7-dioxohept-5-en-2-ylamino)-4-oxobutanoic acid
(S,E)-4-(7-Ethoxy-1,7-dioxo-1-(2-oxo-1-(2-oxo-2-(4-(trifluoromethyl)benzylamino)ethyl)-1,2-dihydropyridin-3-ylamino)hept-5-en-2-ylamino)-4-oxobutanoic acid
(S,E)-4-(1-(1-(2-(3,3-Dimethylbutylamino)-2-oxoethyl)-2-oxo-1,2-dihydropyridin-3-ylamino)-7-ethoxy-1,7-dioxohept-5-en-2-ylamino)-4-oxobutanoic acid (S,E)-Ethyl 6-acetamido-7-(1-(2-(3,3-dimethylbutylamino)-2-oxoethyl)-2-oxo-1,2-dihydropyridin-3-ylamino)-7-oxohept-2-enoate
(S,E)-Ethyl 6-acetamido-7-(1-(2-((S)-1-methoxy-4,4-dimethyl-1-oxopentan-2-ylamino)-2-oxoethyl)-2-oxo-1,2-dihydropyridin-3-ylamino)-7-oxohept-2-enoate
4-((S,E)-7-Ethoxy-1-(1-(2-((S)-1-methoxy-4,4-dimethyl-1-oxopentan-2-ylamino)-2-oxoethyl)-2-oxo-1,2-dihydropyridin-3-ylamino)-1,7-dioxohept-5-en-2-ylamino)-4-oxobutanoic acid
(S,E)-ethyl 6-acetamido-7-(1-(2-(3-ethylpentylamino)-2-oxoethyl)-2-oxo-1,2-dihydropyridin-3-ylamino)-7-oxohept-2-enoate
(S,E)-4-(7-Ethoxy-1-(1-(2-(3-ethylpentylamino)-2-oxoethyl)-2-oxo-1,2-dihydropyridin-3-ylamino)-1,7-dioxohept-5-en-2-ylamino)-4-oxobutanoic acid (S,E)-4-(7-Ethoxy-1,7-dioxo-1-(2-oxo-1-(2-oxo-2-(2-(trifluoromethyl)benzylamino)ethyl)-1,2-dihydropyridin-3-ylamino)hept-5-en-2-ylamino)-4-oxobutanoic acid
(S,E)-4-(7-Ethoxy-1,7-dioxo-1-(2-oxo-1-(2-oxo-2-(4-(trifluoromethyl)piperidin-1-yl)ethyl)-1,2-dihydropyridin-3-ylamino)hept-5-en-2-ylamino)-4-oxobutanoic acid
(S,E)-Ethyl 6-acetam ido-7-oxo-7-(2-oxo-1-(2-oxo-2-(2-(pyrrol id in-1-yl)ethylamino)ethyl)-1,2-dihydropyridin-3-ylamino)hept-2-enoate
(S,E)-4-(7-Ethoxy-1,7-dioxo-1-(2-oxo-1-(2-oxo-2-(2-(thiophen-2-yl)ethylamino)ethyl)-1,2-dihydropyridin-3-ylamino)hept-5-en-2-ylamino)-4-oxobutanoic acid
(S,E)-Ethyl 6-acetamido-7-(1-(2-((1-ethylpiperidin-4-yl)methylamino)-2-oxoethyl)-2-oxo-1,2-dihydropyridin-3-ylamino)-7-oxohept-2-enoate
(S,E)-4-(7-Ethoxy-1,7-dioxo-1-(2-oxo-1-(2-oxo-2-(2-(2-oxoimidazolidin-1-yl)ethylamino)ethyl)-1,2-dihydropyridin-3-ylamino)hept-5-en-2-ylamino)-4-oxobutanoic acid
(6S,E)-Ethyl 6-acetamido-7-(1-(2-(2-methylbutylamino)-2-oxoethyl)-2-oxo-1,2-dihydropyridin-3-ylamino)-7-oxohept-2-enoate
4-((2S,E)-7-Ethoxy-1-(1-(2-(2-methylbutylamino)-2-oxoethyl)-2-oxo-1,2-dihydropyridin-3-ylamino)-1,7-dioxohept-5-en-2-ylamino)-4-oxobutanoic acid
(6S,E)-Ethyl 6-acetamido-7-(1-(2-(3-methylpiperidin-1-yl)-2-oxoethyl)-2-oxo-1,2-dihydropyridin-3-ylamino)-7-oxohept-2-enoate
(2S,3R)-2-(2-(3-((S,E)-7-Ethoxy-2-(1-methyl-1 H-imidazole-5-carboxamido)-7-oxohept-5-enamido)-2-oxopyridin-1 (2H)-yl)acetamido)-3-methylpentanoic acid
(S,E)-Ethyl 6-acetamido-7-(1-(2-(isobutylamino)-2-oxoethyl)-2-oxo-1,2-dihydropyridin-3-ylamino)-7-oxohept-2-enoate
(6S,E)-Ethyl 6-acetamido-7-(1-(2-(3-methylbutan-2-ylamino)-2-oxoethyl)-2-oxo-1,2-dihydropyridin-3-ylamino)-7-oxohept-2-enoate
(S,E)-Ethyl 7-(1-(2-(isopentylamino)-2-oxoethyl)-2-oxo-1,2-dihydropyridin-3-ylamino)-7-oxo-6-(3-ureidopropanamido)hept-2-enoate
(S,E)-Ethyl 6-acetamido-7-(1-(2-((S)-1-methoxy-3-methyl-1-oxobutan-2-ylamino)-2-oxoethyl)-2-oxo-1,2-dihydropyridin-3-ylamino)-7-oxohept-2-enoate
(S,E)-Ethyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate (2S,3R)-2-(2-(3-((S,E)-2-Benzamido-7-ethoxy-7-oxohept-5-enamido)-2-oxopyridin-1 (2H)-yl)acetamido)-3-methylpentanoic acid
(2S,3R)-2-(2-(3-((S,E)-7-Methoxy-2-(1-methyl-1H-imidazole-5-carboxamido)-7-oxohept-5-enamido)-2-oxopyridin-1(2H)-yl)acetamido)-3-methylpentanoic acid
(2S,3R)-2-(2-(3-((S,E)-2-benzamido-7-methoxy-7-oxohept-5-enamido)-2-oxopyridin-1(2H)-yl)acetamido)-3-methylpentanoic acid
(S,E)-Ethyl 6-acetamido-7-(1-(2-(2-ethylbutylamino)-2-oxoethyl)-2-oxo-1,2-d ihydropyrid in-3-ylam ino)-7-oxohept-2-enoate
(S,E)-Methyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate
(6S,E)-Methyl 6-acetamido-7-(1-(2-(3-methylpiperidin-1 -yl)-2-oxoethyl)-2-oxo-1,2-dihydropyridin-3-ylamino)-7-oxohept-2-enoate
(S,E)-Methyl 6-acetamido-7-oxo-7-(2-oxo-1-(2-oxo-2-(4-(trifluoromethyl)piperidin-1-yl)ethyl)-1,2-dihydropyridin-3-ylamino)hept-2-enoate
(6S,E)-Methyl 6-acetam ido-7-oxo-7-(2-oxo-1-(2-oxo-2-(3-(trifluoromethyl)piperidin-1-yl)ethyl)-1,2-dihydropyridin-3-ylamino)hept-2-enoate
(S,E)-Methyl 7-(1-(2-(2-ethylbutylamino)-2-oxoethyl)-2-oxo-1,2-dihydropyridin-3-ylamino)-6-(nicotinamido)-7-oxohept-2-enoate
(6S,E)-Methyl 6-(2-aminopropanamido)-7-(1-(2-(2-ethylbutylamino)-2-oxoethyl)-2-oxo-1,2-dihydropyridin-3-ylamino)-7-oxohept-2-enoate 2,2,2-trifluoroacetate
(S,E)-Methyl 6-(2-aminoacetamido)-7-(3-((2-(2-ethylbutylamino)-2-oxoethyl)(methyl)amino)-3-oxoprop-1-en-2-ylamino)-7-oxohept-2-enoate 2,2,2-trifluoroacetate
(S, E)-Methyl 6-(2-aminobenzamido)-7-(1-(2-(2-ethylbutylamino)-2-oxoethyl)-2-oxo-1,2-dihydropyridin-3-ylamino)-7-oxohept-2-enoate 2,2,2-trifluoroacetate
(S, E)-Methyl 6-(3',6'-bis(dimethylamino)-3-oxo-3H-spiro[isobenzofuran-1,9'-xanthene]-5-ylcarboxamido)-7-(1-(2-(2-ethylbutylamino)-2-oxoethyl)-2-oxo-1,2-dihydropyridin-3-ylamino)-7-oxohept-2-enoate
(S,E)-Ethyl 6-acetamido-7-(1-(2-(isopentylamino)-2-oxoethyl)-2-oxo-1,2-dihydropyridin-3-ylamino)-7-oxohept-2-enoate
(S, E)-Methyl 6-acetamido-7-(1-(2-(isopentylamino)-2-oxoethyl)-2-oxo-1,2-dihydropyridin-3-ylamino)-7-oxohept-2-enoate
(S,E)-Ethyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate
(2S,3R)-2-(2-(3-((S,E)-2-Benzamido-7-methoxy-7-oxohept-5-enamido)-2-oxopyridin-1(2H)-yl)acetamido)-3-methylpentanoic acid
(R,E)-Methyl 7-(1-(2-(2-ethylbutylamino)-2-oxoethyl)-2-oxo-1,2-dihydropyridin-3-ylamino)-6-(1-methyl-1H-imidazole-5-carboxamido)-7-oxohept-2-enoate
(S,E)-2-acetamido-N-(1-(2-(isopentylamino)-2-oxoethyl)-2-oxo-1,2-dihydropyridin-3-yl)-6-(methylsulfonyl)hex-5-enamide
(S,E)-Benzyl 6-acetamido-7-(1-(2-(isopentylamino)-2-oxoethyl)-2-oxo-1,2-di-hydropyridin-3-ylamino)-7-oxohept-2-enoate
(S, E)-Methyl 6-acetamido-7-(1-(2-(2-ethylbutylam ino)-2-oxoethyl)-2-oxo-1,2-dihydropyridin-3-ylamino)-7-oxohept-2-enoate

9. Verbindungen gemäß einem der Ansprüche 1 - 8 zur Verwendung in der Medizin.

10. Verbindungen gemäß einem der Ansprüche 1 - 9 zur Verwendung als Inhibitor für Transglutaminase 2.

11. Verbindungen gemäß einem der Ansprüche 1 - 10 zur Verwendung bei der Behandlung oder Prophylaxe von Zöliakie, Fibrosen, Thrombose, neurodegenerative Erkrankungen, Chorea Huntington, Parkinson, Alzheimer, Katarakt, Akne, Psoriasis, Hautalterung, Candiose und anderen transglutaminase abhängigen Erkrankungen.

12. Pharmazeutische Zusammensetzung enthaltend mindestens eine Verbindung gemäß einem der Ansprüche 1 - 8 und mindestens einen pharmakologisch verträglichen Träger, Hilfsstoff oder Lösungsmittel.

13. Pharmazeutische Zusammensetzung gemäß Anspruch 12, des weiteren umfassend einen Wirkstoff ausgewählt aus der Gruppe umfassend Vitamine, Entzündungshemmer, Peptidasen, Proteasen, monoklonale Antikörper, Immunmodulatoren und "tight junctions" Modulatoren.

14. Verfahren zur Herstellung der Verbindungen gemäß Anspruch 1 nach folgenden Syntheseschemata: 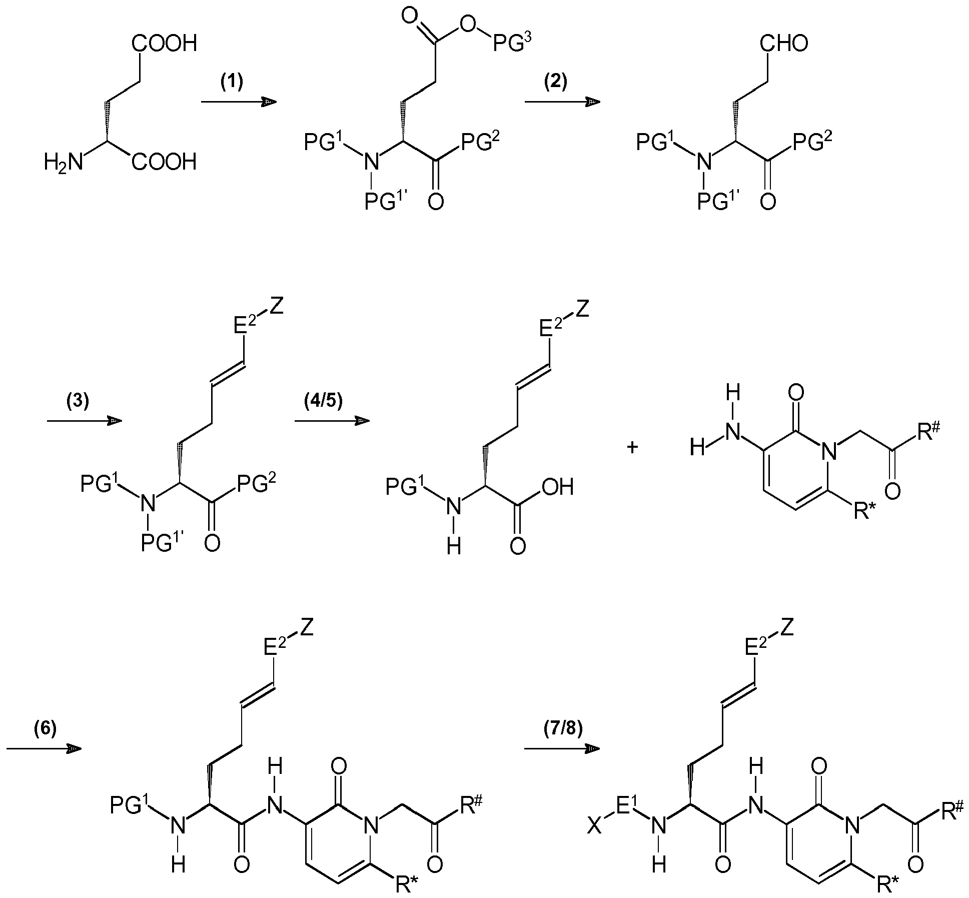
(0) Bereistellung von Glutaminsäure,
(1) Versehen der Glutaminsäure am C-Terminus und N-Terminus, sowie an der Carboxylfunktion der Seitenkette mit Schutzgruppen (PG1, PG2 und PG3),
(2) Reduktion der Carboxylfunktion der Seitenkette der Glutaminsäure zum Aldehyd,
(3) Überführung des erhaltenen Aldehyds in eine akzeptorsubstituierte elektrophile Doppelbindung,
(4) Entfernung einer Schutzgruppe am N-Terminus,
(5) Entfernung der Schutzgruppe am C-Terminus,
(6) Verlängerung des C-Terminus mit einem Pyridinonfragment,
(7) Entfernung der zweiten Schutzgruppe am N-Terminus
(8) Verlängerung des N-Terminus
oder 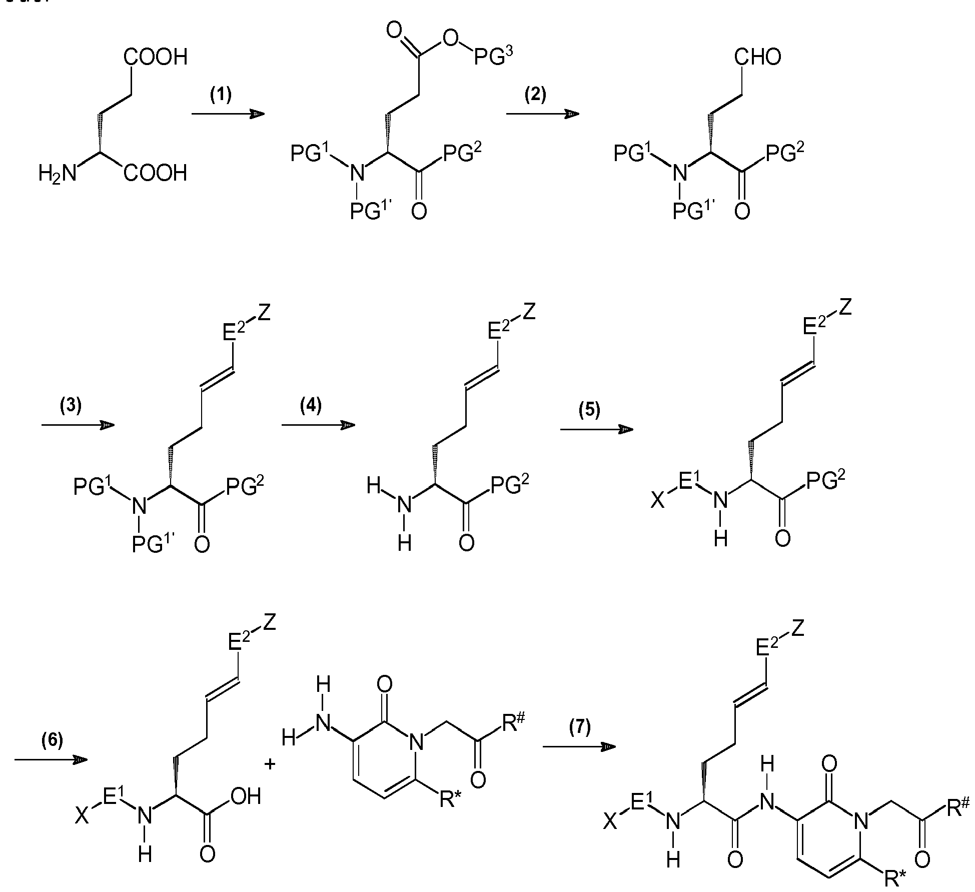
(0) Bereistellung von Glutaminsäure,
(1) Versehen der Glutaminsäure am C-Terminus und N-Terminus, sowie an der Carboxylfunktion der Seitenkette mit Schutzgruppen (PG1, PG2 und PG3),
(2) Reduktion der Carboxylfunktion der Seitenkette der Glutaminsäure zum Aldehyd,
(3) Überführung des erhaltenen Aldehyds in eine akzeptorsubstituierte elektrophile Doppelbindung,
(4) Entfernung der Schutzgruppen am N-Terminus,
(5) Verlängerung des N-Terminus,
(6) Entfernung der Schutzgruppe am C-Terminus,
(7) Verlängerung des C-Terminus mit einem Pyridinonfragment.
